# EUROPEAN PATENT SPECIFICATION

(11) **EP 1 058 678 B1**
(45) Date of publication and mention of the grant of the patent: **11.12.2002**
(21) Application number: 99908515.2
(22) Date of filing: 26.02.1999
(51) Int. Cl.: C07C 243/00

(54) **METAL-CATALYZED ARYLATIONS AND VINYLATIONS OF HYDRAZINES, HYDRAZONES, HYDROXYLAMINES AND OXIMES**
METALLKATALYSIERTE ARYLIERUNGEN UND VINYLIERUNGEN VON HYDRAZINEN, HYDRAZONEN, HYDROXYLAMINEN UND OXIMEN
ARYLATIONS CATALYSEES PAR DES METAUX D'HYDRAZINES ET D'HYDRAZONES, ET SUBSTRATS ASSOCIES

(30) Priority: 26.02.1998 US 30936; 06.04.1998 US 55557
(43) Date of publication of application: 13.12.2000
(73) Proprietor: Massachusetts Institute of Technology, Cambridge, MA 02145 (US)
(72) Inventor: BUCHWALD, Stephen L., Newton, MA 02158 (US); WAGAW, Seble, Cambridge, MA 02139 (US); GEIS, Oliver, 14167 Berlin (DE)
(74) Representative: Horner, Martin Grenville
(86) International application number: US9904217
(87) International publication number: WO99043643

(56) References cited:
- EP-A- 0 078 768
- EP-A- 0 187 285
- EP-A- 0 224 831
- EP-A- 0 790 233
- US-A- 3 867 452
- US-A- 4 374 271

## Description

### Background of the Invention

The present invention relates to improved methods for preparing aryl hydrazines, aryl hydrazones, *O*-aryl hydroxylamines, *N*-aryl hydroxylamines, *O*-aryl oximes and the like which are useful intermediates and end products in pharmaceutical and agricultural applications. Certain products of the present invention are utilized in a novel entry into the classical Fischer indole synthesis.

European patent application EP 0 790 233 A2, published 20 August 1997, describes a method of producing a 2-hydrazinonaphthalene compound, comprising reacting a 2-naphthol compound with a hydrazine in the presence of an acid catalyst.

United States patent 4,374,271 issued February 15, 1983, relates to nitrophenylhydrazine compounds and their use as chemical intermediates and as herbicides. Specifically, a class of N-amino substituted aniline and 1,3-phenylenediamine compounds having one or two nitro substituents and halo and trifluoromethyl substituents on the aromatic ring is described. The 3-halophenylhydrazine compounds were prepared by reaction of the corresponding 1,3-dihalobenzene derivative and a hydrazine compound. The reaction takes place at from about room temperature to about 100 C, preferably in the presence of a hydrocarbon solvent. Notably, hydrogen bromide or chloride is formed as a by-product of the reaction; therefore, at least two equivalents of the hydrazine reagent must be used.

Despite the recent successes with palladium-catalyzed cross-coupling reactions of Ar-X with amines, comparable couplings of aryl halides with hydrazines and the like have not been reported. Existing methods for the conversion of Ar-X to the corresponding aryl hydrazines often require harsh or restrictive reaction conditions and/or the presence of activating groups on the aromatic ring.

Thus there remains a need for an effective method of preparing a wide range of aryl hydrazines and the like under mild conditions and in high yields. There is a further need for an efficient catalytic system with high efficiencies and turnover numbers for the synthesis of these compounds.

### Summary of the Invention

The present invention provides general and attractive routes to a wide range of aryl hydrazines, aryl hydrazones, *O*-aryl hydroxylamines, *N*-aryl hydroxylamines, *O*-aryl oximes and the like. The methods provide several improvements over methods known heretofore, namely, the efficient synthesis of these compounds under mild conditions and in high yields. In particular, the method of the invention may be used in coupling reactions between aryl halides or sulfonates and ketone or aldehyde hydrazones; the products of these coupling reactions provide indoles via the Fischer indole synthesis. The methods of the present invention also provide furans, benzofurans, pyrroles and the like via series of transformations analogous to the Fischer indole synthesis. In other aspects of the invention, means are provided for synthesizing combinatorial libraries of indoles, carbazoles, pyrroles, benzofurans, furans and the like.

Additionally, the present invention provides improved methods for the synthesis of aryl amines. These improvements are based, in part, on the unexpected discovery that the order in which the various components of the reaction mixture are combined can have an appreciable effect on reaction rate and the like.

### Brief Description of the Figures

**Figure 1** presents examples of the effects on reaction rate of variations in the composition of the catalyst and the order of addition of the various reagents to the reaction mixture.
**Figure 2** presents the results of four reactions run utilizing the new order of addition of the various reagents to the reaction mixture.

### Detailed Description of the Invention

In one aspect of the invention, an aryl hydrazine is prepared by reacting hydrazine, a substituted hydrazine, or a hydrazide salt, with an activated aromatic compound in the presence of a base and a metal catalyst that includes a Group VIIIA metal atom such as iron, cobalt, nickel, ruthenium, rhodium, palladium, iridium or platinum; Group 10 metals -- platinum, palladium and nickel -- are the most preferred metals. The activated aromatic compound comprises an activated substituent, X, which generally is selected from the group consisting of halides and sulfonate esters. When the reaction takes place using an hydrazide salt, an additional base may not be required.

In certain embodiments, the subject method can be represented by the general reaction scheme shown below: 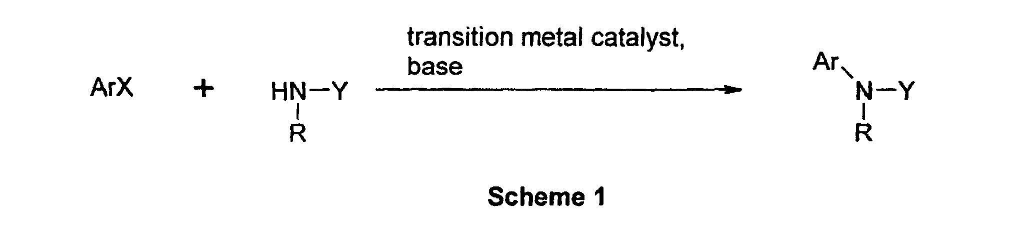 wherein
Ar represents an optionally substituted aromatic or heteroaromatic group;
X represents an activated group, e.g., a halide or a sulfonate, which can be replaced by a nucleophilic nitrogen in a transition metal-catalyzed arylation reaction;
Y represents NR₂, OR, N=CR₂, N(R)S(O)₂R, or N(R)C(O)NR₂;
R represents independently for each occurrence, as valence and stability permit, H, an optionally substituted alkyl, alkenyl or aryl; formyl, acyl, sulfonyl or -(CH₂)ₘ-R₈;
R₈ represents independently for each occurrence an optionally substituted aryl, cycloalkyl, cycloalkenyl, heterocycle or polycycle; and
m is an integer in the range 0 to 8 inclusive.

In additional embodiments, the subject method is represented by scheme 1 and the attendant definitions, wherein Ar is selected from the group consisting of optionally substituted monocyclic aromatic and heteroaromatic groups.

In additional embodiments, the subject method is represented by scheme 1 and the attendant definitions, wherein X is selected from the group consisting of halides and sulfonates.

In additional embodiments, the subject method is represented by scheme 1 and the attendant definitions, wherein X is selected from the group consisting of chloride, bromide, iodide, mesylate, triflate, tosylate and nonaflate.

In additional embodiments, the subject method is represented by scheme 1 and the attendant definitions, wherein Y is selected from the group consisting of NR₂, OR and N=CR₂.

In additional embodiments, the subject method is represented by scheme 1 and the attendant definitions, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is selected from the group consisting of NR₂, OR and N=CR₂.

In certain embodiments, the subject method can be represented by the general reaction scheme shown below: 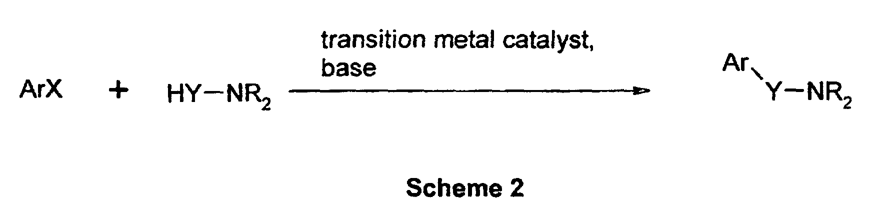 wherein
Ar represents an optionally substituted aromatic or heteroaromatic group;
X represents an activated group, e.g., a halide or a sulfonate, which can be replaced by Y in a transition metal-catalyzed arylation reaction;
Y represents O, S, or Se;
R represents independently for each occurrence, as valence and stability permit, H, an optionally substituted alkyl, alkenyl or aryl; alkylidene, formyl, acyl, sulfonyl, or -(CH₂)ₘ-R₈; the two instances of R taken together may represent an alkylidene group;
R₈ represents independently for each occurrence an optionally substituted aryl, cycloalkyl, cycloalkenyl, heterocycle or polycycle; and
m is an integer in the range 0 to 8 inclusive.

In additional embodiments, the subject method is represented by scheme 2 and the attendant definitions, wherein Ar is selected from the group consisting of optionally substituted monocyclic aromatic and heteroaromatic groups.

In additional embodiments, the subject method is represented by scheme 2 and the attendant definitions, wherein X is selected from the group consisting of halides and sulfonates.

In additional embodiments, the subject method is represented by scheme 2 and the attendant definitions, wherein X is selected from the group consisting of chloride, bromide, iodide, mesylate, triflate, tosylate and nonaflate.

In additional embodiments, the subject method is represented by scheme 2 and the attendant definitions, wherein Y is O.

In additional embodiments, the subject method is represented by scheme 2 and the attendant definitions, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is O.

In certain embodiments, the subject reaction may be an intramolecular reaction. In this instance, it will be realized, with reference to Scheme 1, that YNHR is covalently attached to Ar via T which represents a tether between Ar and Y. The intramolecular reaction results in a product containing at least one more ring than the substrate and said method is represented in general in Scheme 3: 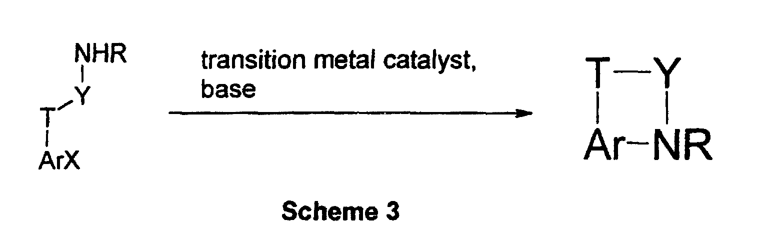 wherein
Ar represents an optionally substituted aromatic or heteroaromatic group;
X represents an activated group, e.g., a halide or a sulfonate, which can be replaced by a nucleophilic nitrogen in a transition metal-catalyzed arylation reaction;
Y represents NR, O, *sp*²-hybridized N in a π-bond to T, NS(O)₂R, or NC(O)NR₂;
T represents a covalent tether connecting Y and Ar, said tether comprising between 0 and 4 backbone atoms; the backbone of said tether may comprise a π-bond, provided that the configuration of said π-bond is such that the described intramolecular reaction is geometrically feasible, or that said π-bond can adopt a configuration under the reaction conditions that renders the intramolecular reaction geometrically feasible; said tether may be optionally unsubstituted, bearing any number of substituents of any type permitted by stability and the rules of valence;
R represents independently for each occurrence, as valence and stability permit, H, an optionally substituted alkyl, alkenyl or aryl; formyl, acyl, sulfonyl or -(CH₂)ₘ-R₈;
R₈ represents independently for each occurrence an optionally substituted aryl, cycloalkyl, cycloalkenyl, heterocycle or polycycle; and
m is an integer in the range 0 to 8 inclusive.

In additional embodiments, the subject method is represented by scheme 3 and the attendant definitions, wherein Ar is selected from the group consisting of optionally substituted monocyclic aromatic and heteroaromatic groups.

In additional embodiments, the subject method is represented by scheme 3 and the attendant definitions, wherein X is selected from the group consisting of halides and sulfonates.

In additional embodiments, the subject method is represented by scheme 3 and the attendant definitions, wherein X is selected from the group consisting of chloride, bromide, iodide, mesylate, triflate, tosylate and nonaflate.

In additional embodiments, the subject method is represented by scheme 3 and the attendant definitions, wherein Y is selected from the group consisting of NR, O, and *sp*²-hybridized N in a π-bond to T.

In additional embodiments, the subject method is represented by scheme 3 and the attendant definitions, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is selected from the group consisting of NR, O, and *sp*²-hybridized N in a π-bond to T.

In certain embodiments, the subject method may be another form of intramolecular reaction. In this instance, it will be realized that, with reference to Scheme 2, N(R)YH is covalently attached to Ar via T which represents a tether between Ar and N. The intramolecular reaction results in a product containing at least one more ring than the substrate and said method is represented in general in Scheme 4: 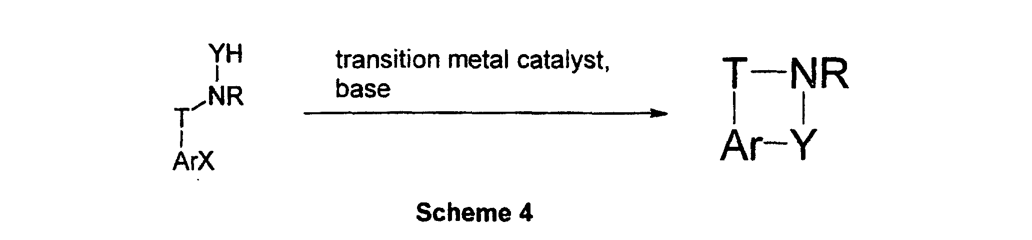 wherein
Ar represents an optionally substituted aromatic or heteroaromatic group;
X represents an activated group, e.g., a halide or a sulfonate, which can be replaced by Y in a transition metal-catalyzed arylation reaction;
Y represents O, S, or Se;
T represents a covalent tether connecting NR and Ar, said tether comprising between 0 and 4 backbone atoms; the backbone of said tether may comprise a π-bond, provided that the configuration of said π-bond is such that the described intramolecular reaction is geometrically feasible, or that said π-bond can adopt a configuration under the reaction conditions that renders the intramolecular reaction geometrically feasible; said tether may be optionally substituted, bearing any number of substituents of any type permitted by stability and the rules of valence;
R represents independently for each occurrence, as valence and stability permit, H, an optionally substituted alkyl, alkenyl or aryl; formyl, acyl, sulfonyl or -(CH₂)ₘ-R₈;
R₈ represents independently for each occurrence an optionally substituted aryl, cycloalkyl, cycloalkenyl, heterocycle or polycycle; and
m is an integer in the range 0 to 8 inclusive.

In additional embodiments, the subject method is represented by scheme 4 and the attendant definitions, wherein Ar is selected from the group consisting of optionally substituted monocyclic aromatic and heteroaromatic groups.

In additional embodiments, the subject method is represented by scheme 4 and the attendant definitions, wherein X is selected from the group consisting of halides and sulfonates.

In additional embodiments, the subject method is represented by scheme 4 and the attendant definitions, wherein X is selected from the group consisting of chloride, bromide, iodide, mesylate, triflate, tosylate and nonaflate.

In additional embodiments, the subject method is represented by scheme 4 and the attendant definitions, wherein Y is O.

In additional embodiments, the subject method is represented by scheme 4 and the attendant definitions, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is O.

In certain embodiments, the subject method can be represented by the general reaction scheme depicted in Scheme 5: 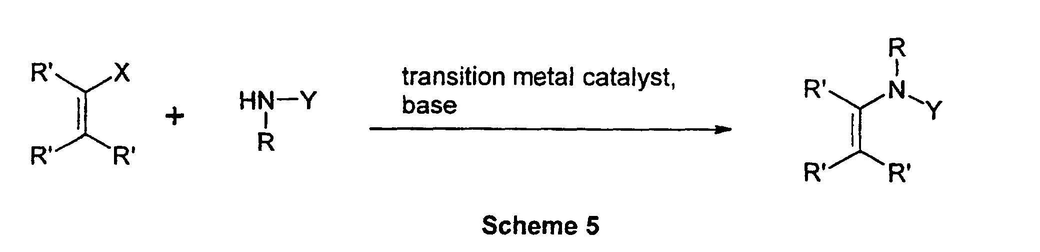 wherein
X represents an activated group, e.g., a halide or a sulfonate, which can be replaced by a nucleophilic nitrogen in a transition metal-catalyzed vinylation reaction;
Y represents NR₂, OR, N=CR₂, N(R)S(O)₂R, or N(R)C(O)NR₂;
R represents independently for each occurrence, as valence and stability permit, H, an optionally substituted alkyl, alkenyl or aryl; formyl, acyl, sulfonyl or -(CH₂)ₘ-R₈;
R' represents independently for each occurrence, as valence and stability permit, H, halogen, lower alkyl, lower alkenyl, lower alkynyl, carbonyl group (e.g., ester, carboxylate, or formate), thiocarbonyl (e.g., thiolester, thiolcarboxylate, or thiolformate), ketone, aldehyde, amino, acylamino, amido, amidino, cyano, nitro, azido, sulfonyl, sulfoxido, sulfate, sulfonate, sulfamoyl, sulfonamido, phosphoryl, phosphonate, phosphinate, -(CH₂)ₘ-R₈, -(CH₂)ₘ-OH, -(CH₂)ₘ-O-lower alkyl, -(CH₂)ₘ-O-lower alkenyl, -(CH₂)ₘ-O-(CH₂)ₙ-R₈, -(CH₂)ₘ-SH, -(CH₂)ₘ-S-lower alkyl, -(CH₂)ₘ-S-lower alkenyl, -(CH₂)ₘ-S-(CH₂)ₙ-R₈, or protecting groups of the above, or a solid or polymeric support;
R₈ represents independently for each occurrence an optionally substituted aryl, cycloalkyl, cycloalkenyl, heterocycle or polycycle; and
n and m are integers independently for each occurrence selected from the range of 0 to 8 inclusive.

In additional embodiments, the subject method is represented by scheme 5 and the attendant definitions, wherein X is selected from the group consisting of halides and sulfonates.

In additional embodiments, the subject method is represented by scheme 5 and the attendant definitions, wherein X is selected from the group consisting of chloride, bromide, iodide, mesylate, triflate, tosylate and nonaflate.

In additional embodiments, the subject method is represented by scheme 5 and the attendant definitions, wherein Y is selected from the group consisting of NR₂, OR and N=CR₂.

In additional embodiments, the subject method is represented by scheme 5 and the attendant definitions, wherein X is selected from the group consisting of chloride, bromide, iodide, mesylate, triflate, tosylate and nonaflate; and Y is selected from the group consisting of NR₂, OR and N=CR₂.

In certain embodiments, the subject method can be represented by the general reaction scheme depicted in Scheme 6: 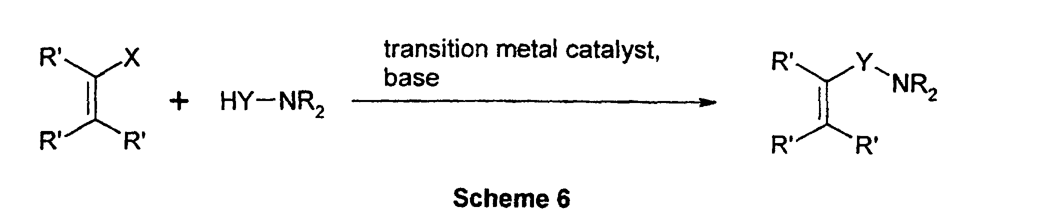 wherein
X represents an activated group, e.g., a halide or a sulfonate, which can be replaced by Y in a transition metal-catalyzed vinylation reaction;
Y represents O, S, or Se;
R represents independently for each occurrence, as valence and stability permit. H, an optionally substituted alkyl, alkenyl or aryl; alkylidene, formyl, acyl, sulfonyl, or -(CH₂)ₘ-R₈; the two instances of R taken together may represent an alkylidene group;
R' represents independently for each occurrence, as valence and stability permit, H, halogen, lower alkyl, lower alkenyl, lower alkynyl, carbonyl group (e.g. ester, carboxylate, or formate), thiocarbonyl (e.g. thiolester, thiolcarboxylate, or thiolformate), ketone, aldehyde, amino, acylamino, amido, amidino, cyano, nitro, azido, sulfonyl, sulfoxido, sulfate, sulfonate, sulfamoyl, sulfonamido, phosphoryl, phosphonate, phosphinate, -(CH₂)ₘ-R₈, -(CH₂)ₘ-OH, -(CH₂)ₘ-O-lower alkyl, -(CH₂)ₘ-O-lower alkenyl, -(CH₂)ₘ-O-(CH₂)ₙ-R₈, -(CH₂)ₘ-SH, -(CH₂)ₘ-S-lower alkyl, -(CH₂)ₘ-S-lower alkenyl, -(CH₂)ₘ-S-(CH₂)ₙ-R₈, or protecting groups of the above, or a solid or polymeric support;
R₈ represents independently for each occurrence an optionally substituted aryl, cycloalkyl, cycloalkenyl, heterocycle or polycycle; and
n and m are integers independently for each occurrence selected from the range of 0 to 8 inclusive.

In additional embodiments, the subject method is represented by scheme 6 and the attendant definitions, wherein X is selected from the group consisting of halides and sulfonates.

In additional embodiments, the subject method is represented by scheme 6 and the attendant definitions, wherein X is selected from the group consisting of chloride, bromide, iodide, mesylate, triflate, tosylate and nonaflate.

In additional embodiments, the subject method is represented by scheme 6 and the attendant definitions, wherein Y is O.

In additional embodiments, the subject method is represented by scheme 6 and the attendant definitions, wherein X is selected from the group consisting of chloride, bromide, iodide, mesylate, triflate, tosylate and nonaflate; and Y is O.

Artisans of ordinary skill will recognize the potential for intramolecular variants of the vinylation reactions presented in Schemes 5 and 6. Specifically, a comparison of the embodiment of the subject method depicted in Scheme 1 with Scheme 3, and a similar comparison of the embodiments depicted in Schemes 2 and 4, will serve to teach the embodiments that comprise an intramolecular vinylation reaction.

While not being bound by any particular mode of operation, it is hypothesized that the mechanism of the Pd-catalyzed arylation of hydrazines and the like may proceed via a pathway similar to that depicted in Scheme 7. Scheme 7 presents a proposed reaction pathway for the synthesis of an aryl hydrazine via an intermolecular reaction. Any ligands that may be present on the palladium atom during this process have been omitted for clarity. With reference to Scheme 7, oxidative addition of the Pd(0) complex to the C-X bond of the activated aryl moiety (ArX) affords the Pd(II) organometallic intermediate **A.** The hydrazine could then displace X⁻ from **A** and thereby generate cation **B.** Cation **B** would then suffer deprotonation to afford charge neutral intermediate **C**, which would subsequently undergo reductive elimination to yield the product aryl hydrazine and regenerate the active catalyst. The reaction sequence is likely to be similar for intramolecular reactions. Alternatively, and particularly for nickel catalysts, the active transition metal species in the oxidative addition step may involve the metal in the +1 oxidation state.

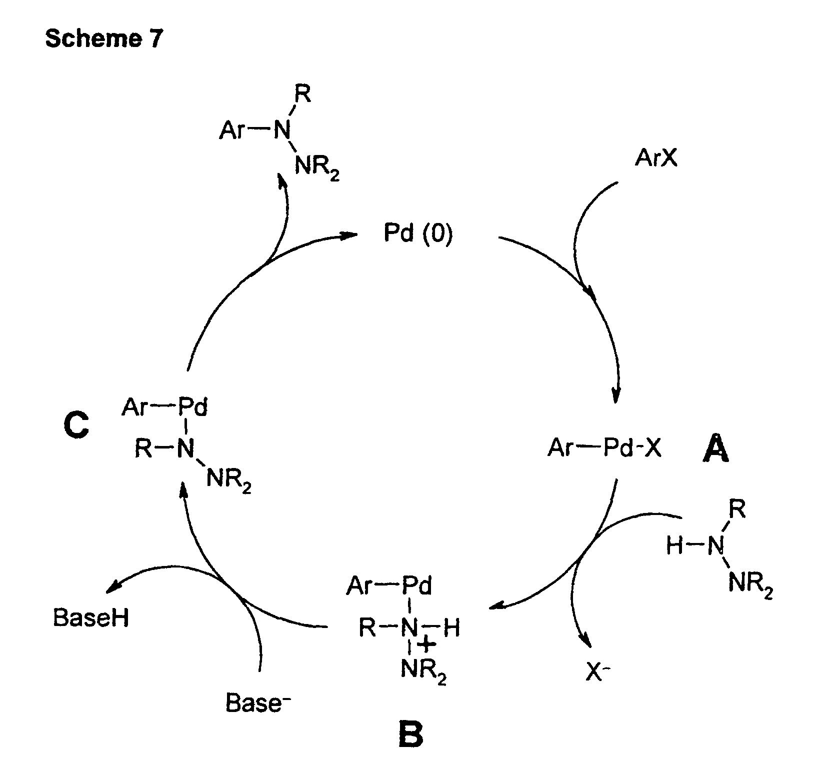

In certain embodiments of the invention, there is no need to use large excesses of either reactant -- hydrazine and the like or aromatic compound. The reaction proceeds quickly and in high yield to the reaction product using substantially stoichiometric amounts of reagents. Thus, the hydrazine may be present in as little as a two-fold excess and preferably in no greater than a 20% excess relative to the aromatic compound. Alternatively, the aromatic compound may be present in as little as a two-fold excess and preferably in no greater than a 20% excess relative to the hydrazine.

The reaction can proceed at mild temperatures and pressures to give high yields of the product aryl hydrazine or the like. Thus, yields of greater than 45%, preferably greater than 75% and even more preferably greater than 80% may be obtained by reaction at mild temperatures according to the invention. The reaction may be carried out at temperature less than 120°C, and preferably in the range of 50-120°C. In certain embodiments, the reaction is carried out at a temperature in the range of 80-100°C.

The reaction can be run in a wide range of solvent systems, including polar aprotic solvents. Alternatively, in certain embodiments, the subject reactions may be carried in the absence of added solvent.

The ability to provide a synthesis scheme for hydrazines and the like which can be carried out under mild conditions and/or with non-polar solvents has broad application, especially in the agricultural and pharmaceutical industries, as well as in the polymer industry. In this regard, the subject reaction is more amenable to use of reactants or products which include sensitive functionalities, e.g., which would otherwise be labile under harsh reaction conditions.

A product synthesized by a method of the present invention may be either an end-product or an intermediate in a synthesis scheme. In cases where the product synthesized by a method of the present invention is an intermediate, the product may be subjected to one or more additional transformations to yield the desired end-product. The set of additional transformations contemplated comprises isomerizations, hydrolyses, oxidations, reductions, additions, eliminations, olefinations, functional group interconversions, transition metal-mediated reactions, transition metal-catalyzed reactions, bond-forming reactions, cleavage reactions, fragmentation reactions, thermal reactions, photochemical reactions, cycloadditions, sigmatropic rearrangements, electrocyclic reactions, chemoselective reactions, regioselective reactions, stereoselective reactions, diastereoselective reactions, enantioselective reactions, and kinetic resolutions. The invention expressly comprises use of a method of the present invention as a step -- either initial, intermediate or final - in the synthesis of known or new pharmaceuticals, e.g., antivirals, antibiotics and analgesics.

The subject hydrazine arylation reactions can be used as part of a combinatorial synthesis scheme to yield libraries of aryl hydrazines and the like. Accordingly, another aspect of the present invention relates to use of the subject method to generate variegated libraries of aryl hydrazines and the like, and to the libraries themselves. The libraries can be soluble or linked to insoluble supports, e.g., either through substituents of the aryl group or the hydrazine etc.

### Definitions

For convenience, before further description of the present invention, certain terms employed in the specification, examples, and appended claims are collected here.

The term "substrate aryl group" refers to an aryl group containing an electrophilic atom which is susceptible to the subject cross-coupling reaction, e.g., the electrophilic atom bears a leaving group. In reaction scheme 1, the substrate aryl is represented by ArX, and X is the leaving group. The aryl group, Ar, is said to be substituted if, in addition to X, it is substituted at yet other positions. The substrate aryl group can be a single ring molecule, or can be a component of a larger molecule.

The terms "hydrazine and/or the like" refer to a hydrazine, hydrazone, hydroxylamine, oxime etc. and salts thereof which can attack the electrophilic atom of the substrate aryl group and displace the leaving group in the subject cross-coupling reaction. In Schemes 1 and 2, the nucleophilic hydrazine and/or the like are represented by HN(R)-Y, and HY-NR₂, respectively. The hydrazine and/or the like can be a component of a molecule separate from the substrate aryl group, or a substituent of the same molecule (e.g., for intramolecular cross-couplings).

The term "nucleophile" is recognized in the art, and as used herein means a chemical moiety having a reactive pair of electrons.

The term "electrophile" is art-recognized and refers to chemical moieties which can accept a pair of electrons from a nucleophile as defined above. Electrophilic moieties useful in the method of the present invention include halides and sulfonates.

The terms "electrophilic atom", "electrophilic center" and "reactive center" as used herein refer to the atom of the substrate aryl moiety which is attacked by, and forms a new bond to the nucleophilic heteroatom of the hydrazine and the like. In most (but not all) cases, this will also be the aryl ring atom from which the leaving group departs.

The term "electron-withdrawing group" is recognized in the art, and denotes the tendency of a substituent to attract valence electrons from neighboring atoms, i.e., the substituent is electronegative with respect to neighboring atoms. A quantification of the level of electron-withdrawing capability is given by the Hammett sigma (s) constant. This well known constant is described in many references, for instance, J. March, Advanced Organic Chemistry, McGraw Hill Book Company, New York, (1977 edition) pp. 251-259. The Hammett constant values are generally negative for electron donating groups (s[P] = - 0.66 for NH₂) and positive for electron withdrawing groups (s[P] = 0.78 for a nitro group), s[P] indicating para substitution. Exemplary electron-withdrawing groups include nitro, ketone, aldehyde, sulfonyl, trifluoromethyl, -CN, chloride, and the like. Exemplary electron-donating groups include amino, methoxy, and the like.

The term "reaction product" means a compound which results from the reaction of the hydrazine or the like and the substrate aryl group. In general, the term "reaction product" will be used herein to refer to a stable, isolable aryl ether adduct, and not to unstable intermediates or transition states.

The term "catalytic amount" is recognized in the art and means a substoichiometric amount of reagent relative to a reactant. As used herein, a catalytic amount means from 0.0001 to 90 mole percent reagent relative to a reactant, more preferably from 0.001 to 50 mole percent, still more preferably from 0.01 to 10 mole percent, and even more preferably from 0.1 to 5 mole percent reagent to reactant.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight chain or branched chain alkyl has 30 or fewer carbon atoms in its backbone (e.g., C₁-C₃₀ for straight chain, C₃-C₃₀ for branched chain), and more preferably 20 or fewer. Likewise, preferred cycloalkyls have from 3-10 carbon atoms in their ring structure, and more preferably have 5, 6 or 7 carbons in the ring structure.

Moreover, the term "alkyl" (or "lower alkyl") as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, a halogen, a hydroxyl, a carbonyl (such as a carboxyl, an ester, a formyl, or a ketone), a thiocarbonyl (such as a thioester, a thioacetate, or a thioformate), an alkoxyl, a phosphoryl, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, -CN and the like. Exemplary substituted alkyls are described below. Cycloalkyls can be further substituted with alkyls, alkenyls, alkoxys, alkylthios, aminoalkyls, carbonyl-substituted alkyls, -CF₃, -CN, and the like.

The term "aralkyl", as used herein, refers to an alkyl group substituted with an aryl group (e.g., an aromatic or heteroaromatic group).

The terms "alkenyl" and "alkynyl" refer to unsaturated aliphatic groups analogous in length and possible substitution to the alkyls described above, but that contain at least one double or triple bond respectively.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Preferred alkyl groups are lower alkyls. In certain embodiments, a substituent designated herein as alkyl is a lower alkyl.

The term "aryl" as used herein includes 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics". The aromatic ring can be substituted at one or more ring positions with such substituents as described above, for example, halogen, azide, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocyclyls.

The abbreviations Me, Et, Ph, Tf, Nf, Ts, Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by organic chemists of ordinary skill in the art appears in the first issue of each volume of the *Journal of Organic Chemistry;* this list is typically presented in a table entitled Standard List of Abbreviations. The abbreviations contained in said list, and all abbreviations utilized by organic chemists of ordinary skill in the art are hereby incorporated by reference.

The terms *ortho, meta* and *para* apply to 1,2-, 1,3- and 1,4-disubstituted benzenes, respectively. For example, the names 1,2-dimethylbenzene and *ortho*-dimethylbenzene are synonymous.

The terms "heterocyclyl" or "heterocyclic group" refer to 3- to 10-membered ring structures, more preferably 3- to 7-membered rings, whose ring structures include one to four heteroatoms. Heterocycles can also be polycycles. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The terms "polycyclyl" or "polycyclic group" refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle can be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "carbocycle", as used herein, refers to an aromatic or non-aromatic ring in which each atom of the ring is carbon.

The term "heteroatom" as used herein means an atom of any element other than carbon or hydrogen. Preferred heteroatoms are nitrogen, oxygen, sulfur and phosphorous.

As used herein, the term "nitro" means -NO₂; the term "halogen" designates -F, -Cl, -Br or -I; the term "sulfhydryl" means -SH; the term "hydroxyl" means -OH; and the term "sulfonyl" means -SO₂-.

The terms "amine" and "amino" are art recognized and refer to both unsubstituted and substituted amines, e.g., a moiety that can be represented by the general formula: 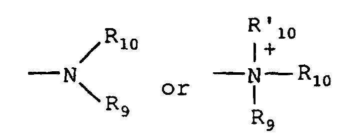 wherein R₉, R₁₀ and R'₁₀ each independently represent a hydrogen, an alkyl, an alkenyl, -(CH₂)ₘ-R₈, or R₉ and R₁₀ taken together with the N atom to which they are attached complete a heterocycle having from 4 to 8 atoms in the ring structure; R₈ represents an aryl, a cycloalkyl, a cycloalkenyl, a heterocycle or a polycycle; and m is zero or an integer in the range of 1 to 8. In preferred embodiments, only one of R₉ or R₁₀ can be a carbonyl, e.g., R₉, R₁₀ and the nitrogen together do not form an imide. In even more preferred embodiments, R₉ and R₁₀ (and optionally R'₁₀) each independently represent a hydrogen, an alkyl, an alkenyl, or -(CH₂)ₘ-R₈. Thus, the term "alkylamine" as used herein means an amine group, as defined above, having a substituted or unsubstituted alkyl attached thereto, i.e., at least one of R₉ and R₁₀ is an alkyl group.

The term "acylamino" is art-recognized and refers to a moiety that can be represented by the general formula: 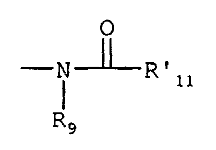 wherein R9 is as defined above, and R'₁₁ represents a hydrogen, an alkyl, an alkenyl or -(CH₂)ₘ-R₈, where m and R₈ are as defined above.

The term "amido" is art recognized as an amino-substituted carbonyl and includes a moiety that can be represented by the general formula: 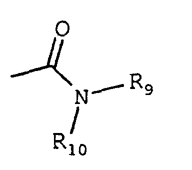 wherein R₉, R₁₀ are as defined above. Certain embodiments of the amide will not include imides which may be unstable.

The term "alkylthio" refers to an alkyl group, as defined above, having a sulfur radical attached thereto. In certain embodiments, the "alkylthio" moiety is represented by one of -S-alkyl, -S-alkenyl, -S-alkynyl, and -S-(CH₂)ₘ-R₈, wherein m and R₈ are defined above. Representative alkylthio groups include methylthio, ethyl thio, and the like.

The term "carbonyl" is art recognized and includes such moieties as can be represented by the general formula: 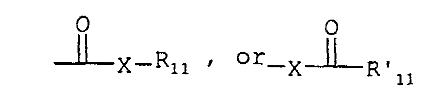 wherein X is a bond or represents an oxygen or a sulfur, and R₁₁ represents a hydrogen, an alkyl, an alkenyl, -(CH₂)ₘ-R₈ or a pharmaceutically acceptable salt, R'₁₁ represents a hydrogen, an alkyl, an alkenyl or -(CH₂)ₘ-R₈, where m and R₈ are as defined above. Where X is an oxygen and R₁₁ or R'₁₁ is not hydrogen, the formula represents an "ester". Where X is an oxygen, and R₁₁ is as defined above, the moiety is referred to herein as a carboxyl group, and particularly when R₁₁ is a hydrogen, the formula represents a "carboxylic acid". Where X is an oxygen, and R'₁₁ is hydrogen, the formula represents a "formate". In general, where the oxygen atom of the above formula is replaced by sulfur, the formula represents a "thiolcarbonyl" group. Where X is a sulfur and R₁₁ or R'₁₁ is not hydrogen, the formula represents a "thiolester." Where X is a sulfur and R₁₁ is hydrogen, the formula represents a "thiolcarboxylic acid." Where X is a sulfur and R₁₁' is hydrogen, the formula represents a "thiolformate." On the other hand, where X is a bond, and R₁₁ is not hydrogen, the above formula represents a "ketone" group. Where X is a bond, and R₁₁ is hydrogen, the above formula represents an "aldehyde" group.

The terms "alkoxyl" or "alkoxy" as used herein refers to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, -O-alkynyl, -O-(CH₂)ₘ-R₈, where m and R₈ are described above.

The term "sulfonate" is art recognized and includes a moiety that can be represented by the general formula:: 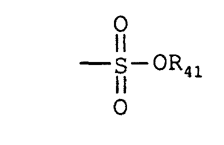 in which R₄₁ is an electron pair, hydrogen, alkyl, cycloalkyl, or aryl.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, methanesulfonate ester, and nonafluorobutanesulfonate ester functional groups and molecules that contain said groups, respectively.

The term "sulfate" is art recognized and includes a moiety that can be represented by the general formula: 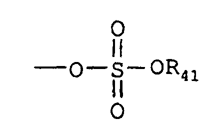 in which R₄₁ is as defined above.

The term "sulfonamido" is art recognized and includes a moiety that can be represented by the general formula: 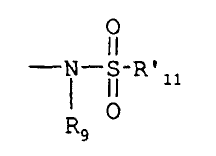 in which R₉ and R'₁₁ are as defined above.

The term "sulfamoyl" is art-recognized and includes a moiety that can be represented by the general formula: 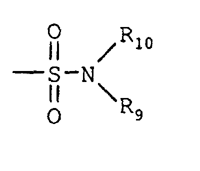 in which R₉ and R₁₀ are as defined above.

The terms "sulfoxido" or "sulfinyl", as used herein, refers to a moiety that can be represented by the general formula: 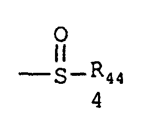 in which R₄₄ is selected from the group consisting of hydrogen, alkyl, alkenyl, alkynyl, cycloalkyl, heterocyclyl, aralkyl, or aryl.

A "phosphoryl" can in general be represented by the formula: 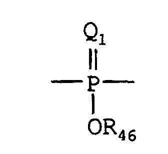 wherein Q₁ represented S or O, and R₄₆ represents hydrogen, a lower alkyl or an aryl. When used to substitute, e.g., an alkyl, the phosphoryl group of the phosphorylalkyl can be represented by the general formula: 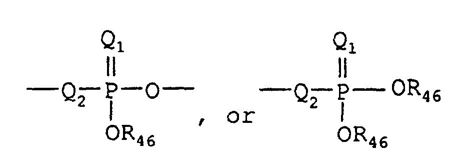 wherein Q₁ represented S or O, and each R₄₆ independently represents hydrogen, a lower alkyl or an aryl, Q₂ represents O, S or N. When Q₁ is an S, the phosphoryl moiety is a "phosphorothioate".

A "phosphoramidite" can be represented in the general formula: 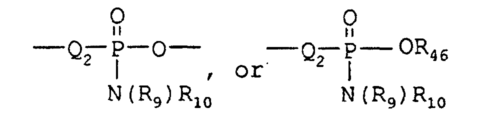 wherein R₉ and R₁₀ are as defined above, and Q₂ represents O, S or N.

A "phosphonamidite" can be represented in the general formula: 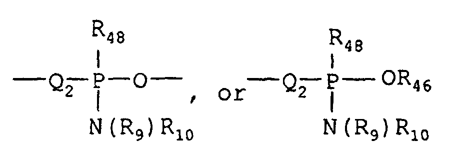 wherein R₉ and R₁₀ are as defined above, Q₂ represents O, S or N, and R₄₈ represents a lower alkyl or an aryl, Q₂ represents O, S or N.

A "selenoalkyl" refers to an alkyl group having a substituted seleno group attached thereto. Exemplary "selenoethers" which may be substituted on the alkyl are selected from one of -Se-alkyl, -Se-alkenyl, -Se-alkynyl, and -Se-(CH₂)ₘ-R₈, m and R₈ being defined above.

Analogous substitutions can be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The phrase "protecting group" as used herein means temporary modifications of a potentially reactive functional group which protect it from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M *Protective Groups in Organic Synthesis,* 2^{nd} ed.; Wiley: New York, 1991).

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described hereinabove. The permissible substituents can be one or more and the same or different for appropriate organic compounds. For purposes of this invention, the heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

A "polar solvent" means a solvent which has a dipole moment (ε) of 2.9 or greater, such as DMF, THF, ethylene gylcol dimethyl ether, DMSO, acetone, acetonitrile, methanol, ethanol, isopropanol, n-propanol, t-butanol or 2-methoxyethyl ether. Preferred solvents are DMF, diglyme, and acetonitrile.

An "aprotic solvent" means a solvent that is not a hydrogen bond donor. Examples of such solvents are acetonitrile, toluene, DMF, diglyme, THF or DMSO.

A "polar, aprotic solvent" means a solvent which has a dipole moment (ε) of 2.9, and is not a hydrogen bond donor, for example DMF, acetonitrile, DMSO and THF.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

### Exemplary Catalyzed Reactions

As described above, one invention of the Applicants' features a transition metal-catalyzed cross-coupling reaction which comprises combining a hydrazine with an aryl group (a "substrate aryl") bearing an activated group X. The reaction includes at least a catalytic amount of a transition metal catalyst and the combination is maintained under conditions appropriate for the metal catalyst to catalyze the nucleophilic addition of the reactive hydrazine to the electrophilic atom of the substrate aryl.

In an illustrative embodiment, the subject method can be used for the intermolecular reaction between an activated aryl substrate and a hydrazine to give an aryl hydrazine:

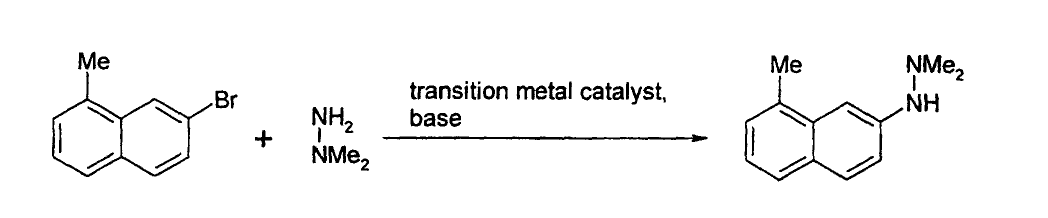

In a second illustrative embodiment, the subject method can be used to bring about an intramolecular arylation of a hydrazine; said arylation yields a diazaheterocycle. In some cases, including the case illustrated below, the initially-formed diazaheterocycle can be oxidized to yield a new diazaaromatic compound:

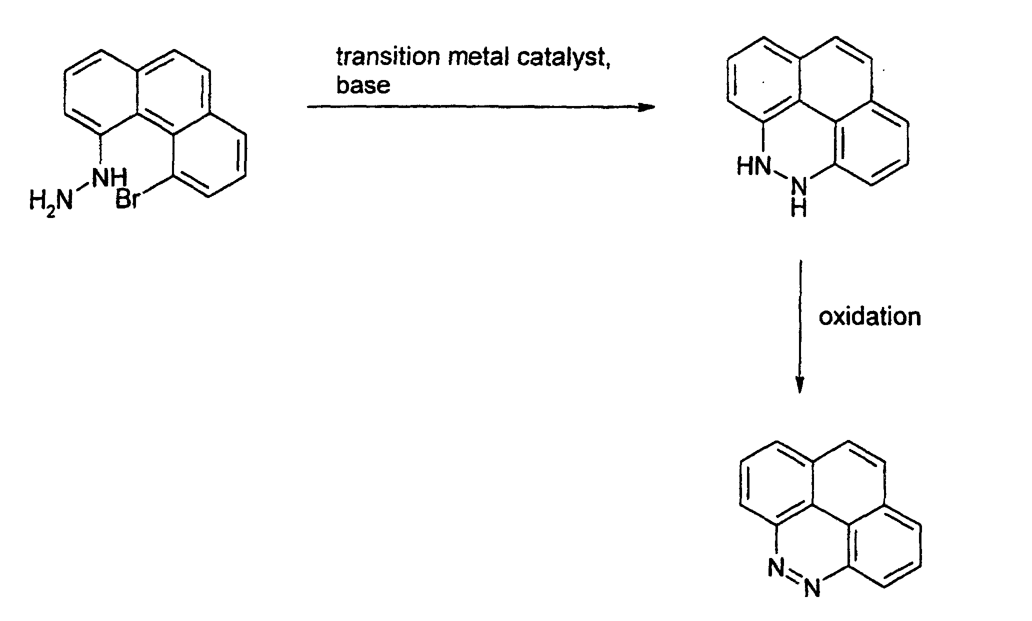

Another aspect of the Applicants' invention features a transition metal-catalyzed cross-coupling reaction which comprises combining a hydroxylamine with an aryl group (a "substrate aryl") bearing an activated group X. The reaction includes at least a catalytic amount of a transition metal catalyst and the combination is maintained under conditions appropriate for the metal catalyst to catalyze the nucleophilic addition of a reactive nitrogen or oxygen of a hydroxylamine, depending upon substrates and conditions selected, or a reactive oxygen of an oxime to the electrophilic atom of the substrate aryl.

In an embodiment illustrative of this aspect of the invention, the subject method can be exploited for the preparation of an *O*-alkenyl oxime from a vinyl halide and an oxime:

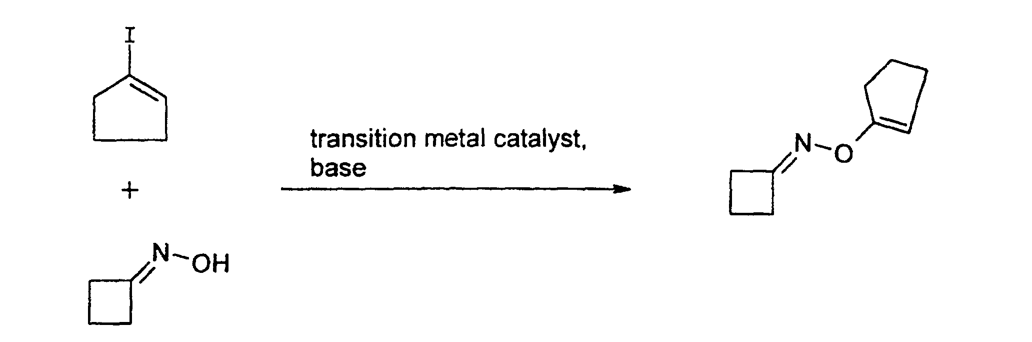

In an second embodiment illustrative of this aspect of the invention, the subject method can be exploited in the arylation of an *O*-alkyl hydroxylamine to give an *N*-aryl-*O*-alkyl hydroxylamine:

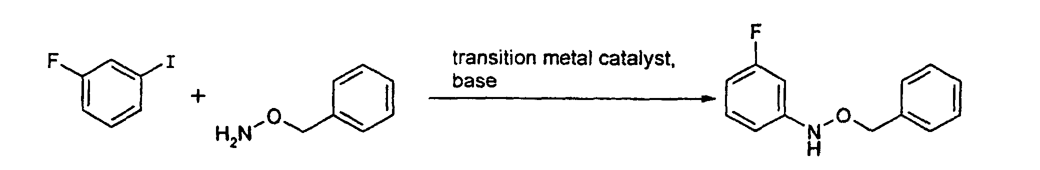

Intramolecular variants are available of the aspects of the invention that center on hydroxyl amines. An illustrative embodiment is shown below:

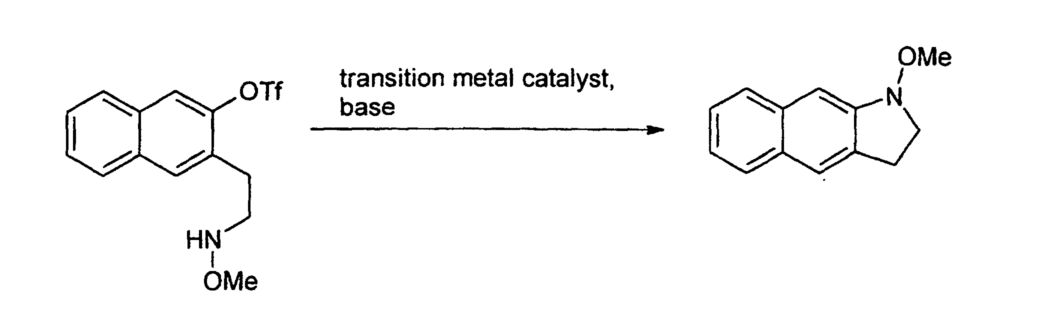

A second illustrative embodiment of an intramolecular arylation of a hydroxyl amine is shown below. Also illustrated below is the potential for the preparation of 1,*n*-amino alcohols via a subsequent reduction of the nitrogen-oxygen single bond in products of the subject arylation.

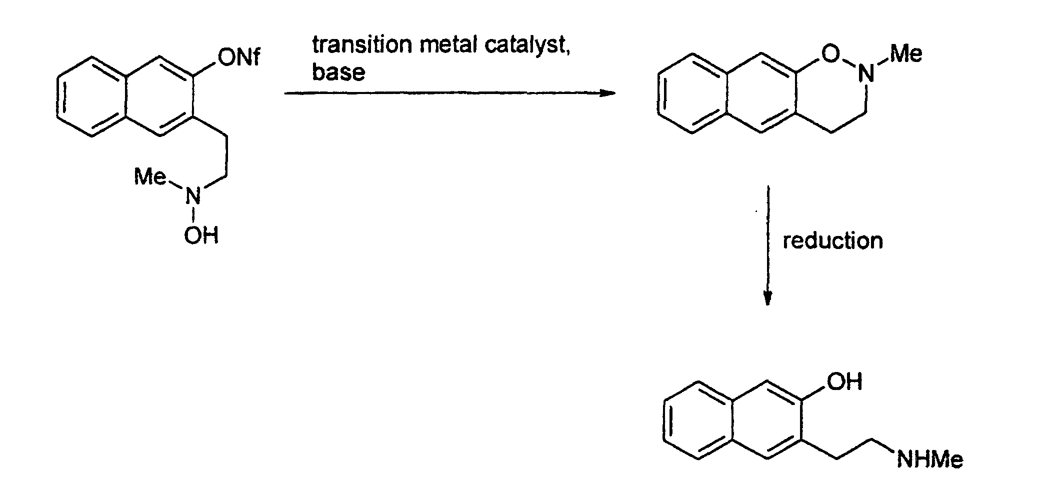

The subject method can be applied to the synthesis of *N*-arylhydrazones. A preferred embodiment, illustrated below, involves the transition metal catalyzed arylation of benzophenone hydrazone:

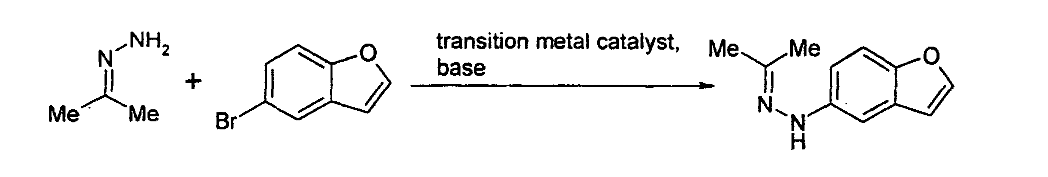

In still another illustrative embodiment, the subject reaction between a hydrazone and an activated aryl substrate, followed by reduction of the resulting *N*-aryl hydrazone, can be utilized for the synthesis of anilines:

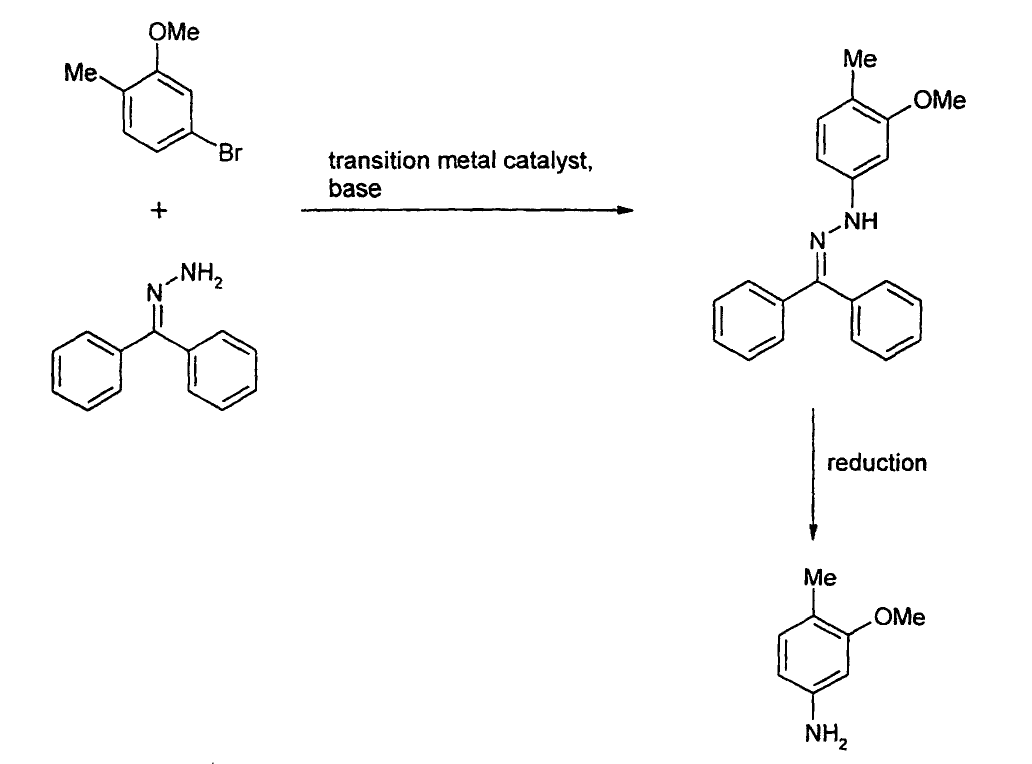

In a preferred illustrative embodiment, the subject arylation of benzophenone hydrazone is the initial step in a conceptually novel entry into the Fischer indole synthesis. The entire sequence of events in this novel strategy for indole synthesis is illustrated below. The second step in this strategy involves two discrete events: 1) an acid-catalyzed exchange of a new aldehyde or ketone for benzophenone to form a new hydrazone -- labeled "not isolated" -- and benzophenone (not shown); and 2) the acid-catalyzed formation of an indole from the second hydrazone via what is known in the art as the Fischer Indole synthesis:

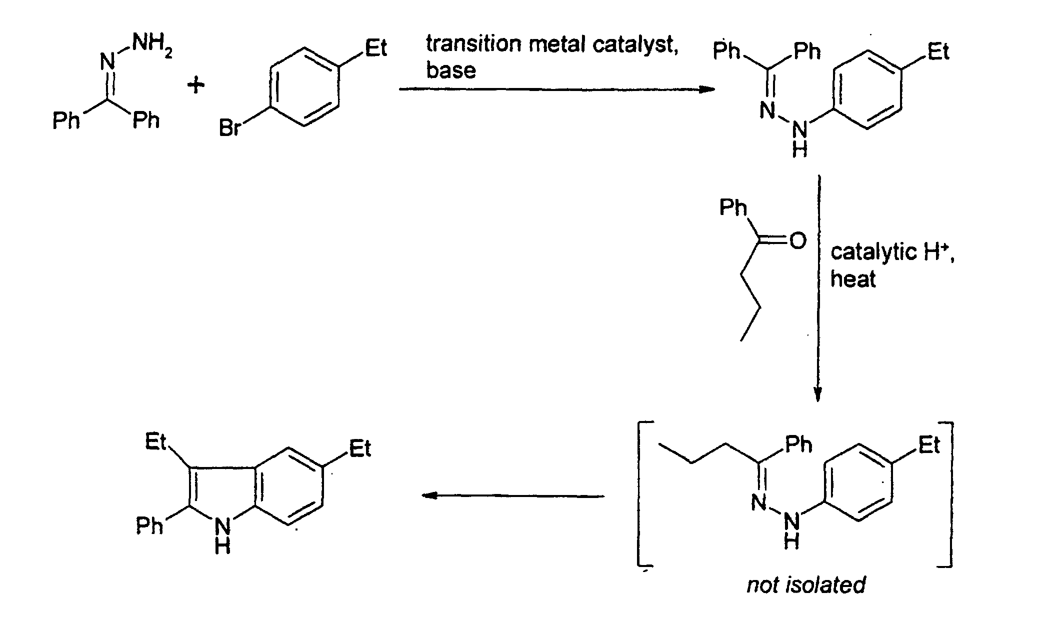

In another preferred illustrative embodiment, the subject arylation of an oxime is the initial step in a novel benzofuran synthesis. The entire sequence of events in this novel strategy for benzofuran synthesis is illustrated below. The second step in this strategy involves two discrete events: 1) an acid-catalyzed exchange of a new aldehyde or ketone for benzophenone to form a second *O*-aryl oxime -- labeled "not isolated" -- and benzophenone (not shown); and 2) the acid-catalyzed formation of an benzofuran from the second *O*-aryl oxime via a mechanism presumed to be analogous to that of the corresponding steps of the Fischer indole synthesis:

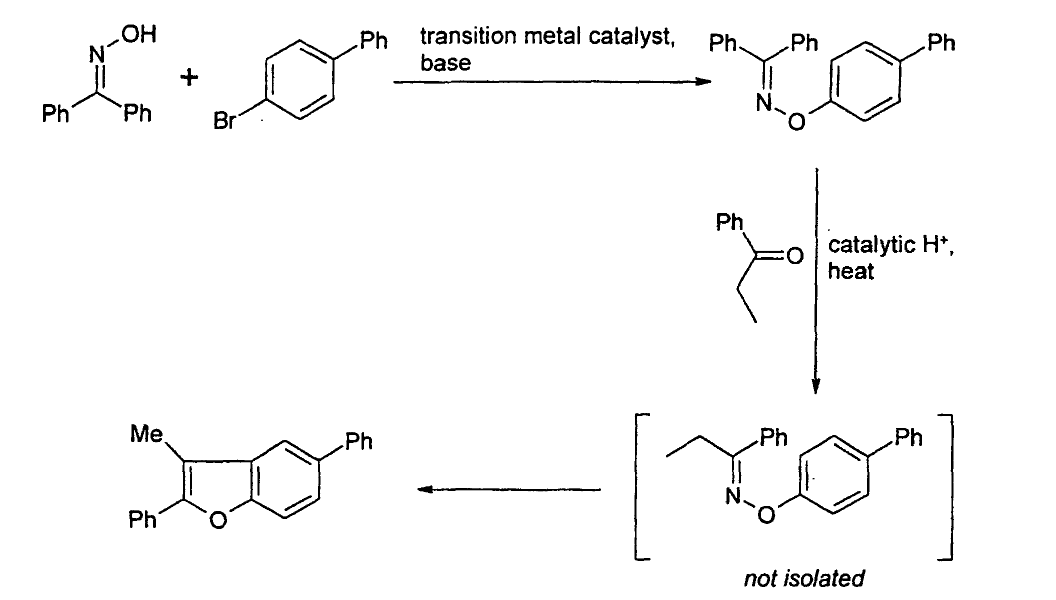

In another preferred illustrative embodiment, the subject arylation of benzophenone hydrazone is followed by a second subject arylation to give an *N*,*N*-diaryl benzophenone hydrazone. The subsequent step of this embodiment is the acid-catalyzed "exchange in" of a new carbonyl component to produce benzophenone and a new hydrazone (refer to the teachings of the preceding illustrative example); the new hydrazone then undergoes an acid-catalyzed Fischer indole synthesis reaction to give the product indole. The Fischer indole synthesis step in this embodiment is highly selective; the more electron-rich aryl moiety of the intermediate *N,N*-diaryl hydrazone is selectively incorporated into the indole nucleus of the product, and the less electron-rich aryl moiety appears as a substituent at the 1-position of the indole product.

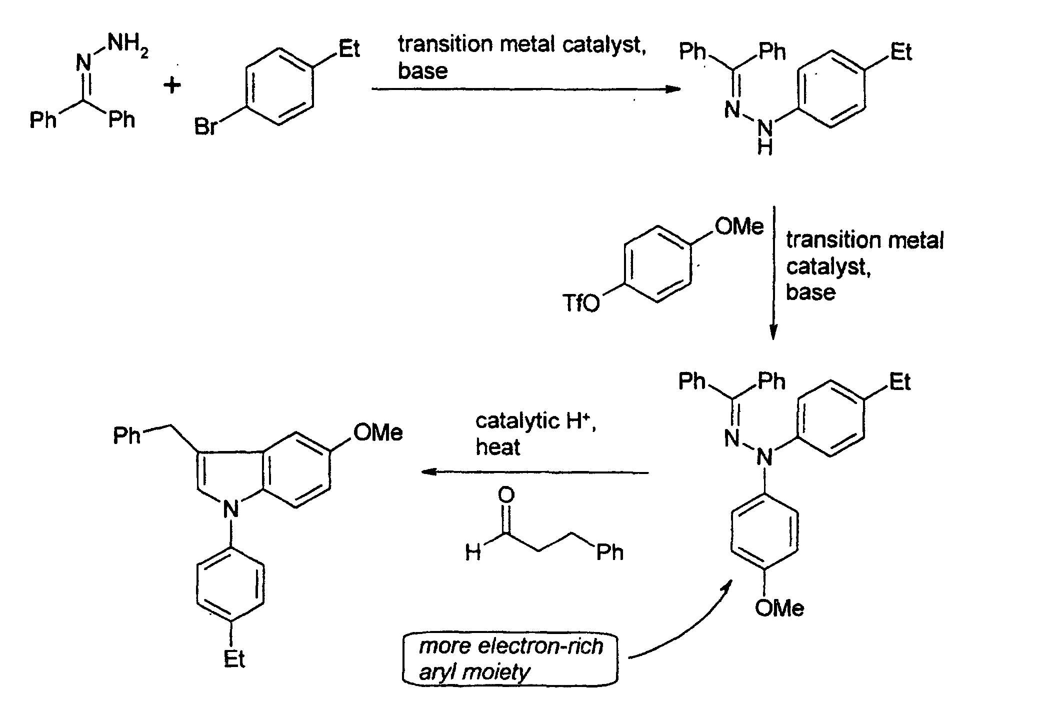

In an additional preferred embodiment, the arylation-Fischer Indole synthesis strategy is carried out on a solid support:

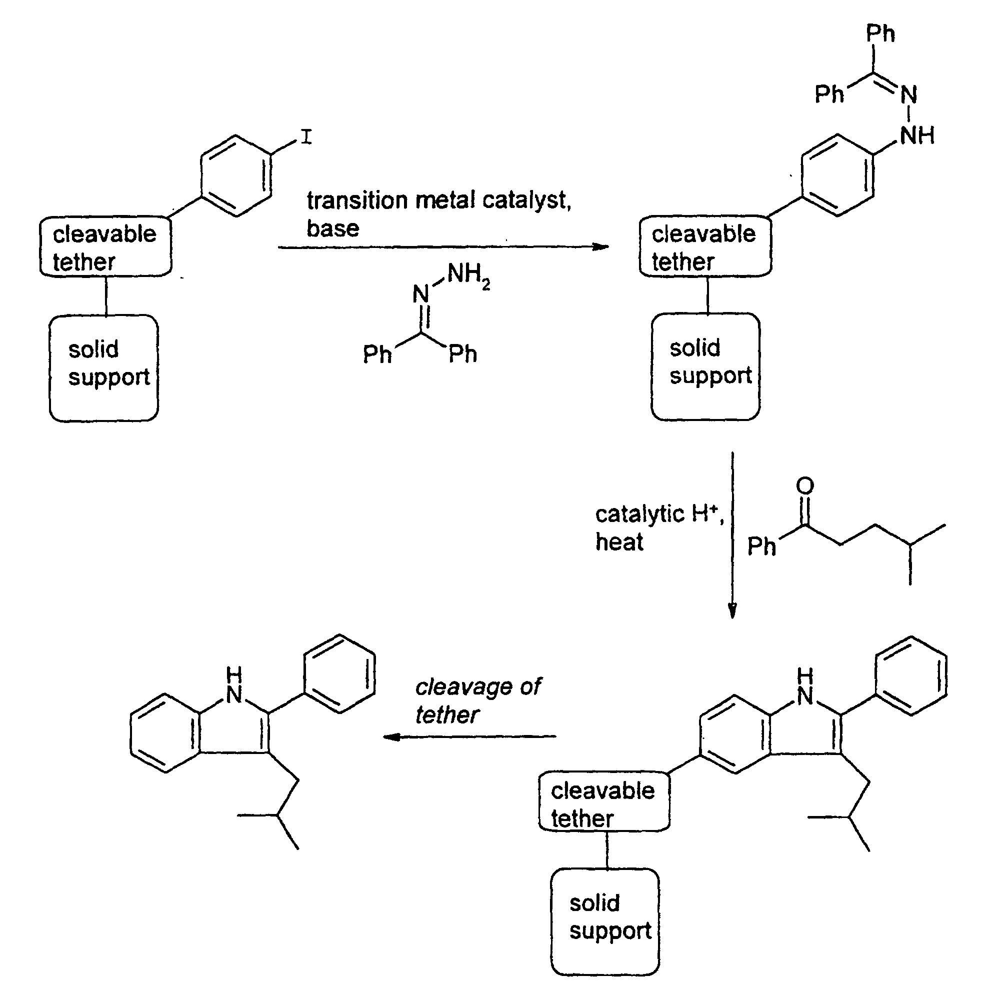

In a further preferred embodiment, a combinatorial approach to the arylation-Fischer Indole synthesis strategy on a solid support is pursued to yield a library of indoles:

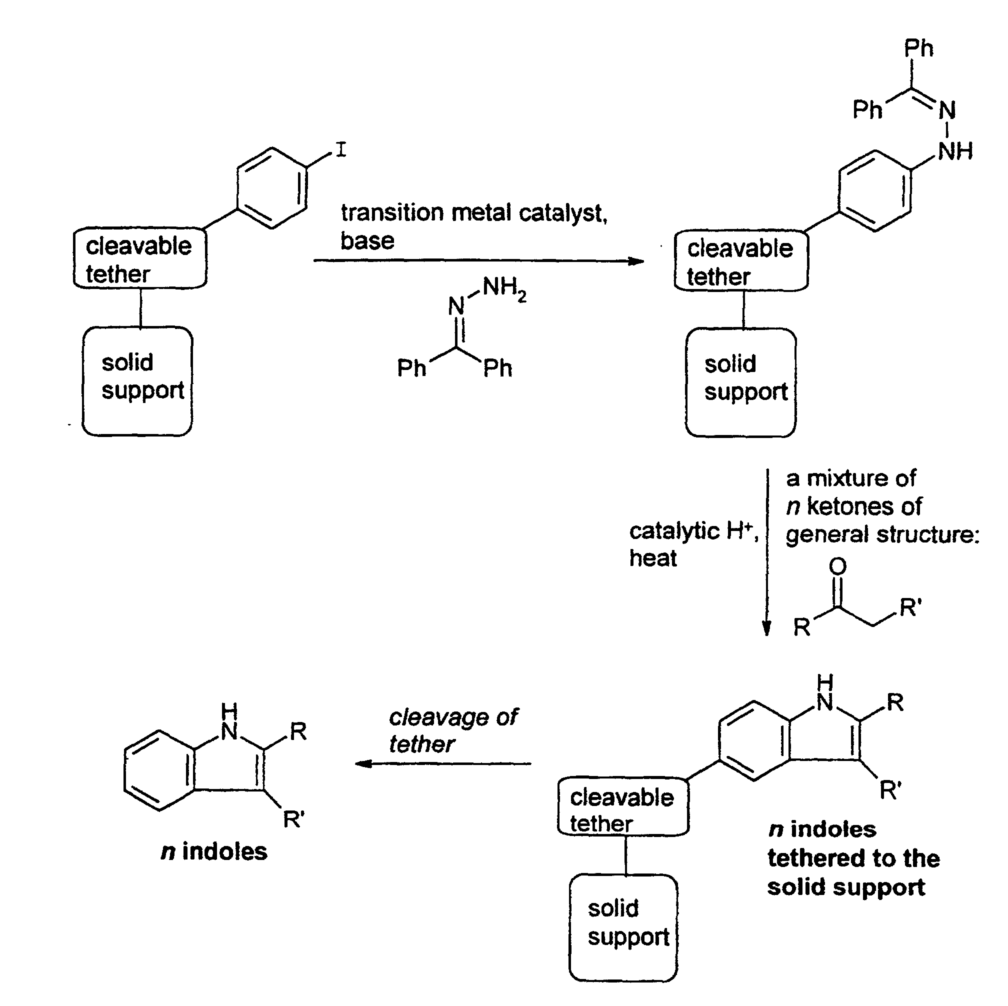

In another preferred embodiment, the subject arylation of an oxime can be followed by a step analogous to the Fischer indole synthesis to provide a benzofuran:

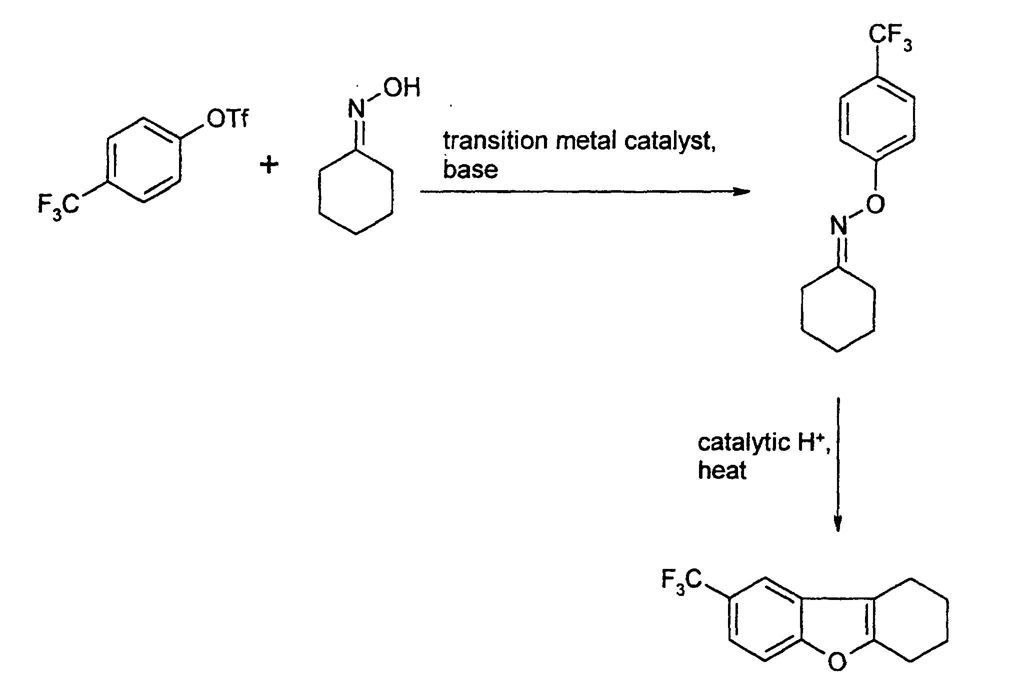

In a preferred embodiment, the subject vinylation of a hydrazone can be followed by a step analogous to the Fischer indole synthesis to provide a pyrrole:

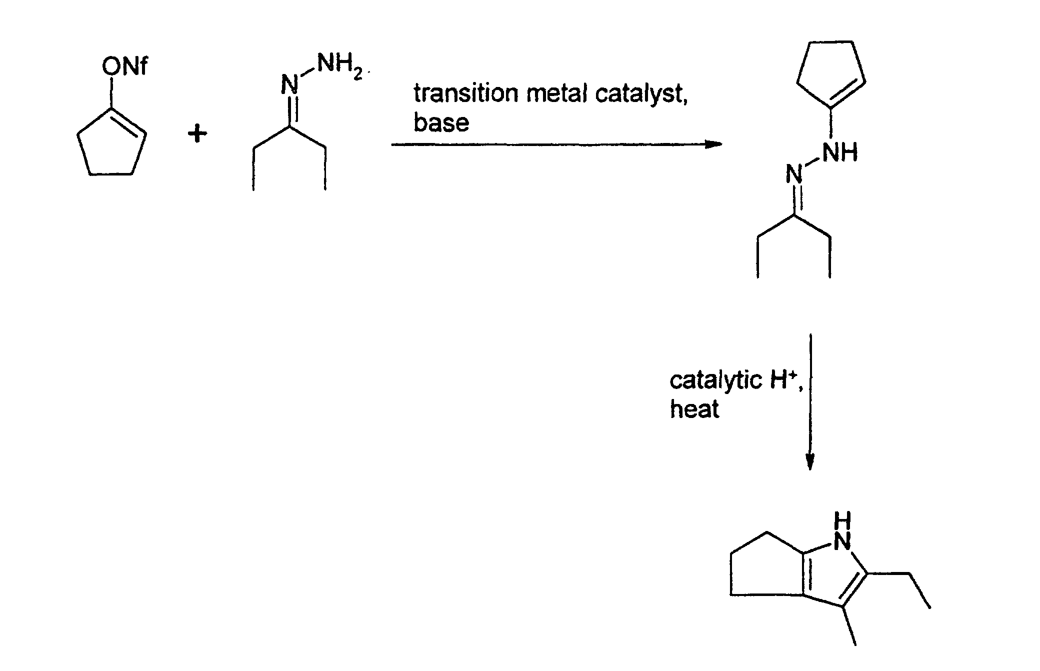

In a preferred embodiment, the subject vinylation of an oxime can be followed by a step analogous to the Fischer indole synthesis to provide a furan:

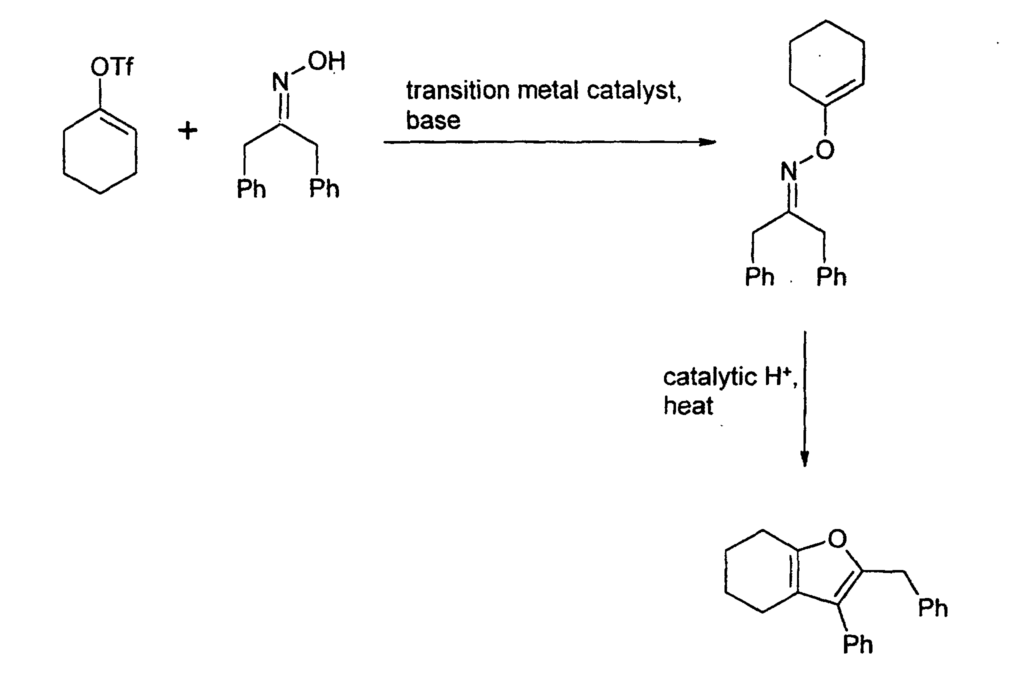

The substrate aryl compounds include compounds derived from simple aromatic rings (single or polycylic) such as benzene, naphthalene, anthracene and phenanthrene; or heteroaromatic rings (single or polycyclic), such as pyrrole, thiophene, thianthrene, furan, pyran, isobenzofuran, chromene, xanthene, phenoxathiin, pyrrole, imidazole, pyrazole, thiazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, perimidine, phenanthroline, phenazine, phenarsazine, phenothiazine, furazan, phenoxazine, pyrrolidine, oxolane, thiolane, oxazole, piperidine, piperazine, morpholine and the like. In certain embodiments, the reactive group, X, is substituted on a five, six or seven membered ring (though it can be part of a larger polycyle).

In certain embodiments, the aryl substrate may be selected from the group consisting of phenyl and phenyl derivatives, heteroaromatic compounds, polycyclic aromatic and heteroaromatic compounds, and functionalized derivatives thereof. Suitable aromatic compounds derived from simple aromatic rings and heteroaromatic rings, include but are not limited to, pyridine, imidizole, quinoline, furan, pyrrole, thiophene, and the like. Suitable aromatic compounds derived from fused ring systems, include but are not limited to naphthalene, anthracene, tetralin, indole and the like.

Suitable aromatic compounds may have the formula ZₚArX, where X is an activated substituent. An activated substituent, X, is characterized as being a good leaving group. In general, the leaving group is a group such as a halide or sulfonate. For the purposes of the present invention, an activated substituent is that moiety whose conjugate acid, HX, has a pKa of less than 5.0. Suitable activated substituents include, by way of example only, halides such as chloride, bromide and iodide, and sulfonate esters such as triflate, mesylate, nonaflate and tosylate. In certain embodiments, the leaving group is a halide selected from iodine and bromine. Chlorine and fluorine can also be used as leaving groups, though other electronegative substitution on the aryl group may be required to activate those halogens as leaving groups in the subject metal cross-coupling reactions.

Z represents one or more optional substituents on the aromatic ring, though each occurence of Z (p>1) is independently selected. By way of example only, each incidence of substitution independently can be, as valence and stability permit, a halogen, a lower alkyl, a lower alkenyl, a lower alkynyl, a carbonyl (e.g., an ester, a carboxylate, or a formate), a thiocarbonyl (e.g., a thiolester, a thiolcarboxylate, or a thiolformate), a ketyl, an aldehyde, an amino, an acylamino, an amido, an amidino, a cyano, a nitro, an azido, a sulfonyl, a sulfoxido, a sulfate, a sulfonate, a sulfamoyl, a sulfonamido, a phosphoryl, a phosphonate, a phosphinate, -(CH₂)ₘ-R₈, -(CH₂)ₘ-OH, -(CH₂)ₘ-O-lower alkyl, -(CH₂)ₘ-O-lower alkenyl, -(CH₂)ₘ-O-(CH₂)ₙ-R₈, -(CH₂)ₘ-SH, -(CH₂)ₘ-S-lower alkyl, -(CH₂)ₘ-S-lower alkenyl, -(CH₂)ₘ-S-(CH₂)ₙ-R₈, or protecting groups of the above or a solid or polymeric support; R₈ represents a substituted or unsubstituted aryl, aralkyl, cycloalkyl, cycloalkenyl, or heterocycle; and n and m are independently for each occurrence zero or an integer in the range of 1 to 6. P is preferably in the range of 0 to 5. For fused rings, where the number of substitution sites on the aryl group increases, p may be adjusted appropriately.

In certain embodiments, suitable substituents Z include alkyl, aryl, acyl, heteroaryl, amino, carboxylic ester, carboxylic acid, hydrogen group, ether, thioether, amide, carboxamide, nitro, phosphonic acid, hydroxyl, sulfonic acid, halide, pseudohalide groups, and substituted derivatives thereof, and p is in the range of 0 to 5. In particular, the reaction has been found compatible with acetals, amides and silyl ethers as functional groups. For fused rings, where the number of substitution sites on the aromatic ring increases, p may be adjusted appropriately. In addition, the above mentioned moieties may be covalently linked to an alcohol moiety in intramolecular reactions.

In certain embodiments, the resonance structure of the aryl group Ar, or at least one substituent Z, is electron-withdrawing from the substituted position of X.

A wide variety of substrate aryl groups are useful in the methods of the present invention. The choice of substrate will depend on factors such as the hydrazine or the like to be employed and the desired product, and an appropriate aryl substrate will be apparent to the skilled artisan. It will be understood that the aryl substrate preferably will not contain any interfering functionalities. It will further be understood that not all activated aryl substrates will react with every hydrazine.

The reactive hydrazine group or the like can be a molecule separate from the substrate aryl group, or a substituent of the same molecule (e.g., in an intramolecular embodiment).

The hydrazine or the like is selected to provide the desired reaction product. In general, the hydrazine or the like may be selected from the set comprising hydrazines, hydrazones, hydroxylamines and the like. The hydrazine or the like may be functionalized. The hydrazine or the like may be selected from a wide variety of structural types, including but not limited to, acyclic, cyclic or heterocyclic compounds, fused ring compounds or phenol derivatives. The aromatic compound and the hydrazine or the like may be included as moieties of a single molecule, whereby the arylation reaction proceeds as an intramolecular reaction.

In certain embodiments, the reactive hydrazine group or the like which is used in the subject coupling reaction can be represented by general formula RHN-Y or HY-NR₂. R represents, as valence and stability permit, a substituted or unsubstituted alkyl or alkenyl group, or -(CH₂)ₘ-R₈, wherein R₈ represents a substituted or unsubstituted aryl, cycloalkyl, cycloalkenyl, heterocycle or polycycle, and m is zero or an integer in the range of 1 to 8. In other embodiments, R is linker to a solid support.

In certain embodiments, the hydrazine or the like is generated *in situ* by conversion of a precursor under the reaction conditions, e.g. by deprotonation of a hydrohalide salt of the hydrazine or the like.

Alternatively, the corresponding hydrazide or the like salt, e.g., NaN(R)-Y, LiN(R)-Y, KN(R)-Y, NaY-NR₂, LiY-NR₂, KY-NR₂, etc., may be prepared and used in place of the hydrazine or the like. When the corresponding hydrazide or the like is used in the reaction, an additional base may not be required.

The active form of the transition metal catalyst is not well characterized. Therefore, it is contemplated that the "transition metal catalyst" of the present invention, as that term is used herein, shall include any catalytic transition metal and/or catalyst precursor as it is introduced into the reaction vessel and which is, if necessary, converted *in situ* into the active form, as well as the active form of the catalyst which participates in the reaction.

In certain embodiments, the transition metal catalyst complex is provided in the reaction mixture is a catalytic amount. In certain embodiments, that amount is in the range of 0.0001 to 20 mol%, and preferably 0.05 to 5 mol%, and most preferably 1-3 mol%, with respect to the limiting reagent, which may be either the aromatic compound or the hydrazine or the like (or corresponding hydrazide etc.) or both, depending upon which reagent is in stoichiometric excess. In the instance where the molecular formula of the catalyst complex includes more than one metal, the amount of the catalyst complex used in the reaction may be adjusted accordingly. By way of example, Pd₂(dba)₃ has two metal centers; and thus the molar amount of Pd₂(dba)₃ used in the reaction may be halved without sacrifice to catalytic activity.

Additionally, heterogeneous catalysts containing forms of these elements are also suitable catalysts for any of the transition metal catalyzed reactions of the present invention. Catalysts containing palladium and/or nickel are preferred. It is expected that these catalysts will perform similarly because they are known to undergo similar reactions, namely oxidative-addition reactions and reductive-elimination reactions, which are thought to be involved in the formation of the aryl hydrazines etc. of the present invention. However, the different ligands are thought to modify the catalyst performance by, for example, modifying reactivity and preventing undesirable side reactions.

As suitable, the catalysts employed in the subject method involve the use of metals which can mediate cross-coupling of the aryl groups ArX and the hydrazine or the like as defined above. In general, any transition metal (e.g., having *d* electrons) may be used to form the catalyst, e.g., a metal selected from one of Groups 3-12 of the periodic table or from the lanthanide series. However, in certain embodiments, the metal will be selected from the group of late transition metals, e.g. preferably from Groups 5-12 and even more preferably Groups 7-11. For example, suitable metals include platinum, palladium, iron, nickel, ruthenium and rhodium. The particular form of the metal to be used in the reaction is selected to provide, under the reaction conditions, metal centers which are coordinately unsaturated and not in their highest oxidation state. The metal core of the catalyst should be a zero valent transition metal, such as Pd or Ni with the ability to undergo oxidative addition to Ar-X bond. The zero-valent state, M⁰, may be generated in situ from M⁺².

To further illustrate, suitable transition metal catalysts include soluble or insoluble complexes of platinum, palladium and nickel. Nickel and palladium are particularly preferred and palladium is most preferred. A zero-valent metal center is presumed to participate in the catalytic carbon-heteroatom bond forming sequence. Thus, the metal center is desirably in the zero-valent state or is capable of being reduced to metal(0). Suitable soluble palladium complexes include, but are not limited to, tris(dibenzylideneacetone) dipalladium [Pd₂(dba)₃], bis(dibenzylideneacetone) palladium [Pd(dba)₂] and palladium acetate. Suitable catalysts for a heterogeneous arylation reaction include, but are not limited to, palladium on carbon (Pd/C). Alternatively, particularly for nickel catalysts, the active species for the oxidative-addition step may be in the metal (+1) oxidative-addition state.

Catalysts containing palladium and nickel are preferred. It is expected that these catalysts will perform comparably because they are known to undergo similar reactions, namely cross-coupling reactions, which may be involved in the formation of the aryl hydrazines etc. of the present invention.

The coupling can be catalyzed by a palladium catalyst which may take the form of, to illustrate, PdCl₂, Pd(OAc)₂, (CH₃CN)₂PdCl₂, Pd[P(C₆H₅)₃]₄, and polymer supported Pd(0). In other embodiments, the reaction can be catalyzed by a nickel catalyst, such as Ni(acac)₂, NiCl₂[P(C₆H₅)]₂, Ni(1,5-cyclooctadiene)₂, Ni(1,10-phenanthroline)₂, Ni(dppf)₂, NiCl₂(dppf), NiCl₂(1,10-phenanthroline), Raney nickel and the like, wherein "acac" represents acetylacetonate.

The catalyst will preferably be provided in the reaction mixture as metal-ligand complex comprising a bound supporting ligand, that is, a metal-supporting ligand complex. The ligand effects can be key to favoring, *inter alia,* the reductive elimination pathway or the like which produces the hydrazine or the like, over such side reactions as β-hydride elimination. In particular, the use of bulky and less electron-donating ligands (but probably still chelating ligands) should favor the reductive elimination process. In preferred embodiments, the subject reaction employs bulky bidentate ligands such as bisphosphines. The ligand, if chiral can be provided as a racemic mixture or a purified stereoisomer. In certain instances, e.g. the improved method for the synthesis of aryl amines, the use of a racemic, chelating bisphosphine is preferred.

The ligand, as described in greater detail below, may include chelating ligands, such as by way of example only, alkyl and aryl derivatives of phosphines and bisphosphines, amines, diamines, imines, arsines, and hybrids thereof, including hybrids of phosphines with amines. Weakly or non-nucleophilic stabilizing ions are preferred to avoid complicating side reaction of the counter ion attacking or adding to the electrophilic center of the substrate aryl. This catalyst complex may include additional ligands as is necessary to obtain a stable complex. Moreover, the ligand can be added to the reaction mixture in the form of a metal complex, or added as a separate reagent relative to the addition of the metal. By way of example, PdCl₂(BINAP) may be prepared in a separate step and used as the catalyst complex set forth in any of the methods of the invention.

The supporting ligand may be added to the reaction solution as a separate compound or it may be complexed to the metal center to form a metal-supporting ligand complex prior to its introduction into the reaction solution. Supporting ligands are compounds added to the reaction solution which are capable of binding to the catalyst metal center, although an actual metal-supporting ligand complex has not been identified in each and every synthesis. In some certain embodiments, the supporting ligand is a chelating ligand. Although not bound by any theory of operation, it is hypothesized that the supporting ligands prevent unwanted side reactions as well as enhancing the rate and efficiency of the desired process. Additionally, they typically prevent precipitation of the catalytic transition metal. Although the present invention does not require the formation of a metal-supporting ligand complex, such complexes have been shown to be consistent with the postulate that they are intermediates in these reactions and it has been observed the selection of the supporting ligand has an affect on the course of the reaction.

The supporting ligand is present in the range of 0.0001 to 40 mol% relative to the limiting reagent, i.e., hydrazine etc. or aromatic compound. The ratio of the supporting ligand to catalyst complex is typically in the range of about 1 to 20, and preferably in the range of about 1 to 4 and most preferably 2. These ratios are based upon a single metal complex and a single binding site ligand. In instances where the ligand contains additional binding sites (i.e., a chelating ligand) or the catalyst contains more than one metal, the ratio is adjusted accordingly. By way of example, the supporting ligand BINAP contains two coordinating phosphorus atoms and thus the ratio of BINAP to catalyst is adjusted downward to about 1 to 10, preferably about 1 to 2 and most preferably 1. Conversely, Pd₂(dba)₃ contains two palladium metal centers and the ratio of a non-chelating ligand to Pd₂(dba)₃ is adjusted upward to 1 to 40, preferably 1 to 8 and most preferably 4.

In certain embodiments of the subject method, the transition metal catalyst includes one or more phosphine ligands, e.g., as a Lewis basic ligand that controls the stability and electron transfer properties of the transition metal catalyst, and/or stabilizes the metal intermediates. Phosphine ligands are commercially available or can be prepared by methods similar to processes known per se. The phosphines can be monodentate phosphine ligands, such as trimethylphosphine, triethylphosphine, tripropylphosphine, triisopropylphosphine, tributylphosphine, tricyclohexylphosphine, trimethyl phosphite, triethyl phosphite, tripropyl phosphite, triisopropyl phosphite, tributyl phosphite and tricyclohexyl phosphite, in particular triphenylphosphine, tri(*o*-tolyl)phosphine, triisopropylphosphine or tricyclohexylphosphine; or a bidentate phosphine ligand such as 2,2'-bis(diphenylphosphino)-1,1'-binaphthyl (BINAP), 1,2-bis(dimethylphosphino)ethane, 1,2-bis(diethylphosphino)ethane, 1,2-bis(dipropylphosphino)ethane, 1,2-bis(diisopropylphosphino)ethane, 1,2-bis(dibutylphosphino)ethane, 1,2-bis(dicyclohexylphosphino)ethane, 1,3-bis(dicyclohexylphosphino)propane, 1,3-bis(diiso-propylphosphino)propane, 1,4-bis(diisopropylphosphino)-butane and 2,4-bis(dicyclohexylphosphino)pentane.

In certain embodiments, the phosphine ligand is P(*o*-tolyl)₃. Bis(phosphine) ligands are particularly preferred chelating supporting ligands. Suitable bis(phosphine) compounds include but are in no way limited to (±)-2,2'-bis(diphenylphosphino)-1,1'-binaphthyl (and separate enantiomers), (±)-2,2'-bis(di-*p*-tolylphosphino)-1,1'-binaphthyl (and separate enantiomers), 1-1'-bis(diphenylphosphino)ferrocene (dppf), 1,3-bis(diphenylphosphino)propane (dppp), 1,2-bis(diphenylphosphino)benzene, 2,2'-bis(diphenylphosphino)diphenyl ether, 9,9-dimethyl-4,5-bis(diphenylphosphino)xanthene (xantphos), and 1,2-bis(diphenylphosphino)ethane (dppe). Hybrid chelating ligands such as (±)-N,N-dimethyl-1-[2-(diphenylphosphino)ferrocenyl]ethylamine (and separate enantiomers), and (±)-(R)-1-[(S)-2-(diphenylphosphino)-ferrocenyl]ethyl methyl ether (and separate enantiomers) are also within the scope of the invention.

In some instances, it may be necessary to include additional reagents in the reaction to promote reactivity of either the transition metal catalyst or activated aryl nucleus. In particular, it may be advantageous to include a suitable base. In general, a variety of bases may be used in practice of the present invention. It has not been determined if deprotonation occurs prior to or after heteroatom coordination to the transition metal of the active catalyst. The base may optionally be sterically hindered to discourage metal coordination of the base in those circumstances where such coordination is possible, i.e., alkali metal alkoxides. Exemplary bases include such as, for example: an alkoxides such as sodium tert-butoxide, an alkali metal amide such as sodium amide, lithium diisopropylamide or an alkali metal bis(trialkylsilyl)amides, e.g., such as lithium bis(trimethylsilyl)amide or sodium bis(trimethylsilyl) amide, a tertiary amine (e.g. triethylamine, trimethylamine, 4-(dimethylamino)pyridine (DMAP), 1,5-diazabicycl[4.3.0]non-5-ene (DBN), 1,5-diazabicyclo[5.4.0]undec-5-ene (DBU), alkali, alkaline earth carbonate, bicarbonate or hydroxide (e.g. sodium, magnesium, calcium, barium, potassium carbonate, hydroxide and bicarbonate). By way of example only, suitable bases include NaH, LiH, KH, K₂CO₃, Na₂CO₃, Tl₂CO₃, Cs₂CO₃, K(O*t*Bu), Li(O*t*Bu), Na(O*t*Bu) K(OPh), Na(OPh), triethylamine or mixtures thereof. NaH, Na(O*t*Bu) and K₂CO₃ have been found useful in a wide variety of aryl ether bond forming reactions. Preferred bases include Cs₂CO₃, DBU, NaH, KOt-Bu, LiN(*i*-Pr)₂ (LDA), KN(SiMe₃)₂, NaN(SiMe₃)₂, and LiN(SiMe₃)₂.

Base is used in approximately stoichiometric proportions in reactions using hydrazines etc. The present invention has demonstrated that there is no need for large excesses of base in order to obtain good yields of aryl hydrazines etc. under mild reaction conditions. No more than four equivalents and preferably no more than two equivalents are needed. Further, in reactions using the corresponding hydrazide etc. as the reagent, there may be no need for additional base.

In this way a wide range of aryl hydrazines, aryl hydrazones, *O*-aryl oximes, and *N*-and *O*-aryl hydroxylamines may be prepared from available hydrazines, hydrazones, oximes, and hydroxylamines and the corresponding hydrohalide salts. The reaction can be accomplished using a wide range of hydrazines and the like, which are either commercially available or obtainable from conventional syntheses using a variety of methods known in the art.

As is clear from the above discussion, the products which may be produced by the arylation reaction of this invention can undergo further reaction(s) to afford desired derivatives thereof. Such permissible derivatization reactions can be carried out in accordance with conventional procedures known in the art. For example, potential derivatization reactions include esterification, oxidation of alcohols to aldehydes and acids, *N*-alkylation of amides, nitrile reduction, acylation of alcohols by esters, acylation of amines and the like.

### Reaction Conditions

The arylation reactions of the present invention may be performed under a wide range of conditions, though it will be understood that the solvents and temperature ranges recited herein are not limitative and only correspond to a preferred mode of the process of the invention.

In general, it will be desirable that reactions are run using mild conditions which will not adversely affect the reactants, the catalyst, or the product. For example, the reaction temperature influences the speed of the reaction, as well as the stability of the reactants and catalyst. The reactions will usually be run at temperatures in the range of 25°C to 300°C, more preferably in the range 25°C to 150°C.

In general, the subject reactions are carried out in a liquid reaction medium. The reactions may be run without addition of solvent. Alternatively, the reactions may be run in an inert solvent, preferably one in which the reaction ingredients, including the catalyst, are substantially soluble. Suitable solvents include ethers such as diethyl ether, 1,2-dimethoxyethane, diglyme, t-butyl methyl ether, tetrahydrofuran and the like; halogenated solvents such as chloroform, dichloromethane, dichloroethane, chlorobenzene, and the like; aliphatic or aromatic hydrocarbon solvents such as benzene, xylene, toluene, hexane, pentane and the like; esters and ketones such as ethyl acetate, acetone, and 2-butanone; polar aprotic solvents such as acetonitrile, dimethylsulfoxide, dimethylformamide and the like; or combinations of two or more solvents.

The invention also contemplates reaction in a biphasic mixture of solvents, in an emulsion or suspension, or reaction in a lipid vesicle or bilayer. In certain embodiments, it may be preferred to perform the catalyzed reactions in the solid phase with one of the reactants anchored to a solid support.

In certain embodiments it is preferable to perform the reactions under an inert atmosphere of a gas such as nitrogen or argon.

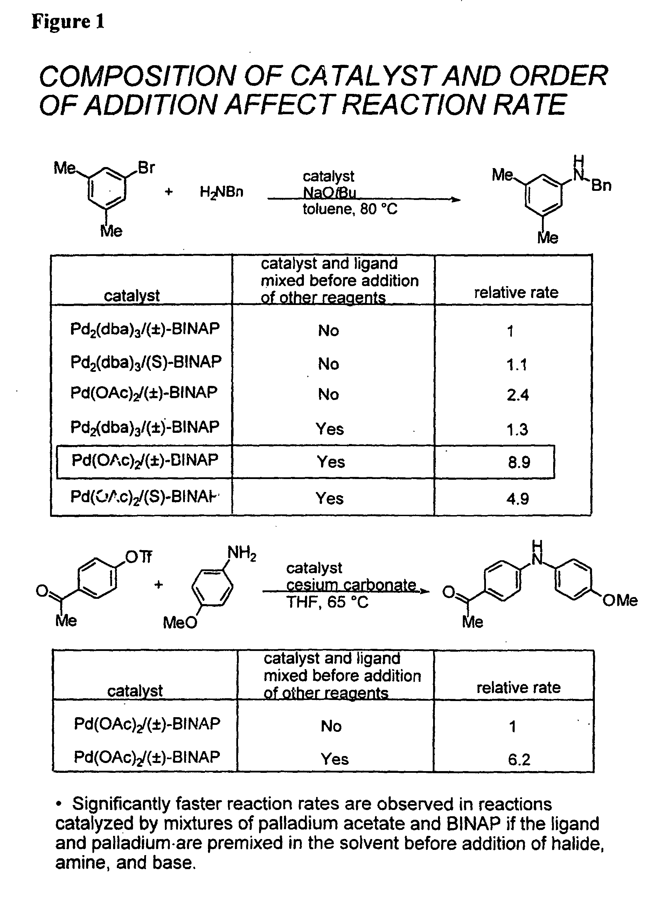

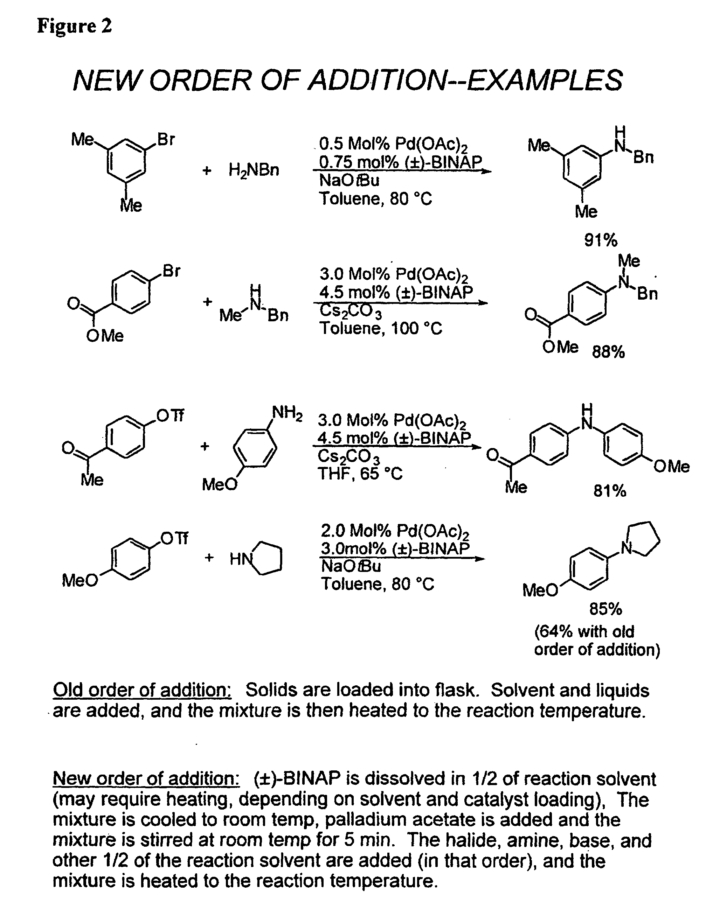

The reaction processes of the present invention can be conducted in continuous, semi-continuous or batch fashion and may involve a liquid recycle operation as desired. The processes of this invention are preferably conducted in batch fashion. Likewise, the manner or order of addition of the reaction ingredients, catalyst and solvent are also not generally critical to the success of the reaction, and may be accomplished in any conventional fashion. In a preferred order of events that leads to an enhancement of the reaction rate, the base, e.g. *t*-BuONa, is the last ingredient to be added to the reaction mixture (see Figures 1 and 2).

As disclosed in detail in the exemplification, a preferred protocol for the amination of aryl halides is provided herein. This preferred protocol comprises combining the transition metal and ligand prior to the addition of the other reagents. This highly preferred method proceeds with rate enhancements of more than a factor of two, more preferably more than a factor of four, and even more preferably more than a factor of six, relative to the rates obtained in the standard protocol (see U.S Patent 5,576,460). Furthermore, in certain instances this highly preferred protocol for the amination of aryl halides provides the product aryl amines in higher yield than provided by the standard protocol (see U.S Patent 5,576,460). This invention contemplates the application of this order of events to all of the subject transition metal-catalyzed arylation and vinylation reactions, and anticipates corresponding increases in reaction rate and efficiency thereof.

The reaction can be conducted in a single reaction zone or in a plurality of reaction zones, in series or in parallel or it may be conducted batchwise or continuously in an elongated tubular zone or series of such zones. The materials of construction employed should be inert to the starting materials during the reaction and the fabrication of the equipment should be able to withstand the reaction temperatures and pressures. Means to introduce and/or adjust the quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one of the starting materials to the other. Also, the reaction steps can be combined by the joint addition of the starting materials to the metal catalyst. When complete conversion is not desired or not obtainable, the starting materials can be separated from the product and then recycled back into the reaction zone.

The processes may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

Furthermore, one or more of the reactants can be immobilized or incorporated into a polymer or other insoluble matrix by, for example, derivativation with one or more of substituents of the aryl group.

### Combinatorial Libraries

The subject reactions readily lend themselves to the creation of combinatorial libraries of compounds for the screening of pharmaceutical, agrochemical or other biological or medically-related activity or material-related qualities. A combinatorial library for the purposes of the present invention is a mixture of chemically related compounds which may be screened together for a desired property; said libraries may be in solution or covalently linked to a solid support. The preparation of many related compounds in a single reaction greatly reduces and simplifies the number of screening processes which need to be carried out. Screening for the appropriate biological, pharmaceutical, agrochemical or physical property may be done by conventional methods.

Diversity in a library can be created at a variety of different levels. For instance, the substrate aryl groups used in a combinatorial approach can be diverse in terms of the core aryl moiety, e.g., a variegation in terms of the ring structure, and/or can be varied with respect to the other substituents.

A variety of techniques are available in the art for generating combinatorial libraries of small organic molecules. See, for example, Blondelle et al. (1995) Trends Anal. Chem. 14:83; the Affymax U.S. Patents 5,359,115 and 5,362,899: the Ellman U.S. Patent 5,288,514: the Still et al. PCT publication WO 94/08051; Chen et al. (1994) JACS 116:2661: Kerr et al. (1993) JACS 115:252; PCT publications WO92/10092, WO93/09668 and WO91/07087; and the Lerner et al. PCT publication WO93/20242). Accordingly, a variety of libraries on the order of about 16 to 1,000,000 or more diversomers can be synthesized and screened for a particular activity or property.

In an exemplary embodiment, a library of substituted diversomers can be synthesized using the subject reactions adapted to the techniques described in the Still et al. PCT publication WO 94/08051, e.g., being linked to a polymer bead by a hydrolyzable or photolyzable group, e.g., located at one of the positions of substrate. According to the Still et al. technique, the library is synthesized on a set of beads, each bead including a set of tags identifying the particular diversomer on that bead. In one embodiment, which is particularly suitable for discovering enzyme inhibitors, the beads can be dispersed on the surface of a permeable membrane, and the diversomers released from the beads by lysis of the bead linker. The diversomer from each bead will diffuse across the membrane to an assay zone, where it will interact with an enzyme assay. Detailed descriptions of a number of combinatorial methodologies are provided below.

### A) Direct Characterization

A growing trend in the field of combinatorial chemistry is to exploit the sensitivity of techniques such as mass spectrometry (MS), e.g., which can be used to characterize sub-femtomolar amounts of a compound, and to directly determine the chemical constitution of a compound selected from a combinatorial library. For instance, where the library is provided on an insoluble support matrix, discrete populations of compounds can be first released from the support and characterized by MS. In other embodiments, as part of the MS sample preparation technique, such MS techniques as MALDI can be used to release a compound from the matrix, particularly where a labile bond is used originally to tether the compound to the matrix. For instance, a bead selected from a library can be irradiated in a MALDI step in order to release the diversomer from the matrix, and ionize the diversomer for MS analysis.

### B) Multipin Synthesis

The libraries of the subject method can take the multipin library format. Briefly, Geysen and co-workers (Geysen et al. (1984) PNAS 81:3998-4002) introduced a method for generating compound libraries by a parallel synthesis on polyacrylic acid-grated polyethylene pins arrayed in the microtitre plate format. The Geysen technique can be used to synthesize and screen thousands of compounds per week using the multipin method, and the tethered compounds may be reused in many assays. Appropriate linker moieties can also been appended to the pins so that the compounds may be cleaved from the supports after synthesis for assessment of purity and further evaluation (c.f., Bray et al. (1990) Tetrahedron Lett 31:5811-5814; Valerio et al. (1991) Anal Biochem 197:168-177; Bray et al. (1991) Tetrahedron Lett 32:6163-6166).

### C) Divide-Couple-Recombine

In yet another embodiment, a variegated library of compounds can be provided on a set of beads utilizing the strategy of divide-couple-recombine (see, e.g., Houghten (1985) PNAS 82:5131-5135; and U.S. Patents 4,631,211; 5,440,016; 5,480,971). Briefly, as the name implies, at each synthesis step where degeneracy is introduced into the library, the beads are divided into separate groups equal to the number of different substituents to be added at a particular position in the library, the different substituents coupled in separate reactions, and the beads recombined into one pool for the next iteration.

In one embodiment, the divide-couple-recombine strategy can be carried out using an analogous approach to the so-called "tea bag" method first developed by Houghten, where compound synthesis occurs on resin sealed inside porous polypropylene bags (Houghten et al. (1986) PNAS 82:5131-5135). Substituents are coupled to the compound-bearing resins by placing the bags in appropriate reaction solutions, while all common steps such as resin washing and deprotection are performed simultaneously in one reaction vessel. At the end of the synthesis, each bag contains a single compound.

### D) Combinatorial Libraries by Light-Directed, Spatially Addressable Parallel Chemical Synthesis

A scheme of combinatorial synthesis in which the identity of a compound is given by its locations on a synthesis substrate is termed a spatially-addressable synthesis. In one embodiment, the combinatorial process is carried out by controlling the addition of a chemical reagent to specific locations on a solid support (Dower et al. (1991) Annu Rep Med Chem 26:271-280; Fodor, S.P.A. (1991) Science 251:767; Pirrung et al. (1992) U.S. Patent No. 5,143,854; Jacobs et al. (1994) Trends Biotechnol 12:19-26). The spatial resolution of photolithography affords miniaturization. This technique can be carried out through the use protection/deprotection reactions with photolabile protecting groups.

The key points of this technology are illustrated in Gallop et al. (1994) J Med Chem 37:1233-1251. A synthesis substrate is prepared for coupling through the covalent attachment of photolabile nitroveratryloxycarbonyl (NVOC) protected amino linkers or other photolabile linkers. Light is used to selectively activate a specified region of the synthesis support for coupling. Removal of the photolabile protecting groups by light (deprotection) results in activation of selected areas. After activation, the first of a set of amino acid analogs, each bearing a photolabile protecting group on the amino terminus, is exposed to the entire surface. Coupling only occurs in regions that were addressed by light in the preceding step. The reaction is stopped, the plates washed, and the substrate is again illuminated through a second mask, activating a different region for reaction with a second protected building block. The pattern of masks and the sequence of reactants define the products and their locations. Since this process utilizes photolithography techniques, the number of compounds that can be synthesized is limited only by the number of synthesis sites that can be addressed with appropriate resolution. The position of each compound is precisely known; hence, its interactions with other molecules can be directly assessed.

In a light-directed chemical synthesis, the products depend on the pattern of illumination and on the order of addition of reactants. By varying the lithographic patterns, many different sets of test compounds can be synthesized simultaneously; this characteristic leads to the generation of many different masking strategies.

### E) Encoded Combinatorial Libraries

In yet another embodiment, the subject method utilizes a compound library provided with an encoded tagging system. A recent improvement in the identification of active compounds from combinatorial libraries employs chemical indexing systems using tags that uniquely encode the reaction steps a given bead has undergone and, by inference, the structure it carries. Conceptually, this approach mimics phage display libraries, where activity derives from expressed peptides, but the structures of the active peptides are deduced from the corresponding genomic DNA sequence. The first encoding of synthetic combinatorial libraries employed DNA as the code. A variety of other forms of encoding have been reported, including encoding with sequenceable bio-oligomers (e.g., oligonucleotides and peptides), and binary encoding with additional non-sequenceable tags.

### 1) Tagging with sequenceable bio-oligomers

The principle of using oligonucleotides to encode combinatorial synthetic libraries was described in 1992 (Brenner et al. (1992) PNAS 89:5381-5383), and an example of such a library appeared the following year (Needles et al. (1993) PNAS 90:10700-10704). A combinatorial library of nominally 7⁷ (= 823,543) peptides composed of all combinations of Arg, Gln, Phe, Lys, Val, D-Val and Thr (three-letter amino acid code), each of which was encoded by a specific dinucleotide (TA, TC, CT, AT, TT, CA and AC, respectively), was prepared by a series of alternating rounds of peptide and oligonucleotide synthesis on solid support. In this work, the amine linking functionality on the bead was specifically differentiated toward peptide or oligonucleotide synthesis by simultaneously preincubating the beads with reagents that generate protected OH groups for oligonucleotide synthesis and protected NH₂ groups for peptide synthesis (here, in a ratio of 1:20). When complete, the tags each consisted of 69-mers, 14 units of which carried the code. The bead-bound library was incubated with a fluorescently labeled antibody, and beads containing bound antibody that fluoresced strongly were harvested by fluorescence-activated cell sorting (FACS). The DNA tags were amplified by PCR and sequenced, and the predicted peptides were synthesized. Following such techniques, compound libraries can be derived for use in the subject method, where the oligonucleotide sequence of the tag identifies the sequential combinatorial reactions that a particular bead underwent, and therefore provides the identity of the compound on the bead.

The use of oligonucleotide tags permits exquisitely sensitive tag analysis. Even so, the method requires careful choice of orthogonal sets of protecting groups required for alternating co-synthesis of the tag and the library member. Furthermore, the chemical lability of the tag, particularly the phosphate and sugar anomeric linkages, may limit the choice of reagents and conditions that can be employed for the synthesis of non-oligomeric libraries. In certain embodiments, the libraries employ linkers permitting selective detachment of the test compound library member for assay.

Peptides have also been employed as tagging molecules for combinatorial libraries. Two exemplary approaches are described in the art, both of which employ branched linkers to solid phase upon which coding and ligand strands are alternately elaborated. In the first approach (Kerr JM et al. (1993) J Am Chem Soc 115:2529-2531), orthogonality in synthesis is achieved by employing acid-labile protection for the coding strand and base-labile protection for the compound strand.

In an alternative approach (Nikolaiev et al. (1993) Pept Res 6:161-170), branched linkers are employed so that the coding unit and the test compound can both be attached to the same functional group on the resin. In one embodiment, a cleavable linker can be placed between the branch point and the bead so that cleavage releases a molecule containing both code and the compound (Ptek et al. (1991) Tetrahedron Lett 32:3891-3894). In another embodiment, the cleavable linker can be placed so that the test compound can be selectively separated from the bead, leaving the code behind. This last construct is particularly valuable because it permits screening of the test compound without potential interference of the coding groups. Examples in the art of independent cleavage and sequencing of peptide library members and their corresponding tags has confirmed that the tags can accurately predict the peptide structure.

### 2) Non-sequenceable Tagging: Binary Encoding

An alternative form of encoding the test compound library employs a set of non-sequencable electrophoric tagging molecules that are used as a binary code (Ohlmeyer et al. (1993) PNAS 90:10922-10926). Exemplary tags are haloaromatic alkyl ethers that are detectable as their trimethylsilyl ethers at less than femtomolar levels by electron capture gas chromatography (ECGC). Variations in the length of the alkyl chain, as well as the nature and position of the aromatic halide substituents, permit the synthesis of at least 40 such tags, which in principle can encode 2⁴⁰ (e.g., upwards of 10¹²) different molecules. In the original report (Ohlmeyer et al., supra) the tags were bound to about 1% of the available amine groups of a peptide library via a photocleavable o-nitrobenzyl linker. This approach is convenient when preparing combinatorial libraries of peptide-like or other amine-containing molecules. A more versatile system has, however, been developed that permits encoding of essentially any combinatorial library. Here, the compound would be attached to the solid support via the photocleavable linker and the tag is attached through a catechol ether linker via carbene insertion into the bead matrix (Nestler et al. (1994) J Org Chem 59:4723-4724). This orthogonal attachment strategy permits the selective detachment of library members for assay in solution and subsequent decoding by ECGC after oxidative detachment of the tag sets.

Although several amide-linked libraries in the art employ binary encoding with the electrophoric tags attached to amine groups, attaching these tags directly to the bead matrix provides far greater versatility in the structures that can be prepared in encoded combinatorial libraries. Attached in this way, the tags and their linker are nearly as unreactive as the bead matrix itself. Two binary-encoded combinatorial libraries have been reported where the electrophoric tags are attached directly to the solid phase (Ohlmeyer et al. (1995) PNAS 92:6027-6031) and provide guidance for generating the subject compound library. Both libraries were constructed using an orthogonal attachment strategy in which the library member was linked to the solid support by a photolabile linker and the tags were attached through a linker cleavable only by vigorous oxidation. Because the library members can be repetitively partially photoeluted from the solid support, library members can be utilized in multiple assays. Successive photoelution also permits a very high throughput iterative screening strategy: first, multiple beads are placed in 96-well microtiter plates; second, compounds are partially detached and transferred to assay plates; third, a metal binding assay identifies the active wells; fourth, the corresponding beads are rearrayed singly into new microtiter plates; fifth, single active compounds are identified; and sixth, the structures are decoded.

### Exemplification

The invention may be understood with reference to the following examples, which are presented for illustrative purposes only and which are non-limiting. Hydrazines, hydrazones, ketones and aromatic halides utilized as substrates in these examples were either commercially available or were prepared in three or less steps from commercially available reagents. Palladium catalysts were all commercially available.

### Example 1

### Synthesis of N-Phenyl-N-4-tolyl-N'-trifluoroacetichydrazide

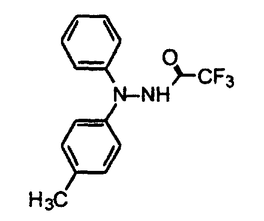

Phenylhydrazine (1.2 equiv, 0.6 mmol, 0.06 mL), 4-bromotoluene (1.0 equiv, 0.5 mmol, 0.06 mL), Pd(OAc)₂ (0.05 equiv, 0.025 mmol, 6 mg), BINAP (0.05 equiv, 0.025 mmol, 16 mg), NaO*t*Bu (1.4 equiv, 0.7 mmol, 67 mg) and diisopropylamine (2 mL) were added to an oven dried test tube which was capped with a septum and purged briefly with argon (∼ 1 min), and then heated to 80° C under argon for 1 hour. The reaction was then cooled to room temperature, diluted with Et₂O (2 mL), filtered through Celite and concentrated under vacuum. Trifluoroacetic anhydride (5 equiv, 2.5 mmol, 0.35 mL), triethylamine (5 equiv, 2.5 mmol, 0.35 mL), and CH₂Cl₂ were added to the crude reaction mixture and allowed to stir at room temperature for 1 hour. The reaction solution was then washed with a saturated NaHCO₃ solution (2 X 10 mL), dried over MgSO₄, filtered and concentrated under vacuum. The crude mixture was purified by flash column chromatography (10% EtOAc/Hex) to give the title product as a white solid (97 mg, 0.35 mmol, 70% yield).

### Example 2

### Synthesis of N-(4-Fluorophenyl)-N-(4-phenylphenyl)hydrazine

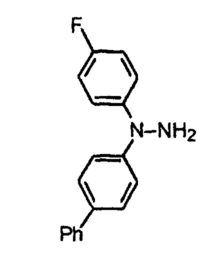

4-Fluorophenylhydrazine hydrochloride (1.2 equiv, 0.6 mmol, 98 mg), 4-bromobiphenyl (1.0 equiv, 0.5 mmol, 117 mg), Pd(OAc)₂ (0.05 equiv, 0.025 mmol, 6 mg), BINAP (0.05 equiv, 0.025 mmol, 16 mg), NaO*t*Bu (1.4 equiv, 0.7 mmol, 67 mg) and diisopropylamine (2 mL) were added to an oven dried test tube which was capped with a septum and purged briefly with argon (∼ 1 min), and then heated to 50° C under argon for 7 hours. The reaction was then cooled to room temperature, diluted with Et₂O (2 mL), filtered through Celite and concentrated under vacuum. The crude mixture was purified by flash column chromatography (10% EtOAc/Hex) to give the title product as a white solid (110 mg, 0.39 mmol, 79% yield).

### Example 3

### Synthesis of N-(4-Chlorophenyl)-N-phenylhydrazine

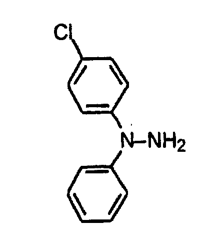

Phenylhydrazine (1.2 equiv, 0.6 mmol, 0.06 mL), 4-chlorobromobenzene (1.0 equiv, 0.5 mmol, 96 mg), Pd(OAc)₂ (0.05 equiv, 0.025 mmol, 6 mg), BINAP (0.05 equiv, 0.025 mmol, 16 mg), NaO*t*Bu (1.4 equiv, 0.7 mmol, 67 mg) and diisopropylamine (2 mL) were added to an oven dried test tube which was capped with a septum and purged briefly with argon (∼ 1 min), and then heated to 80° C under argon for 2.5 hours. The reaction was then cooled to room temperature, diluted with Et₂O (2 mL), filtered through Celite and concentrated under vacuum. The crude mixture was purified by flash column chromatography (10% EtOAc/Hex) to give the title product as a yellow oil (42 mg, 0.19 mmol, 39% yield).

### Example 4

### Synthesis of N-4-Benzotrifluoro-N-t-butyl carbazate

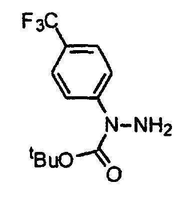

*t*-Butyl carbazate (1.2 equiv, 0.6 mmol, 79 mg), 4-bromobenzotrifluoride (1.0 equiv, 0.5 mmol, 0.07 mL), Pd(OAc)₂ (0.05 equiv, 0.025 mmol, 6 mg), BINAP (0.05 equiv, 0.025 mmol, 16 mg), Cs₂CO₃ (1.4 equiv, 0.7 mmol, 228 mg) and toluene (2 mL) were added to an oven dried test tube which was capped with a septum and purged briefly with argon (∼ 1 min), and then heated to 100° C under argon for 8 hours. The reaction was then cooled to room temperature, diluted with Et₂O (2 mL), filtered through Celite and concentrated under vacuum. The crude mixture was purified by flash column chromatography (10% EtOAc/Hex) to give the title product as a yellow oil (50 mg, 0.18 mmol, 36% yield).

### Example 5

### Synthesis of N,N-dimethyl-N'-phenylhydrazine

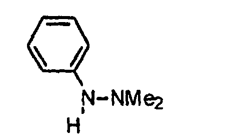

*N,N*-Dimethylhydrazine (2.0 equiv, 1.0 mmol, 0.075 mL), bromobenzene (1 equiv, 0.5 mmol, 0.05 mL), Pd₂(dba)₃ (0.025 equiv, 0.0125 mmol, 12 mg), BINAP (0.05 equiv, 0.025 mmol, 16 mg), LiO*t*Bu (1.2 equiv, 0.6 mmol, 48 mg) and toluene (5 mL) were added to an oven dried test tube which was capped with a septum and purged briefly with argon (∼ 1 min), and then heated to 80° C under argon for 18 hours. The reaction was then cooled to room temperature, diluted with Et₂O (2 mL), filtered through Celite and concentrated under vacuum.. The crude mixture was purified by flash column chromatography (10% EtOAc/Hex) to give the title product as a yellow oil (17 mg, 0.12 mmol, 24% yield).

### Example 6

### Synthesis of Triphenylhydrazine

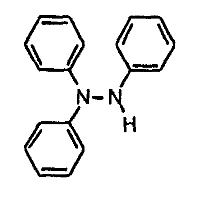

1,1-Diphenylhydrazine hydrochloride (1.2 equiv, 0.6 mmol, 135 mg), bromobenzene (1.0 equiv, 0.5 mmol, 0.05 mL), Pd(OAc)₂ (0.01 equiv, 0.005 mmol, 2 mg), BINAP (0.01 equiv, 0.005 mmol, 3 mg), and NaO*t*Bu (2.8 equiv, 1.4 mmol, 134 mg) and toluene (3 mL) were added to an oven dried test tube which was capped with a septum and purged briefly with argon (∼ 1 min) and then heated to 80° C for 4 hours. The reaction was then cooled to room temperature, diluted with Et₂O (2 mL), filtered through Celite and concentrated under vacuum. The crude mixture was purified by flash column chromatography (5% EtOAc/Hex) to give the title product as a white solid (123 mg, 0.47 mmol, 95% yield).

### Example 7

### Synthesis of N-(4-Benzophenone)-N',N'-Diphenylhydrazine

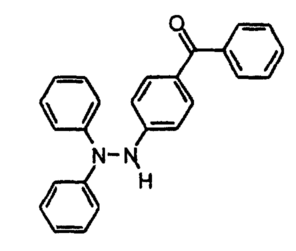

1,1-Diphenylhydrazine hydrochloride (1.2 equiv, 0.6 mmol, 135 mg), 4-bromobenzophenone (1.0 equiv, 0.5 mmol, 131 mg), Pd(OAc)₂ (0.05 equiv, 0.025 mmol, 6 mg), BINAP (0.05 equiv, 0.025 mmol, 16 mg), and NaO*t*Bu (2.8 equiv, 1.4 mmol, 134 mg) and diisopropylamine (2 mL) were added to an oven dried test tube which was capped with a septum and purged briefly with argon (∼ 1 min) and then heated to 80° C for 2 hours. The reaction was then cooled to room temperature, diluted with Et₂O (2 mL), filtered through Celite and concentrated under vacuum. The crude mixture was purified by flash column chromatography (5% EtOAc/Hex) to give the title product as a white solid (141 mg, 0.39 mmol, 77% yield).

### Example 8

### Synthesis of N,N-Diphenyl-N'-(2-chlorophenyl)hydrazine

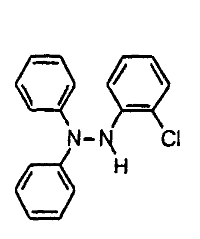

1,1-Diphenylhydrazine hydrochloride (1.2 equiv, 0.6 mmol, 135 mg), 2-chlorobromobenzene (1.0 equiv, 0.5 mmol, 0.06 mL), Pd(OAc)₂ (0.05 equiv, 0.025 mmol, 6 mg), BINAP (0.05 equiv, 0.025 mmol, 16 mg), and NaO*t*Bu (1.4 equiv, 0.7 mmol, 67 mg) and diisopropylamine (2 mL) were added to an oven dried test tube which was capped with a septum and purged briefly with argon (∼ 1 min) and then heated to 80° C for 5 hours. The reaction was then cooled to room temperature, diluted with Et₂O (2 mL), filtered through Celite and concentrated under vacuum. The crude mixture was purified by flash column chromatography (2% EtOAc/Hex) to give the title product as a white solid (109 mg, 0.37 mmol, 74% yield).

### Example 9

### Synthesis of N,N'-Diphenyl-N-(4-benzotrifluoro)hydrazine

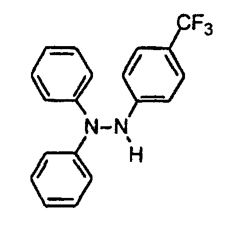

1,2-Diphenylhydrazine (1.2 equiv, 0.6 mmol, 110 mg), 4-bromobenzotrifluoride (1.0 equiv, 0.5 mmol, 0.07 mL), Pd(OAc)₂ (0.05 equiv, 0.025 mmol, 6 mg), BINAP (0.05 equiv, 0.025 mmol, 16 mg), and NaO*t*Bu (1.4 equiv, 0.7 mmol, 67 mg) and diisopropylamine (2 mL) were added to a flame dried test tube which was capped with a septum and purged briefly with argon (∼ 1 min) and then heated to 80° C for 5 hours. The reaction was then cooled to room temperature, diluted with Et₂O (2 mL), filtered through Celite and concentrated under vacuum. The crude mixture was purified by flash column chromatography (5% EtOAc/Hex) to give the title product as a viscous yellow oil (90 mg, 0.27 mmol, 55% yield).

### Example 10

### Synthesis of N,N'-Diethyl-N-phenylhydrazine

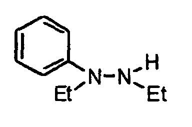

1,2-Diethylhydrazine dihydrochloride (1.2 equiv, 0.6 mmol, 97 mg), bromobenzene (1.0 equiv, 0.5 mmol, 0.05 mL), Pd(OAc)₂ (0.05 equiv, 0.025 mmol, 6 mg), BINAP (0.05 equiv, 0.025 mmol, 16 mg), and NaO*t*Bu (3.8 equiv, 1.9 mmol, 183 mg) and diisopropylamine (2 mL) were added to a flame dried test tube which was capped with a septum and purged briefly with argon (∼ 1 min) and then heated to 80° C for 4 hours. The reaction was then cooled to room temperature, diluted with Et₂O (2 mL), filtered through Celite and concentrated under vacuum. The crude mixture was purified by flash column chromatography (2% EtOAc/Hex) to give the title product as a viscous yellow oil (27 mg, 0.16 mmol, 33% yield).

### Example 11

### N-(4-Phenylphenyl)cyclohexylhydrazone

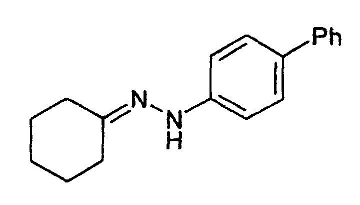

Cyclohexylhydrazone (1.0 equiv, 1.0 mmol, 112 mg), 4-bromobiphenyl (1.0 equiv, 1.0 mmol, 234 mg), Pd(OAc)₂ (0.05 equiv, 0.05 mmol, 11 mg), (*S*)-(-)-BINAP (0.05 equiv, 0.05 mmol, 31 mg), NaO*t*-Bu (1.4 equiv, 1.4 mmol, 134 mg), and toluene (0.2 M, 5 mL) were added to an oven dried test tube, which was then capped and briefly purged with argon and heated to 80° C under argon for 9 hours. The reaction mixture was then cooled to room temperature, diluted with Et₂O, filtered through Celite, and concentrated under vacuum. Purification by flash column chromatography (10% EtOAc/Hex) gave the title product as a white solid (72 mg, 0.27 mmol, 27% yield).

### Example 12

### 6-Phenyl-1,2,3,4-tetrahydrocarbazole

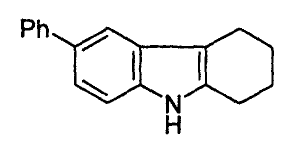

*N*-(4-Phenylphenyl) cyclohexylhydrazone (52 mg, 0.20 mmol) was heated to reflux in a 4% H₂SO₄/H₂O solution with 1 mL EtOH under argon for 4 hours. The reaction mixture was then cooled to room temperature, and neutralized with a saturated NaHCO₃ solution, and then extracted with CH₂Cl₂ (2 X 15 mL). The organic layer was then dried over Na₂SO₄, filtered and concentrated under vacuum. Purification by flash column chromatography (2% EtOAc/Hex) gave the title product as a white solid (22 mg, 0.09 mmol, 45% yield).

### Example 13

### Synthesis of N-(4-Chlorophenyl)benzophenone hydrazone

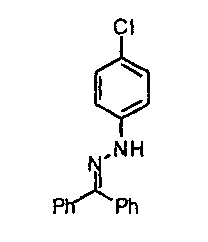

Benzophenone hydrazone (1 equiv, 10 mmol, 2.04 g), 4-chlorobromobenzene (1.0 equiv, 10 mmol, 1.915 g), Pd(OAc)₂ (0.01 equiv, 0.1 mmol, 23 mg), BINAP (0.01 equiv, 0.1 mmol, 63 mg), and toluene (5 mL) were added to an oven dried Schlenk flask and stirred at room temperature for 2 minutes, NaO*t*Bu (1.4 equiv, 14 mmol, 1.345 g) and an additional 5 mL of toluene were then added. The flask was capped with a septum and purged briefly with argon (∼1 min) and heated to 80° C under argon for 13 hours. The reaction mixture was then cooled to room temperature, diluted with Et₂O (10 mL), and concentrated under vacuum to give the crude product as a brown solid. Recrystallization of the crude product from hot isopropanol (100 mL) gave the desired product as a yellow solid (1.971 g, 6.4 mmol, 64% yield). The mother liquor was concentrated under vacuum and purified by column chromatography (5% EtOAc/Hex) to give an additional 550 mg of the desired product (1.79 mmol, 18% yield), to give an 82% overall yield of the title product.

### Example 14

### Synthesis of 6-Chloro-1,2,3,4-tetrahydrocarbazole

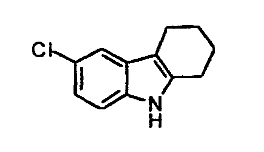

*N*-(4-Chlorophenyl)benzophenone hydrazone (1.0 equiv, 0.5 mmol, 153 mg), cyclohexanone (1.5 equiv, 0.75 mmol, 0.078 mL), and TsOH•H₂O (2 equiv, 1.0 mmol, 190 mg) were dissolved in ethanol (3 mL) and heated to reflux for 41 hours. The reaction mixture was then cooled to room temperature, diluted with Et₂O (5 mL), neutralized with a saturated NaHCO₃ solution, and extracted with Et₂O (3 X 10 mL). The organic extracts were then dried over K₂CO₃, filtered and concentrated under vacuum. Purification by flash column chromatography (10% EtOAc/Hex) gave the title product as a white solid (94 mg, 0.46 mmol, 91% yield).

### Example 15

### Synthesis of N-Benzyl-6-chloro-1,2,3,4-tetrahydrocarbazole

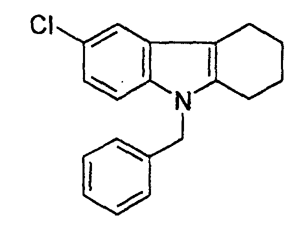

4-Chlorophenyl benzophenone hydrazone (1 equiv, 1 mmol, 306 mg) was dissolved in 5 mL dry Et₂O in a flame dried flask under argon. *n*-BuLi (1.1 equiv, 1.1 mmol, 1.58 M in hexane, 0.69 mL) was then added in a drop wise fashion and the solution was then stirred at room temperature for 10 minutes. Distilled tetramethylethylenediamine (TMEDA, 2.2 equiv, 2.2 mmol, 0.33 mL) was then added, followed by benzyl bromide (1.2 equiv, 1.2 mmol, 0.21 mL). After 3 hours the solution was quenched with an aqueous NH₄Cl solution, extracted with Et₂O (3 X 10 mL), the Et₂O extracts were dried over K₂CO₃, filtered and concentrated under vacuum to give *N*-benzyl-*N*-4-chlorophenylbenzophenone hydrazone as a yellow oil which was used without further purification. This crude product was dissolved in EtOH (3 mL), TsOH•H₂O (380 mg) was then added and the reaction mixture was heated to reflux for 23 hours. The reaction mixture was then cooled to room temperature, neutralized with a saturated NaHCO₃ solution, extracted with Et₂O (2 x 10 mL) and then with EtOAc (2 x 10 mL). The combined organic extracts were then dried over K₂CO₃, filtered and concentrated to give the crude product as a brown oil. Purification by flash column chromatography (2 % EtOAc/Hex) afforded the title product as a pale yellow oil which solidified on standing (269 mg, 0.91 mmol, 91% yield).

### Example 16

### Synthesis of 5-Chloro-3-pentylindole

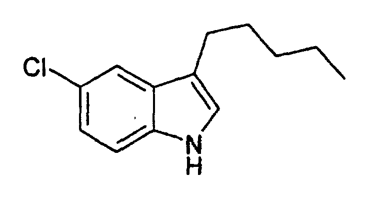

*N*-(4-Chlorophenyl)benzophenone hydrazone (1.0 equiv, 0.5 mmol, 153 mg), heptanal (5 equiv, 2.5 mmol, 0.29 mL), and TsOH•H₂O (10 equiv, 5 mmol, 951 mg) were dissolved in THF (10 mL) and heated to reflux for 41 hours. The reaction mixture was then cooled to room temperature, diluted with Et₂O (5 mL), neutralized with a saturated NaHCO₃ solution, and extracted with Et₂O (3 X 10 mL). The organic extracts were then dried over K₂CO₃, filtered and concentrated under vacuum. Purification by two flash column chromatographies (10% EtOAc/Hex) gave the title product as a yellow oil (29 mg, 0.13 mmol, 26% yield).

### Example 17

### Synthesis of N-(3,4-Dimethoxyphenyl)benzophenone hydrazone

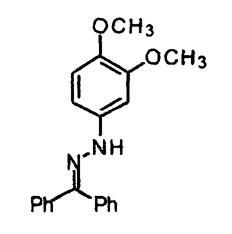

Benzophenone hydrazone (1 equiv, 10 mmol, 2.04 g), 4-bromoveratole (1.0 equiv, 10 mmol, 1.44 mL), Pd(OAc)₂ (0.05 equiv, 0.5 mmol, 112 mg), BINAP (0.05 equiv, 0.5 mmol, 311 mg), and toluene (10 mL) were added to an oven dried Schlenk flask and stirred at room temperature for 2 minutes, NaO*t*Bu (1.4 equiv, 14 mmol, 1.345 g) and an additional 10 mL of toluene were then added. The flask was capped with a septum and purged briefly with argon (∼1 min) and heated to 80° C under argon for 22 hours. The reaction mixture was then cooled to room temperature, diluted with Et₂O (10 mL), and concentrated under vacuum to give the crude product as a brown oil. Purification of the crude mixture by flash column chromatography (15% EtAOc/Hex) gave the title product as a yellow solid (2.161 g, 6.5 mmol, 65% yield).

### Example 18

### Synthesis of 5.6-Dimethoxy-2-methyl-3-propylindole

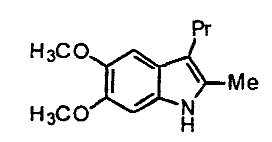

*N*-(3,4-Dimethoxyphenyl)-benzophenone hydrazone (1.0 equiv, 0.32 mmol, 105 mg), 2-hexanone (5 equiv, 1.58 mmol, 0.19 mL), and TsOH•H₂O (10 equiv, 3.2 mmol, 609 mg) were dissolved in THF (10 mL) and heated to reflux for 67 hours. The reaction mixture was then cooled to room temperature, diluted with Et₂O (5 mL), neutralized with a saturated NaHCO₃ solution, and extracted with Et₂O (3 X 10 mL). The organic extracts were then dried over K₂CO₃, filtered and concentrated under vacuum. Purification by flash column chromatography (20% EtOAc/Hex) gave the title product as a yellow oil (60 mg, 0.26 mmol, 80% yield).

### Example 19

### Synthesis of N-(1-Naphthyl)benzophenone hydrazone

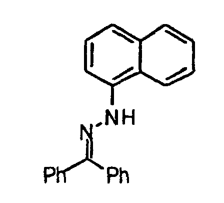

Benzophenone hydrazone (1 equiv, 10 mmol, 2.04 g), 1-bromonaphthalene (1.0 equiv, 10 mmol, 1.39 mL), Pd(OAc)₂ (0.01 equiv, 0.1 mmol, 23 mg), BINAP (0.01 equiv, 0.1 mmol, 63 mg), and toluene (10 mL) were added to an oven dried Schlenk flask and stirred at room temperature for 2 minutes, NaO*t*Bu (1.4 equiv, 14 mmol, 1.345 g) and an additional 10 mL of toluene were then added. The flask was capped with a septum and purged briefly with argon (∼1 min) and heated to 80° C under argon for 4.5 hours. The reaction mixture was then cooled to room temperature, diluted with Et₂O (10 mL), and concentrated under vacuum to give the crude product as a brown solid. Recrystallization of the crude product from hot ethanol (150 mL) gave the desired product as a red solid (1.6939 g, 5.26 mmol, 53% yield). The mother liquor was concentrated until the solution became cloudy, and then heated until the solution became clear, filtered and cooled to -4° C for 12 hours. The resulting crystals were filtered to give an additional 1.062 g of the title product as an orange/red solid (3.3 mmol, 33% yield), to give a 85% overall yield of the desired product.

### Example 20

### Synthesis of 3-Ethoxyacetyl-2-methylbenz[g]indole

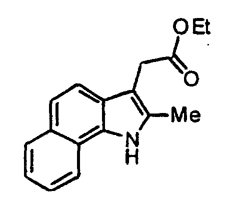

*N*-(1-Naphthyl)-benzophenone hydrazone (1.0 equiv, 1.0 mmol, 322 mg), levulinic acid (1.5 equiv, 1.5 mmol, 0.18 mL), and TsOH•H₂O (2 equiv, 2 mmol mmol, 380 mg) were dissolved in ethanol (10 mL) and heated to reflux for 15 hours. The reaction mixture was then cooled to room temperature, diluted with Et₂O (5 mL), neutralized with a saturated NaHCO₃ solution, and extracted with Et₂O (3 X 10 mL). The organic extracts were then dried over Na₂SO₄, filtered and concentrated under vacuum. Purification by flash column chromatography (20% EtOAc/Hex) gave the title product as a yellow oil (238 mg, 0.89 mmol, 89% yield).

### Example 21

### Synthesis of N-(3,5-Dimethylphenyl)-N-(4-benzotrifluoro)benzophenone hydrazone

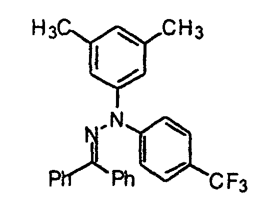

*N*-(3,5-Dimethylphenyl)benzophenone hydrazone (1.0 equiv, 0.5 mmol, 150 mg), 4-bromobenzotrifluoride (1.0 equiv, 0.5 mmol, 0.07 mL), Pd(OAc)₂ (0.01 equiv, 0.005 mmol, 2 mg), DPPF (0.01 equiv, 0.005 mmol, 3 mg), and toluene (1 mL) were added to an oven dried test tube and stirred at room temperature for 2 minutes. NaO*t*Bu (1.4 equiv, 0.7 mmol, 67 mg) and an additional 1 mL toluene were then added. The test tube was then capped with a septum, briefly purged with argon (∼ 1 min) and then heated to 100° C under argon for 14 hours. The reaction solution was then cooled to room temperature, diluted with Et₂O, filtered through Celite, and concentrated under vacuum to give the crude product as a brown oil. Purification by flash column chromatography (2% EtOAc/Hex) gave the title product in approximately 72% purity as determined by ¹H NMR (158 mg, ∼0.26 mmol, ∼ 51% yield).

### Example 22

### Synthesis of N-(4-Benzotrifluoro)-6,8-dimethyl-2,3,4,5-tetrahydrocarbazole

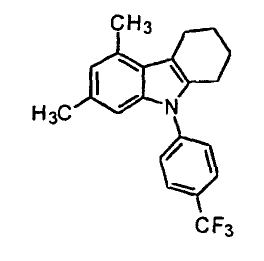

*N*-(3,5-Dimethylphenyl)-*N*-(4-benzotrifluoro)-benzophenone hydrazone (1 l equiv, ∼0.26 mmol, ∼ 51% yield), cyclohexanone (1.5 equiv, 0.75 mmol, 0.078 mL), TsOH•H₂O (2 equiv, 1.0 mmol, 190 mg), and ethanol (3 mL) were heated to reflux for 14 hours. The reaction solution was then cooled to room temperature, neutralized with a saturated NaHCO₃ solution, extracted with Et₂O (3 X 10 mL), and dried over K₂CO₃, and concentrated under vacuum. Purification by flash column chromatography (2% EtOAc/Hex) gave the impure title product (73 mg, ∼0.21 mmol, ∼82% yield).

### Example 23

### Synthesis of N-(3,5-Dimethylphenyl)-N-(4-phenylphenyl)benzophenone hydrazone

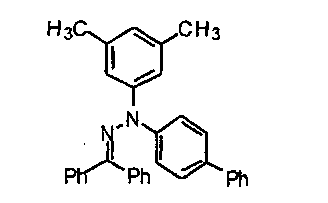

*N*-(3,5-Dimethylphenyl)benzophenone hydrazone (1.0 equiv, 0.5 mmol, 150 mg), 4-bromobiphenyl (1.0 equiv, 0.5 mmol, 117 mg), Pd(OAc)₂ (0.01 equiv, 0.005 mmol, 2 mg), DPE-phos (0.01 equiv, 0.005 mmol, 3 mg), and toluene (1 mL) were added to an oven dried test tube and stirred at room temperature for 2 minutes. NaO*t*Bu (1.4 equiv, 0.7 mmol, 67 mg) and an additional 1 mL toluene were then added. The test tube was then capped with a septum, briefly purged with argon (∼ 1 min) and then heated to 100° C under argon for 14 hours. The reaction solution was then cooled to room temperature, diluted with Et₂O, filtered through Celite, and concentrated under vacuum to give the crude product as a brown oil. Purification by flash column chromatography (2% EtOAc/Hex) gave the title product as a yellow solid (149 mg, 0.33 mmol, 66% yield).

### Example 24

### General procedure for catalytic amination of aryl bromides using a (rac)-BINAP/ Pd(OAc)₂ catalyst system.

An oven-dried Schlenk tube was cooled under an argon purge, and charged with (rac)-BINAP (4.7 mg, 0.0075 mmol, 0.75 mol%) and toluene (1 mL). The mixture was heated to 80 °C with stirring for 5 min until the BINAP had dissolved. The mixture was cooled to room temperature, the septum was removed, the flask was charged with Pd(OAc)₂ (1.1 mg, 0.005 mmol, 0.5 mol%), then the septum was again placed over the joint of the flask. The sides of the flask were rinsed with toluene (0.2 ml), and the flask was purged with argon for 30 seconds, and the mixture was stirred at rt for 1 min. The flask was then charged with 5-bromo-m-xylene (0.135 mL, 1.0 mmol), benzylamine (0.165 mL, 1.5 mmol). The septum was again removed, and sodium *t*-butoxide was added to the reaction mixture. The septum was replaced, and the flask was purged with argon for 30 seconds. The sides of the flask were rinsed with toluene (0.8 mL), and the mixture was then heated in an 80 °C oil bath with stirring until all aryl halide had been consumed as judged by GC analysis. The mixture was then cooled to room temperature and ether (20 mL) was added. The mixture was filtered through Celite and concentrated. The product was purified by flash chromatography on silica gel to give 189 mg (90%) of N-benzyl-3,5-dimethyl aniline as a colorless oil.

### Notes for Example 24:

1. For amination reactions using secondary amines as coupling partners, 1.2 eq of amine may be used instead of 1.5 eq.
2. For reactions run in THF, instead of dissolving the BINAP in hot solvent, the BINAP and Pd(OAc)₂ are mixed in THF (1/2 of total volume used for rxn) and stirred at rt under argon for 10 min (a peach colored suspension will form). The halide, amine, and base are then added as above.
3. Reactions may be run using cesium carbonate as base instead of sodium *t*-butoxide. In these reactions, the amount of solvent is doubled and the reactions are run at 100 °C.

### Example 25

### General procedure for catalytic amination of aryl bromides using a DPEphos/ Pd(OAc)₂ catalyst system.

An oven-dried Schlenk tube was charged with *p*-toluidine (1.20 mmol), palladium acetate (0.005 mmol) and DPEphos (0.0075 mmol), evacuated, and repressurized with argon. 2-Bromoanisole was added to the flask via syringe, followed by toluene (2 mL). The resulting mixture was stirred for 5 min at room temperature, affording a clear yellow solution. The flask was opened and solid sodium *tert*-butoxide (1.40 mmol) was added in one portion, causing the solution to turn a deep red color. The flask was purged for 3 min with argon, and the mixture was heated with stirring to 80 °C until the aryl bromide had been consumed as judged by GC analysis. The mixture was then cooled to room temperature, taken up in diethyl ether (40 mL), and washed with brine. The resulting solution was dried over anhydrous potassium carbonate, filtered, and concentrated. The crude product was purified by flash chromatography on silica gel using 9:1 hexanes:ethyl acetate as the eluant, affording the product as a pale yellow oil in 94% yield.

*Note:* The order of addition described above, i.e., in which the base is added after the palladium acetate has been mixed with the ligand and the amine, has been found to be important in the activation of the catalyst.

### Example 26

### Synthesis of N-(p-Chlorophenyl) benzophenone hydrazone using 0.1 mol% Pd

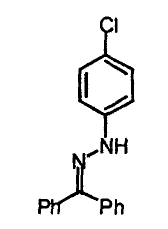

A flame dried Schlenk flask was charged with Pd(OAc)₂ (2 mg, 0.01 mmol), xantphos (6 mg, 0.011 mmol), NEt₃ (3.0 µL, 0.022 mmol), and toluene (1 mL), capped with a septum, and purged with argon for ∼1 minute. The solution was stirred at room temperature for 15 minutes. The septum was removed, benzophenone hydrazone (1.96 g, 10 mmol), 4-chlorobromobenzene (1.91 g, 10 mmol), NaO*t*-Bu (1.34 g, 14 mmol) and toluene (9 mL) were added to the flask, which was then recapped with the septum and evacuated and backfilled with argon 3 times. The reaction mixture was heated to 80 °C for 1.3 hours, cooled to room temperature, diluted with Et₂O (20 mL), filtered through a pad of Celite (∼0.25"), and the Celite was rinsed with EtOAc (30 mL). The filtrate was then concentrated to give the analytically pure title product as a pale yellow solid (3.0 g, 9.78 mmol, 98% yield).

### Example 27

### Synthesis of N-(3,5-Dimethylphenyl) benzophenone hydrazone using 0.1 mol% Pd

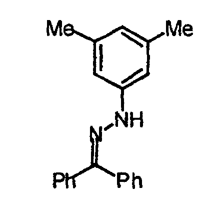

A flame dried Schlenk flask was charged with Pd(OAc)₂ (2 mg, 0.01 mmol), xantphos (6 mg, 0.011 mmol), NEt₃ (3.0 mL, 0.022 mmol), and toluene (1 mL), caped with a septum, and purged with argon for ∼1 minute. The solution was stirred at room temperature for 15 minutes. The septum was removed, benzophenone hydrazone (1.96 g, 10 mmol), 5-bromo-*m*-xylene (1.36 mL, 10 mmol), NaO*t*-Bu (1.34 g, 14 mmol) and toluene (9 mL) were added to the flask, which was then recapped with the septum and evacuated and backfilled with argon 3 times. The reaction mixture was heated to 80 °C for 17 hours, cooled to room temperature and diluted with Et₂O (20 mL), and filtered through a pad of Celite (∼0.25"), and the Celite was rinsed with EtOAc (30 mL) and the filtrate was concentrated en vacuo to give the crude product as a yellow solid. Recrystallization from hot MeOH (80 mL) afforded the analytically pure title product as yellow crystals (2.493 g, 8.31 mmol, 83% yield). The mother liquor was concentrated and purified by flash column chromatography (2% EtOAc/hexane) to afford an additional 306 mg of the desired product as a yellow solid (1.02 mmol, 10% yield), to give a 93% overall yield.

### Example 28

### Synthesis of N-(3,4-dimethoxyphenyl) benzophenone hydrazone using 0.1 mol% Pd

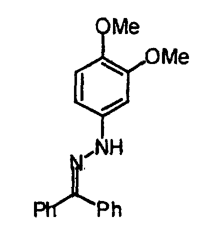

A flame dried Schlenk flask was charged with Pd(OAc)₂ (2 mg, 0.01 mmol), xantphos (6 mg, 0.011 mmol), NEt₃ (3.0 mL, 0.022 mmol), and toluene (1 mL), caped with a septum, and purged with argon for ∼1 minute. The solution was stirred at room temperature for 15 minutes. The septum was removed, benzophenone hydrazone (1.96 g, 10 mmol), 4-bromoveratole (1.44 mL, 10 mmol), NaO*t*-Bu (1.34 g, 14 mmol) and toluene (9 mL) were added to the flask, which was then recaped with the septum and evacuated and backfilled with argon 3 times. The reaction mixture was heated to 100 °C for 17.5 hours, cooled to room temperature and diluted with Et₂O (20 mL), and filtered through a pad of Celite (∼0.25"), and the Celite was rinsed with EtOAc (30 mL) and the filtrate was concentrated to give the crude product as a yellow solid. Recrystallization from hot MeOH afforded the analytically pure title product as yellow crystals (2.323 g, 6.997 mmol, 70% yield).

### Example 29

### Synthesis of N,N-Bis(p-tolyl) benzophenone hydrazone

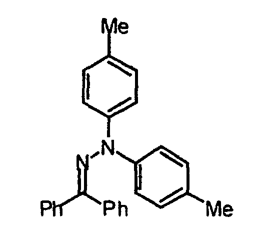

An oven dried test tube was charged with Pd(OAc)₂ (11 mg, 0.05 mmol), Xantphos (9,9-dimethyl-4,5-bis(diphenylphosphino)xanthene) (32 mg, 0.055 mmol), and xylene (0.5 mL), capped with a septum, placed under an atmosphere of argon and stirred at room temperature for 10 minutes. The septum was then removed and benzophenone hydrazone (196 mg, 1 mmol), *p*-bromotoluene (308 µL, 2.5 mmol) and NaO*t*-Bu (231 mg, 2.4 mmol) were then added to the reaction mixture followed by an additional portion of xylene (0.5 mL). The test tube was recapped with the septum, purged with argon for ∼1 minute, and heated to 120 °C for 2 hours. The reaction mixture was then allowed to cool to room temperature, and then diluted with Et₂O (5 mL), and filtered through a short plug of Celite (∼0.25"), and concentrated to a viscous brown oil. Purification by flash column chromatography (2% EtOAc/hexane) afforded the title product as a viscous yellow oil (337 mg, 0.9 mmol, 90% yield).

### Example 30

### Synthesis of N-(3,4-Dimethoxy)-N-p-chlorophenyl benzophenone hydrazone

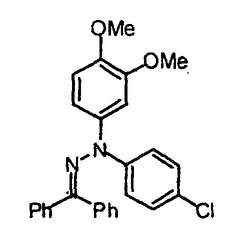

An oven dried test tube was charged with Pd(OAc)₂ (11 mg, 0.05 mmol), xantphos (32 mg, 0.055 mmol), benzophenone hydrazone (196 mg, 1 mmol), 4-bromoveratole (140 µL, 1.0 mmol), NaO*t*-Bu (231 mg, 2.4 mmol), and xylene (1 mL). The test tube was then capped with a septum, purged with argon for ∼1 minute and heated to 100 °C for 20 minutes. 2-Chlorobromobenzene (287 mg, 1.5 mmol) was dissolved in xylene (1 mL), and added via syringe to the reaction mixture, which was then heated to 120 ° C for 20 hours. The reaction mixture was then cooled to room temperature, diluted with Et₂O (5 mL), filtered through a short plug of Celite (∼0.25"). The Celite was then rinsed with an EtOAc (15 mL), and concentrated to a viscous brown oil. Purification by flash column chromatography (10% EtOAc/hexane) afforded the title product as a bright yellow solid (364 mg, 0.82 mmol, 82% yield).

### Example 31

### Synthesis of N-p-Methoxyphenyl-N-(3-pyridyl)benzophenone hydrazone

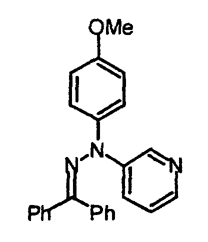

An oven dried test tube was charged with Pd(OAc)₂ (11 mg, 0.05 mmol), xantphos (32 mg, 0.055 mmol), benzophenone hydrazone (196 mg, 1 mmol), 4-bromoanisole (125 µL, 1.0 mmol) and NaO*t*-Bu (231 mg, 2.4 mmol), and xylene (2 mL), capped with a septum, purged with argon for ∼1 minute and heated to 100 °C for 10 minutes. 3-Bromopyridine (144 µL, 1.5 mmol) was added via syringe to the reaction mixture, which was then heated to 120 °C for 19 hours. The reaction mixture was then cooled to room temperature, diluted with Et₂O (5 mL), filtered through a short plug of Celite (∼0.25"), and concentrated to give the crude product as a viscous brown oil. Purification by flash column chromatography (25% EtOAc/hexane on NEt₃ washed silica gel) afforded the title product as a viscous bright yellow oil (321 mg, 0.85 mmol, 85% yield).

### Example 32

### Synthesis of N-p-Tolyl-N-m-cyanophenyl benzophenone hydrazone

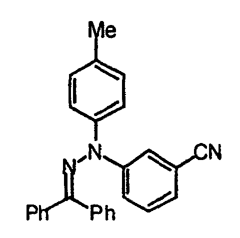

An oven dried test tube was charged with Pd(OAc)₂ (11 mg, 0.05 mmol), xantphos (32 mg, 0.055 mmol), benzophenone hydrazone (196 mg, 1 mmol), 4-bromoanisole (120 µL, 1.0 mmol) and NaO*t*-Bu (231 mg, 2.4 mmol), and xylene (1 mL), capped with a septum, purged with argon for ∼1 minute and heated to 60 °C for 60 minutes. 3-Bromobenzonitrile (231 mg, 1.5 mmol) was dissolved in xylene (0.5 mL) and added via syringe to the reaction mixture, which was then heated to 120 °C for 17 hours. The reaction mixture was then cooled to room temperature, diluted with Et₂O (5 mL), filtered through a short plug of Celite (∼0.25"), and concentrated to give the crude product as a viscous brown oil. Purification by flash column chromatography (5% EtOAc/hexane) afforded the title product as a viscous bright yellow oil (334 mg, 0.86 mmol, 86% yield).

### Example 33

### Synthesis of 3,5-Dimethyl-2-Ethyl-N-p-tolyl indole

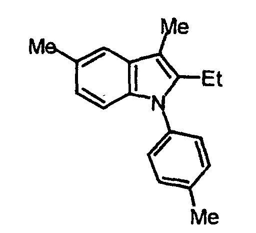

*N*,*N*-Bis(*p*-tolyl) benzophenone hydrazone (277 mg, 0.76 mmol), 3-pentanone (240 µL, 2.29 mmol) and concentrated HCl (1 mL) were dissolved in EtOH (5 mL). The reaction mixutre was heated to reflux for 2.5 hours, cooled to room temperature, neutralized with a saturated NaHCO₃ solution and extracted with Et₂O (3 X 10 mL). The combined Et₂O extracts where then dried over K₂CO₃, filtered and concentrated to give the crude products as a brown oil. Purification by flash column chromatography (2% EtOAc/hexane) afforded the analytically pure product as a white solid (136 mg, 0.52 mmol, 68% yield).

### Example 34

### Synthesis of N-(p-Chlorophenyl)-5,6-dimethoxy-1,2,3,4-tetrahydrocarbazole

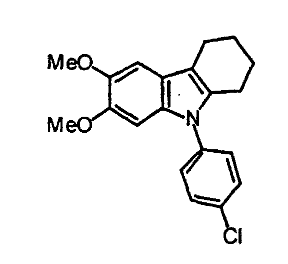

*N*-(3,4-dimethoxy)-*N*-*p*-chlorophenyl benzophenone hydrazone (110 mg, 0.25 mmol), cyclohexanone (40 µL, 0.375 mmol), and concentrated HCl (250 µL) were dissolved in EtOH (1 mL). The reaction mixture was then heated to reflux for 1.5 hours, and then cooled to room temperature, neutralized with a saturated NaHCO₃ solution and extracted with Et₂O (3X10 mL). The combined Et₂O extracts where then dried over K₂CO₃, filtered and concentrated to give the crude products as a brown oil. Purification by flash column chromatography (10% EtOAc/hexane) afforded the analytically pure product as a white solid (72 mg, 0.21 mmol, 86% yield).

### Example 35

### Synthesis of N-(3-Pyridyl)-3-ethylacetoxy-5-methoxy-2-methyl indole

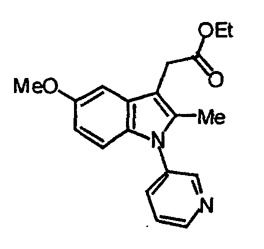

*N*-*p*-Methoxyphenyl-*N*-(3-pyridyl) benzophenone hydrazone (211 mg, 0.56 mmol), laevulinic acid (85 µL, 0.83 mmol), and concentrated HCl (560 µL) were dissolved in EtOH (2 mL). The reaction mixture was heated to reflux for 1.5 hours, and then cooled to room temperature, neutralized with a saturated NaHCO₃ solution and extracted with Et₂O (3 X 10 mL). The combined Et₂O extracts where then dried over K₂CO₃, filtered and concentrated to give the crude products as a brown oil. Purification by flash column chromatography (50% EtOAc/hexane) afforded the analytically pure product as a viscous yellow oil (180 mg, 0.55 mmol, 99% yield).

### Example 36

### Synthesis of N-(3-Cyanophenyl)-2,3,5-trimethyl indole

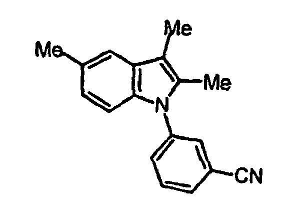

*N*-*p*-Tolyl-*N*-*m*-cyanophenyl benzophenone hydrazone (273 mg, 0.70 mmol), 2-butanone (95 µL, 1.06 mmol), and concentrated HCl (700 µL) were dissolved in EtOH (3 mL) and heated to reflux for 1.5 hours. The reaction was then cooled to room temperature, neutralized with a saturated NaHCO₃ solution and extracted with Et₂O (3X10 mL). The combined Et₂O extracts where then dried over K₂CO₃, filtered and concentrated to give the crude products as a brown oil. Purification by flash column chromatography (2% EtOAc/hexane) afforded the analytically pure product as a clear yellow oil (158 mg, 0.61 mmol, 87% yield).

## Claims

1. A method for the arylation of hydrazines, hydrazones, hydroxylamines and oximes, and salts thereof, comprising reacting, in the presence of a transition metal catalyst, an activated aromatic compound, and a hydrazine, hydrazone, hydroxylamine or oxime, or salt thereof, under conditions suitable for the transition metal catalyst to effect the formation of a new carbon-heteroatom bond between the activated carbon of said activated aromatic compound and a heteroatom of said hydrazine, hydrazone, hydroxylamine or oxime, or salt thereof.

2. A method for the vinylation of hydrazines, hydrazones, hydroxylamines and oximes, and salts thereof, comprising reacting, in the presence of a transition metal catalyst, an activated vinyl compound, and a hydrazine, hydrazone, hydroxylamine or oxime, or salt thereof, under conditions suitable for the transition metal catalyst to effect the formation of a new carbon-heteroatom bond between the activated carbon of said activated vinyl compound and a heteroatom of said hydrazine, hydrazone, hydroxylamine or oxime, or salt thereof.

3. The method of claim 1 or 2, wherein the transition metal of the transition metal catalyst is selected from the group consisting of Fe, Ru, Co, Rh, Ir, Ni, Pd, Pt, and Cu.

4. The method of claim 1 or 2, wherein the transition metal catalyst is present in substoichiometric quantities relative to the limiting reagent among said activated aryl or vinyl compound, and said hydrazine, hydrazone, hydroxylamine or oxime, or salt thereof.

5. The method of claim 3, wherein the transition metal catalyst is present in substoichiometric quantities relative to the limiting reagent among said activated aryl or vinyl compound, and said hydrazine, hydrazone, hydroxylamine or oxime, or salt thereof.

6. The method of claim 1 or 2, wherein the transition metal catalyst is present in less than or equal to 50 mol% relative to the limiting reagent among said activated aryl or vinyl compound, and said hydrazine, hydrazone, hydroxylamine or oxime, or salt thereof.

7. The method of claim 3, wherein the transition metal catalyst is present in less than or equal to 50 mol% relative to the limiting reagent among said activated aryl or vinyl compound, and said hydrazine, hydrazone, hydroxylamine or oxime, or salt thereof.

8. The method of claim 1 or 2, wherein the transition metal catalyst is present in less than or equal to 10 mol% relative to the limiting reagent among said activated aryl or vinyl compound, and said hydrazine, hydrazone, hydroxylamine or oxime, or salt thereof.

9. The method of claim 3, wherein the transition metal catalyst is present in less than or equal to 10 mol% relative to the limiting reagent among said activated aryl or vinyl compound, and said hydrazine, hydrazone, hydroxylamine or oxime, or salt thereof.

10. The method of claim 1 or 2, wherein the transition metal catalyst is present in less than or equal to 5 mol% relative to the limiting reagent among said activated aryl or vinyl compound, and said hydrazine, hydrazone, hydroxylamine or oxime, or salt thereof.

11. The method of claim 3, wherein the transition metal catalyst is present in less than or equal to 5 mol% relative to the limiting reagent among said activated aryl or vinyl compound, and said hydrazine, hydrazone, hydroxylamine or oxime, or salt thereof.

12. The method of claim 1 or 2, wherein the transition metal catalyst is present in less than or equal to 2 mol% relative to the limiting reagent among said activated aryl or vinyl compound, and said hydrazine, hydrazone, hydroxylamine or oxime, or salt thereof.

13. The method of claim 3, wherein the transition metal catalyst is present in less than or equal to 2 mol% relative to the limiting reagent among said activated aryl or vinyl compound, and said hydrazine, hydrazone, hydroxylamine or oxime, or salt thereof.

14. The method of claim 1 or 2, wherein the activated group on the aromatic or vinyl compound is selected from the group consisting of halides and sulfonates.

15. The method of claim 14, wherein the activated group on the aromatic or vinyl compound is selected from the group consisting of chloride, bromide, iodide, tosylate, mesylate, triflate and nonaflate.

16. The method of claim 3, wherein the activated group on the aromatic or vinyl compound is selected from the group consisting of halides and sulfonates.

17. The method of claim 16, wherein the activated group on the aromatic or vinyl compound is selected from the group consisting of chloride, bromide, iodide, tosylate, mesylate, triflate and nonaflate.

18. The method of claim I or 2, wherein the hydrazine, hydrazone, hydroxylamine or oxime, or salt thereof, is selected from the group consisting of hydrazine, alkyl and aryl hydrazines, 1,1- and 1,2-dialkyl and diaryl hydrazines, 1,1- and 1,2-alkyl aryl hydrazines, 1,1,2-trialkyl and triaryl hydrazines, 1,1,2-dialkyl aryl and alkyl diaryl hydrazines, aldehyde and ketone hydrazones, *N*-alkyl and *N*-aryl aldehyde and ketone hydrazones, hydroxylamine, *O*-alkyl and *O*-aryl hydroxylamines, *N*-alkyl and *N*-aryl hydroxylamines, *N,O*-dialkyl and *N,O*-diaryl hydroxylamines, *N,O*-alkyl aryl hydroxylamines, *N,N*-dialkyl and *N,N*-diaryl hydroxylamines, and *N,N*-alkyl aryl hydroxylamines, and aldehyde and ketone oximes, and salts thereof.

19. The method of claim 3, wherein the hydrazine, hydrazone, hydroxylamine or oxime, or salt thereof, is selected from the group consisting of hydrazine, alkyl and aryl hydrazines, 1,1- and 1,2-dialkyl and diaryl hydrazines, 1,1- and 1,2-alkyl aryl hydrazines, 1,1,2-trialkyl and triaryl hydrazines, 1,1,2-dialkyl aryl and alkyl diaryl hydrazines, aldehyde and ketone hydrazones, *N*-alkyl and *N*-aryl aldehyde and ketone hydrazones, hydroxylamine, *O*-alkyl and *O*-aryl hydroxylamines, *N*-alkyl and *N*-aryl hydroxylamines, *N,O*-dialkyl and *N,O*-diaryl hydroxylamines, *N,O*-alkyl aryl hydroxylamines, *N,N*-dialkyl and *N,N*-diaryl hydroxylamines, and *N,N*-alkyl aryl hydroxylamines, and aldehyde and ketone oximes, or salt thereof.

20. The method of claim 1, **characterized by** the general reaction scheme: 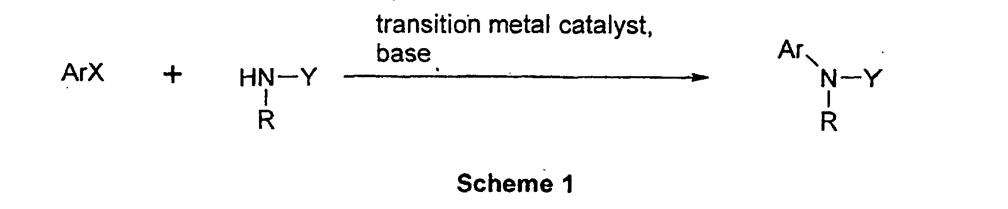 wherein
Ar represents an optionally substituted aromatic or heteroaromatic group;
X represents an activated group, e.g., a halide or a sulfonate, which can be replaced by a nucleophilic nitrogen in a transition metal-catalyzed arylation reaction;
Y represents NR₂, OR, N=CR₂, N(R)S(O)₂R, or N(R)C(O)NR₂;
R represents independently for each occurrence, as valence and stability permit, H, an optionally substituted alkyl, alkenyl or aryl; formyl, acyl, sulfonyl or -(CH₂)ₘ-R₈;
R₈ represents independently for each occurrence an optionally substituted aryl, cycloalkyl, cycloalkenyl, heterocycle or polycycle; and
m is an integer in the range 0 to 8 inclusive.

21. The method of claim 20, wherein Ar is selected from the group consisting of optionally substituted monocyclic aromatic and heteroaromatic groups.

22. The method of claim 20, wherein X is selected from the group consisting of halides and sulfonates.

23. The method of claim 20, wherein X is selected from the group consisting of chloride, bromide, iodide, mesylate, triflate, tosylatc and nonaflate.

24. The method of claim 20, wherein Y is selected from the group consisting of NR₂, OR and N=CR₂.

25. The method of claim 20, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is selected from the group consisting of NR₂, OR and N=CR₂.

26. The method of claim 1, **characterized by** the general reaction scheme: 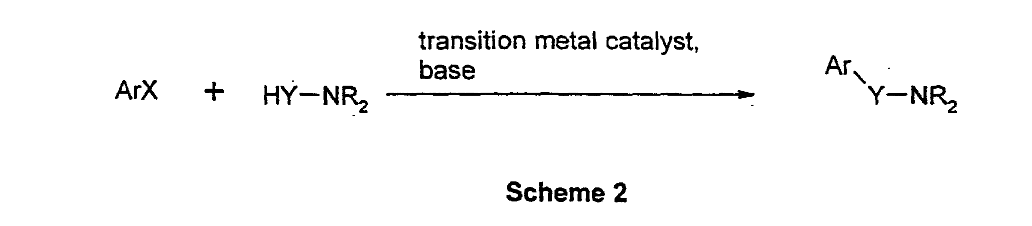 wherein
Ar represents an optionally substituted aromatic or heteroaromatic group;
X represents an activated group, e.g., a halide or a sulfonate, which can be replaced by Y in a transition metal-catalyzed arylation reaction;
Y represents O, S, or Se;
R represents independently for each occurrence, as valence and stability permit, H, an optionally substituted alkyl, alkenyl or aryl; alkylidene, formyl, acyl, sulfonyl, or -(CH₂)ₘ-R₈; the two instances of R taken together may represent an alkylidene group;
R₈ represents independently for each occurrence an optionally substituted aryl, cycloalkyl, cycloalkenyl, heterocycle or polycycle; and
m is an integer in the range 0 to 8 inclusive.

27. The method of claim 26, wherein Ar is selected from the group consisting of optionally substituted monocyclic aromatic and heteroaromatic groups.

28. The method of claim 26, wherein X is selected from the group consisting of halides and sulfonates.

29. The method of claim 26, wherein X is selected from the group consisting of chloride, bromide, iodide, mesylate, triflate, tosylate and nonaflate.

30. The method of claim 26, wherein Y is O.

31. The method of claim 26, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is O.

32. A method **characterized by** the general reaction scheme: 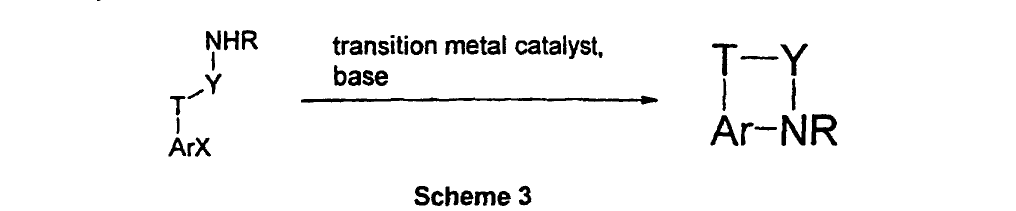 wherein
Ar represents an optionally substituted aromatic or heteroaromatic group;
X represents an activated group, e.g., a halide or a sulfonate, which can be replaced by a nucleophilic nitrogen in a transition metal-catalyzed arylation reaction;
Y represents NR, O, s*p*²-hybridized N in a π-bond to T, NS(O)₂R, or NC(O)NR₂;
T represents a covalent tether connecting Y and Ar, said tether comprising between 0 and 4 backbone atoms; the backbone of said tether may comprise a π-bond, provided that the configuration of said π-bond is such that the described intramolecular reaction is geometrically feasible, or that said π-bond can adopt a configuration under the reaction conditions that renders the intramolecular reaction geometrically feasible; said tether may be optionally unsubstituted, bearing any number of substituents of any type permitted by stability and the rules of valence;
R represents independently for each occurrence, as valence and stability permit, H, an optionally substituted alkyl, alkenyl or aryl; formyl, acyl, sulfonyl or -(CH₂)ₘ-R₈;
R₈ represents independently for each occurrence an optionally substituted aryl, cycloalkyl, cycloalkenyl, heterocyclc or polycycle; and
m is an integer in the range 0 to 8 inclusive.

33. The method of claim 32, wherein Ar is selected from the group consisting of optionally substituted monocyclic aromatic and heteroaromatic groups.

34. The method of claim 32, wherein X is selected from the group consisting of halides and sulfonates.

35. The method of claim 32, wherein X is selected from the group consisting of chloride, bromide, iodide, mesylatc, triflate, tosylate and nonaflate.

36. The method of claim 32, wherein Y is selected from the group consisting of NR, O, and *sp*²-hybridized N in a π-bond to T.

37. The method of claim 32, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is selected from the group consisting of NR, O, and *sp*²-hybridized N in a π-bond to T.

38. A method **characterized by** the general reaction scheme: 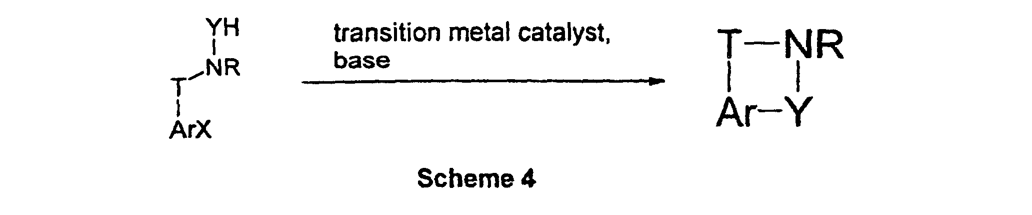 wherein
Ar represents an optionally substituted aromatic or heteroaromatic group;
X represents an activated group, e.g., a halide or a sulfonate, which can be replaced by Y in a transition metal-catalyzed arylation reaction;
Y represents O, S, or Se;
T represents a covalent tether connecting NR and Ar, said tether comprising between 0 and 4 backbone atoms; the backbone of said tether may comprise a π-bond, provided that the configuration of said π-bond is such that the described intramolecular reaction is geometrically feasible, or that said π-bond can adopt a configuration under the reaction conditions that renders the intramolecular reaction geometrically feasible; said tether may be optionally substituted, bearing any number of substituents of any type permitted by stability and the rules of valence;
R represents independently for each occurrence, as valence and stability permit, H, an optionally substituted alkyl, alkenyl or aryl; formyl, acyl, sulfonyl or -(CH₂)ₘ-R₈;
R₈ represents independently for each occurrence an optionally substituted aryl, cycloalkyl, cycloalkenyl, heterocycle or polycycle; and
m is an integer in the range 0 to 8 inclusive.

39. The method of claim 38, wherein Ar is selected from the group consisting of optionally substituted monocyclic aromatic and heteroaromatic groups.

40. The method of claim 38, wherein X is selected from the group consisting of halides and sulfonates.

41. The method of claim 38, wherein X is selected from the group consisting of chloride, bromide, iodide, mesylate, triflate, tosylate and nonaflate.

42. The method of claim 38, wherein Y is O.

43. The method of claim 38, wherein Ar is selected from the group consisting of optionally substituted monocyclic aromatic and heteroaromatic groups; X is selected from the group consisting of chloride, bromide, iodide, mesylate, triflate, tosylate and nonaflate; and Y is O.

44. The method of claim 2, **characterized by** the general reaction scheme: 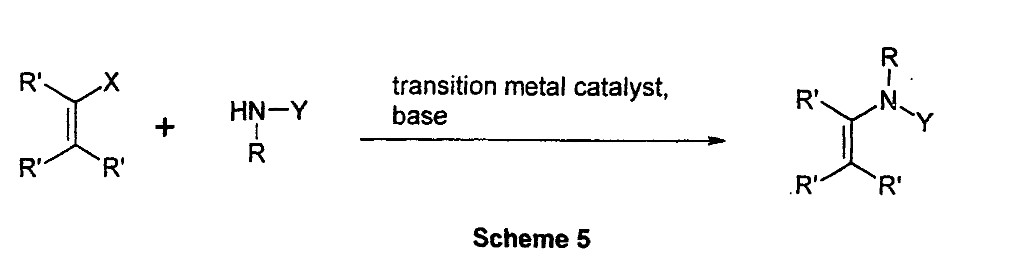 wherein
X represents an activated group, e.g., a halide or a sulfonate, which can be replaced by a nucleophilic nitrogen in a transition metal-catalyzed vinylation reaction;
Y represents NR₂, OR, N=CR₂, N(R)S(O)₂R, or N(R)C(O)NR₂;
R represents independently for each occurrence, as valence and stability permit, H, an optionally substituted alkyl, alkenyl or aryl; formyl, acyl, sulfonyl or -(CH₂)ₘ-R₈;
R' represents independently for each occurrence, as valence and stability permit, H, halogen, lower alkyl, lower alkenyl, lower alkynyl, carbonyl group (e.g., ester, carboxylate, or formate), thiocarbonyl (e.g., thiolester, thiolcarboxylate, or thiolformate), ketone, aldehyde, amino, acylamino, amido, amidino, cyano, nitro, azido, sulfonyl, sulfoxido, sulfate, sulfonate, sulfamoyl, sulfonamido, phosphoryl, phosphonate, phosphinate, -(CH₂)ₘ-R₈, -(CH₂)ₘ-OH, -(CH₂)ₘ-O-lower alkyl, -(CH₂)ₘ-O-lower alkenyl, -(CH₂)ₘ-O-(CH₂)ₙ-R₈, -(CH₂)ₘ-SH, -(CH₂)ₘ-S-lower alkyl, -(CH₂)ₘ-S-lower alkenyl, -(CH₂)ₘ-S-(CH₂)ₙ-R₈, or protecting groups of the above, or a solid or polymeric support;
R₈ represents independently for each occurrence an optionally substituted aryl, cycloalkyl, cycloalkenyl, heterocycle or polycycle; and
n and m are integers independently for each occurrence selected from the range of 0 to 8 inclusive.

45. The method of claim 44, wherein X is selected from the group consisting of halides and sulfonates.

46. The method of claim 44, wherein X is selected from the group consisting of chloride, bromide, iodide, mesylate, triflate, tosylate and nonaflate.

47. The method of claim 44, wherein Y is selected from the group consisting of NR₂, OR and N=CR₂.

48. The method of claim 44, wherein X is selected from the group consisting of chloride, bromide, iodide, mesylate, triflate, tosylate and nonaflate; and Y is selected from the group consisting of NR₂, OR and N=CR₂.

49. The method of claim 2, **characterized by** the general reaction scheme: 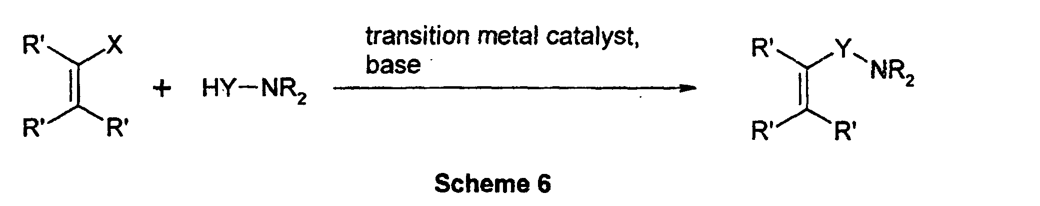 wherein
X represents an activated group, e.g., a halide or a sulfonate, which can be replaced by Y in a transition metal-catalyzed vinylation reaction;
Y represents O, S, or Se;
R represents independently for each occurrence, as valence and stability permit, H, an optionally substituted alkyl, alkenyl or aryl; alkylidene, formyl, acyl, sulfonyl, or -(CH₂)ₘ-R₈; the two instances of R taken together may represent an alkylidene group;
R' represents independently for each occurrence, as valence and stability permit, H, halogen, lower alkyl, lower alkenyl, lower alkynyl, carbonyl group (e.g. ester, carboxylate, or formate), thiocarbonyl (e.g. thiolester, thiolcarboxylate, or thiolformate), ketone, aldehyde, amino, acylamino, amido, amidino, cyano, nitro, azido, sulfonyl, sulfoxido, sulfate, sulfonate, sulfamoyl, sulfonamido, phosphoryl, phosphonate, phosphinate, -(CH₂)ₘ-R₈, -(CH₂)ₘ-OH, -(CH₂)ₘ-O-lower alkyl, -(CH₂)ₘ-O-lower alkenyl, -(CH₂)ₘ-O-(CH₂)ₙ-R₈, -(CH₂)ₘ-SH, -(CH₂)ₘ-S-lower alkyl, -(CH₂)ₘ-S-lower alkenyl, -(CH₂)ₘ-S-(CH₂)ₙ-R₈, or protecting groups of the above, or a solid or polymeric support;
R₈ represents independently for each occurrence an optionally substituted aryl, cycloalkyl, cycloalkenyl, heterocycle or polycycle; and
n and m are integers independently for each occurrence selected from the range of 0 to 8 inclusive.

50. The method of claim 49, wherein X is selected from the group consisting of halides and sulfonates.

51. The method of claim 49, wherein X is selected from the group consisting of chloride, bromide, iodide, mesylate, triflate, tosylate and nonaflate.

52. The method of claim 49, wherein Y is O.

53. The method of claim 49, wherein X is selected from the group consisting of chloride, bromide, iodide, mesylate, triflate, tosylate and nonaflate; and Y is O.

54. The method of claim 1 or 2, wherein the transition metal catalyst and the ligand are premixed before addition of the remaining reagents.

55. A method for the transition metal catalyzed amination of activated aromatic compounds, wherein the transition metal catalyst and the ligand are premixed before addition of the remaining reagents.

56. A method for the synthesis of pyrroles, indoles, furans, benzofurans, wherein a product produced according to claim 1 or 2 is subjected to Bronsted or Lewis acidic conditions to produce a pyrrole, indole, furan, benzofuran.

57. The method of claim 56, wherein a variegated library of heterocyclic products is produced via parallel, combinatorial synthetic methods.

58. The method of claim 57, wherein the members of the library are attached to a solid support.

59. The method of claim 54, wherein the base utilized is cesium carbonate or sodium *tert*-butoxide.

60. The method of claim 55, wherein the base utilized is cesium carbonate or sodium *tert*-butoxide.

61. The method of claim 54, wherein the ligand utilized is selected from the set of achiral and chiral chelating ligands.

62. The method of claim 55, wherein the ligand utilized is selected from the set of achiral and chiral chelating ligands.

## Patentansprüche

1. , Verfahren für die Arylierung von Hydrazinen, Hydrazonen, Hydroxylaminen und Oximen sowie Salzen davon, umfassend das Umsetzen in Gegenwart eines Übergangsmetall-Katalysators, einer aktivierten aromatischen Verbindung und eines Hydrazins, Hydrazons, Hydroxylamins oder Oxims oder Salz davon unter Bedingungen, die für den Übergangsmetall-Katalysator geeignet sind, die Erzeugung einer neuen Kohlenstoff-Heteroatom-Bindung zwischen dem aktivierten Kohlenstoff der aktivierten aromatischen Verbindung und einem Heteroatom des Hydrazins, Hydrazons, Hydroxylamins oder Oxims oder eines Salzes davon herbeizuführen.

2. Verfahren für die Vinylierung von Hydrazinen, Hydrazonen, Hydroxylaminen und Oximen und Salzen davon, umfassend das Umsetzen in Gegenwart eines Übergangsmetall-Katalysators einer aktivierten Vinyl-Verbindung und eines Hydrazins, Hydrazons, Hydroxylamins oder Oxims oder eines Salzes davon unter Bedingungen, die für den Übergangsmetall-Katalysator geeignet sind, die Erzeugung einer neuen Kohlenstoff-Heteroatom-Bindung zwischen dem aktivierten Kohlenstoff der aktivierten Vinyl-Verbindung und einem Heteroatom des Hydrazins, Hydrazons, Hydroxylamins oder Oxims oder eines Salzes davon herbeizuführen.

3. Verfahren nach Anspruch 1 oder 2, bei welchem das Übergangsmetall des Übergangsmetall-Katalysators ausgewählt ist aus der Gruppe, bestehend aus Fe, Ru, Co, Rh, Ir, Ni, Pd, Pt and Cu.

4. Verfahren nach Anspruch 1 oder 2, bei welchem der Übergangsmetall-Katalysator in substöchiometrischen Mengen im Bezug auf das begrenzende Reagenz unter der aktivierten Aryl- oder Vinyl-Verbindung und dem Hydrazin, Hydrazon, Hydroxylamin oder Oxim oder Salz davon vorliegt.

5. Verfahren nach Anspruch 3, bei welchem der Übergangsmetall-Katalysator in substöchiometrischen Mengen im Bezug auf das begrenzende Reagenz unter der aktivierten Aryl- oder Vinyl-Verbindung und dem Hydrazin, Hydrazon, Hydroxylamin oder Oxim oder Salz davon vorliegt,

6. Verfahren nach Anspruch 1 oder 2, bei welchem der Übergangsmetall-Katalysator mit 50 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

7. Verfahren nach Anspruch 3, bei welchem der Übergangsmetall-Katalysator mit 50 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

8. Verfahren nach Anspruch 1 oder 2, bei welchem der Übergangsmetall-Katalysator mit 10 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

9. Verfahren nach Anspruch 3, bei welchem der Übergangsmetall-Katalysator mit 10 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

10. Verfahren nach Anspruch 1 oder 2, bei welchem der Übergangsmetall-Katalysator mit 5 Molprozent oder weniger bezogen auf das begrenzende Reagenz unter der alctivierten Aryl- oder Vinyl-Verbindung and dem Hydrazin, Hydrazon, Hydroxylamin oder Oxim oder Salz davon vorliegt.

11. Verfahren nach Anspruch 3, bei welchem der Übergangsmetall-Katalysator mit 5 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

12. Verfahren nach Anspruch 1 oder 2, bei welchem der Übergangsmetall-Katalysator mit 2 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

13. Verfahren nach Anspruch 3, bei welchem der Übergangsmetall-Katalysator mit 2 Molprozent oder weniger bezogen auf das begrenzende Reagenz unter der aktivierten Aryl- oder Vinyl-Verbindung und dem Hydrazin, Hydrazon, Hydroxylamin oder Oxim oder Salz davon vorliegt.

14. Verfahren nach Anspruch 1 oder 2, bei welchem die aktivierte Gruppe an der aromatischen oder Vinyl-Verbindung ausgewählt ist aus der Gruppe, bestehend aus Halogeniden und Sulfonaten.

15. Verfahren nach Anspruch 14, bei welchem die aktivierte Gruppe an der aromatischen oder Vinyl-Verbindung ausgewählt ist aus der Gruppe, bestehend aus Chlorid, Bromid, Iodid, Tosylat, Mesylat, Triflat und Nonaflat,

16. Verfahren nach Anspruch 3, bei welchem die aktivierte Gruppe an der aromatischen oder Vinyl-Verbindung ausgewählt ist aus der Gruppe, bestehend aus Halogeniden und Sulfonaten.

17. Verfahren nach Anspruch 16, bei welchem die aktivierte Gruppe an der aromatischen oder Vinyl-Verbindung ausgewählt ist aus der Gruppe, bestehend aus Chlorid, Bromid, Iodid, Tosylat, Mesylat, Triflat und Nonaflat.

18. Verfahren nach Anspruch 1 oder 2, bei welchem das Hydrazin, Hydrazon, Hydroxylamin oder Oxim oder Salz davon ausgewählt ist aus der Gruppe, bestehend aus: Hydrazin, Alkyl- und Arylhydrazinen, 1,1- und 1,2-Dialkyl- und Diarylhydrazinen, 1,1-und 1,2-Alkylarylhydrazinen, 1,1,2-Trialkyl- und Triarylhydrazinen, 1,1,2-Dialkylarylund Alkyldiarylhydrazinen, Aldehyd- und Keton-Hydrazonen, *N*-Alkyl- und *N*-Arylaldehyd und -Keton-Hydrazonen, Hydroxylamin, *O*-Alkyl- und *O*-Arylhydroxylaminen, *N*-Alkyl- und *N*-Arylhydroxylaminen, *N,O*-Dialkyl- und *N,O*-Diarylhydroxylaminen, *N,O*-Alkylarylhydroxylaminen, *N,N*-Dialkyl- und *N,N*-Diarylhydroxylaminen und *N,N*-Alkylarylhydroxylaminen und Aldehyd- und Keton-Oximen und Salzen davon.

19. Verfahren nach Anspruch 3, bei welchem das Hydrazin, Hydrazon, Hydroxylamin oder Oxim oder Salz davon ausgewählt ist aus der Gruppe, bestehend aus: Hydrazin, Alkyl- und Arylhydrazinen, 1,1- und 1,2-Dialkyl- und -Diarylhydrazinen, 1,1- und 1,2-Alkylarylhydrazinen, 1,1,2-Trialkyl- und Triarylhydrazinen, 1,1,2-Dialkylaryl- und Alkyldiarylhydrazinen, Aldehyd- und Keton-Hydrazonen, *N*-Alkyl- und *N*-Arylaldehyd und -Keton-Hydrazonen, Hydroxylamin, *O*-Alkyl- und *O*-Arylhydroxylaminen, *N*-Alkylund *N*-Arylhydroxylaminen, *N,O*-Dialkyl- und *N,O*-Diarylhydroxylaminen, *N,O*-Alkylarylhydroxylaminen, *N,N*-Dialkyl- und *N,N*-Diarylhydroxylaminen und *N,N*-Alkylarylhydroxylaminen und Aldehyd- und Keton-Oximen oder einem Salz davon.

20. Verfahren nach Anspruch 1, **gekennzeichnet durch** das allgemeine Reaktionsschema: 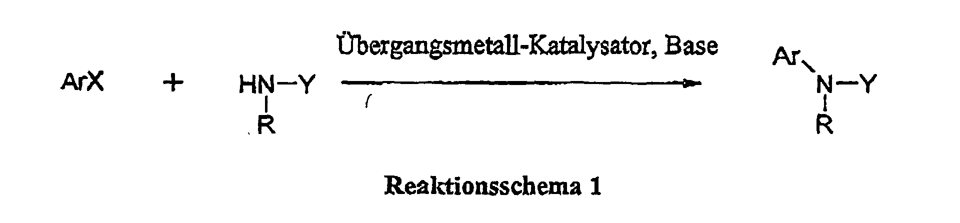 worin sind:
Ar stellt eine wahlweise substituierte, aromatische oder heteroaromatische Gruppe dar;
X stellt eine aktivierte Gruppe dar, z.B. ein Halogenid oder ein Sulfonat, die **durch** einen nukleophilen Stickstoff in einer **durch** eine Übergangsmetall-katalysierten Arylierungsreaktion ausgetauscht werden kann;
Y stellt NR₂, OR, N=CR₂, N(R)S(O)₂R oder N(R)C(O)NR₂ dar;
R stellt unabhängig für das jeweilige Auftreten, wie Wertigkeit und Stabilität es ermöglichen, H dar, ein wahlweise substituiertes Alkyl, Alkenyl oder Aryl; Formyl, Acyl, Sulfonyl oder -(CH₂)ₘ-R₈;
R₈ stellt unabhängig vom jeweiligen Auftreten ein wahlweise substituiertes Aryl, Cycloalkyl, Cycloalkenyl, heterocyclische Verbindung oder polycyclische Verbindung dar; und
m ist eine ganze Zahl im Bereich von Null bis 8, einschließlich.

21. Verfahren nach Anspruch 20, bei welchem Ar ausgewählt ist aus der Gruppe, bestehend aus wahlweise substituierten, monocyclischen, aromatischen und heteroaromatischen Gruppen.

22. Verfahren nach Anspruch 20, bei welchem X ausgewählt ist aus der Gruppe, bestehend aus Halogeniden und Sulfonaten.

23. Verfahren nach Anspruch 20, bei welchem X ausgewählt ist aus der Gruppe, bestehend aus Chlorid, Bromid, Iodid, Mesylat, Triflat, Tosylat und Nonaflat.

24. Verfahren nach Anspruch 20, bei welchem Y ausgewählt ist aus der Gruppe, bestehend aus NR₂, OR und N=CR₂.

25. Verfahren nach Anspruch 20, bei welchem Ar ausgewählt ist aus der Gruppe, bestehend aus wahlweise substituierten monocyclischen, aromatischen und heteroaromatischen Gruppen; X ist ausgewählt aus dex Gruppe, bestehend aus Chlorid, Bromid, Iodid, Mesylat, Triflat, Tosylat und Nonaflat; und Y ist ausgewählt aus der Gruppe, bestehend aus NR₂, OR und N=CR₂.

26. Verfahren nach Anspruch 1, **gekennzeichnet durch** das allgemeine Reaktionsschema: 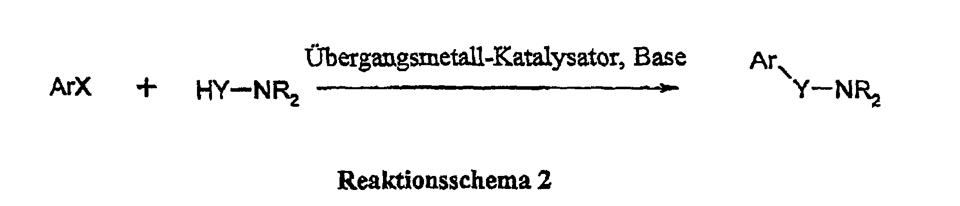 worin sind:
Ar stellt eine wahlweise substituierte, aromatische oder heteroaromatische Gruppe dar;
X stellt eine aktivierte Gruppe dar, z.B. ein Halogenid oder ein Sulfonat, die **durch** Y in einer **durch** ein Übergangsmetall-katalysierten Arylierungsreaktion ausgetauscht werden kann;
Y stellt O, S oder Se dar;
R stellt unabhängig für das jeweilige Auftreten, wie Wertigkeit und Stabilität es erlauben, H dar, ein wahlweise substituiertes Alkyl, Alkenyl oder Aryl; Alkyliden, Formyl, Acyl, Sulfonyl oder -(CH₂)ₘ-R₈; wobei die zwei Fälle für R zusammengenommen eine Alkyliden-Gruppe darstellen können;
R₈ stellt unabhängig für das jeweilige Auftreten ein wahlweise substituiertes Aryl, Cycloalkyl, Cycloalkenyl, heterocyclische oder polycyclische Verbindung dar; und
m ist eine ganze Zahl im Bereich von Null bis 8, einschließlich.

27. Verfahren nach Anspruch 26, bei welchem Ar ausgewählt ist aus der Gruppe, bestehend aus wahlweise substituierten monocyclischen, aromatischen und heteroaromatischen Gruppen.

28. Verfahren nach Anspruch 26, bei welchem X ausgewählt ist aus der Gruppe, bestehend aus Halogeniden und Sulfonaten.

29. Verfahren nach Anspruch 26, bei welchem X ausgewählt ist aus der Gruppe, bestehend aus Chlorid, Bromid, Iodid, Mesylat, Triflat, Tosylat and Nonaflat.

30. Verfahren nach Anspruch 26, bei welchem Y O ist.

31. Verfahren nach Anspruch 26, bei welchem Ar ausgewählt ist aus der Gruppe, bestehend aus wahlweise substituierten monocyclischen, aromatischen und heteroaromatischen Gruppen; X ist ausgewählt aus der Gruppe, bestehend aus Chlorid, Bromid, Iodid, Mesylat, Triflat, Tosylat und Nonaflat; und Y ist O.

32. Verfahren, **gekennzeichnet durch** das allgemeine Reaktionsschema 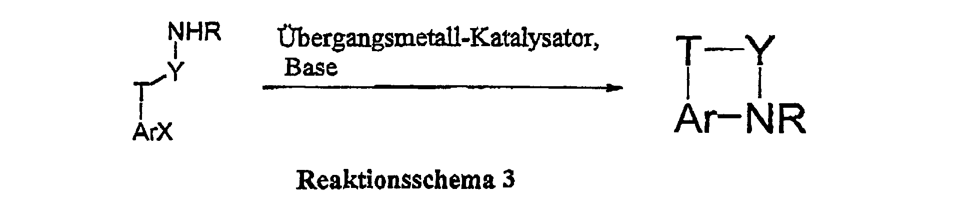 worin sind:
Ar stellt eine wahlweise substituierte, aromatische oder heteroaromatische Gruppe dar;
X stellt eine aktivierte Gruppe dar, z.B. ein Halogenid oder ein Sulfonat, die **durch** einen nukleophilen Stickstoff in einer **durch** ein Übergangsmetall-katalysierten Arylierungsreaktion ausgetauscht werden können;
Y stellt NR, O, *sp*²-hybridisiertes N in einer π-Bindung zu T, NS(O)₂R oder NC(O)NR₂ dar;
T stellt eine kovalente Verkettung dar, die Y und Ar verknüpft, wobei die Verkettung zwischen 0 und 4 Gerüstatome aufweist; wobei das Gerüst der Verkettung eine π-Bindung unter der Voraussetzung aufweisen kann, dass die Konfiguration der π-Bindung derart ist, dass die beschriebene intramolekulare Reaktion geometrisch ausführbar ist, oder dass die π-Bindung eine Konfiguration unter den Reaktionsbedingungen annehmen kann, die die intramolekulare Reaktion geometrisch durchführbar macht; wobei die Verkettung wahlweise unsubstituiert sein kann, eine beliebige Zahl von Substituenten eines beliebigen Typs aufweisen kann, der aufgrund der Stabilität und der Regeln für die Wertigkeit möglich ist;
R stellt unabhängig für das jeweilige Auftreten, wie Wertigkeit und Stabilität es erlauben, H dar, ein wahlweise substituiertes Alkyl, Alkenyl oder Aryl; Formyl, Acyl, Sulfonyl oder -(CH₂)ₘ-R₈;
R₈ stellt unabhängig für das jeweilige Auftreten ein wahlweise substituiertes Aryl, Cycloalkyl, Cycloalkenyl, heterocyclische Verbindung oder polycyclische Verbindung dar; und
m ist eine ganze Zahl im Bereich von Null bis 8, einschließlich.

33. Verfahren nach Anspruch 32, bei welchem Ar ausgewählt ist aus der Gruppe, bestehend aus wahlweise substituierten monocyclischen, aromatischen und heteroaromatischen Gruppen.

34. Verfahren nach Anspruch 32, bei welchem X ausgewählt ist aus der Gruppe, bestehend aus Halogeniden und Sulfonaten.

35. Verfahren nach Anspruch 32, bei welchem X ausgewählt ist aus der Gruppe, bestehend aus Chlorid, Bromid, Iodid, Mesylat, Triflat, Tosylat und Nonaflat.

36. Verfahren nach Anspruch 32, bei welchem Y ausgewählt ist aus der Gruppe, bestehend aus NR, O und *sp*²-hybridisiertem N in einer π-Bindung zu T.

37. Verfahren nach Anspruch 32, bei welchem Ar ausgewählt ist aus der Gruppe, bestehend aus wahlweise substituierten monocyclischen, aromatischen und heteroaromatischen Gruppen; X ist ausgewählt aus der Gruppe, bestehend aus Chlorid, Bromid, Iodid, Mesylat, Triflat, Tosylat und Nonaflat; und Y ist ausgewählt aus der Gruppe, bestehend aus NR, O und *sp*²-hybridisiertem N in einer π-Bindung zu T.

38. Verfahren, **gekennzeichnet durch** das allgemeine Reaktionsschema: 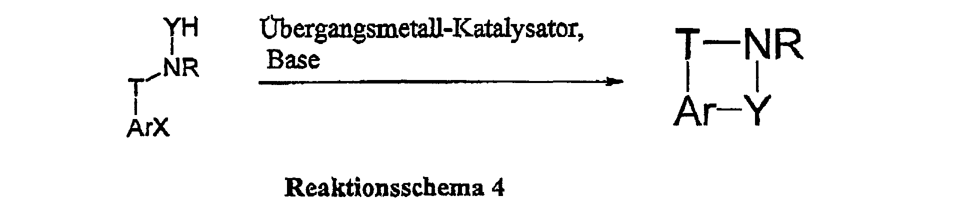 worin sind:
Ar stellt eine wahlweise substituierte, aromatische oder heteroaromatische Gruppe dar;
X stellt eine aktivierte Gruppe dar, z.B. ein Halogenid oder ein Sulfonat, die **durch** Y in einer **durch** ein Übergangsmetall-katalysierten Arylierungsreaktion ausgetauscht werden konnen;
Y stellt O, S oder Se dar;
T stellt eine kovalente Verkettung dar, die NR und Ar verknüpft, wobei die Verkettung zwischen 0 und 4 Gerüstatome aufweist; wobei das Gerüst der Vericettung eine π-Bindung unter der Voraussetzung aufweisen kann, dass die Konfiguration der π-Bindung derart ist, dass die beschriebene intramolekulare Reaktion geometrisch durchführbar ist, oder dass die π-Bindung eine Konfiguration unter den Reaktionsbedingungen annehmen kann, die die intramolekulare Reaktion geometrisch durchführbar macht; wobei die Verkettung wahlweise substituiert sein kann, eine beliebige Zahl von Substituenten eines beliebigen Typs tragen kann, der aufgrund der Stabilität und der Regeln der Wertigkeit erlaubt ist;
R stellt unabhängig für das jeweilige Auftreten, wie Wertigkeit und Stabilität es erlauben, H dar, eine wahlweise substituiertes Alkyl, Alkenyl oder Aryl; Formyl, Acyl, Sulfonyl oder -(CH₂)ₘ-R₈;
R₈ stellt unabhängig für das jeweilige Auftreten ein wahlweise substituiertes Aryl, Cycloalkyl, Cycloalkenyl, heterocyclische oder polycyclische Verbindung dar; und
m ist eine ganze Zahl im Bereich von Null bis 8, einschließlich.

39. Verfahren nach Anspruch 38, bei welchem das Ar ausgewählt ist aus der Gruppe, bestehend aus wahlweise substituierten monocyclischen, aromatischen und heteroaromatischen Gruppen.

40. Verfahren nach Anspruch 38, bei welchem X ausgewählt ist aus der Gruppe, bestehend aus Halogeniden und Sulfonaten.

41. Verfahren nach Anspruch 38, bei welchem X ausgewählt ist aus der Gruppe, bestehend aus Chlorid, Bromid, Iodid, Mesylat, Triflat, Tosylat und Nonaflat

42. Verfahren nach Anspruch 38, bei welchem Y O ist.

43. Verfahren nach Anspruch 38, bei welchem Ar ausgewählt ist aus der Gruppe, bestehend aus wahlweise substituierten monocyclischen, aromatischen und heteroaromatischen Gruppen; X ist ausgewählt aus der Gruppe, bestehend aus Chlorid, Bromid, Iodid Mesylat, Triflat, Tosylat und Nonaflat; und Y ist O.

44. , Verfahren nach Anspruch 2, **gekennzeichnet durch** das allgemeine Reaktionsschema: 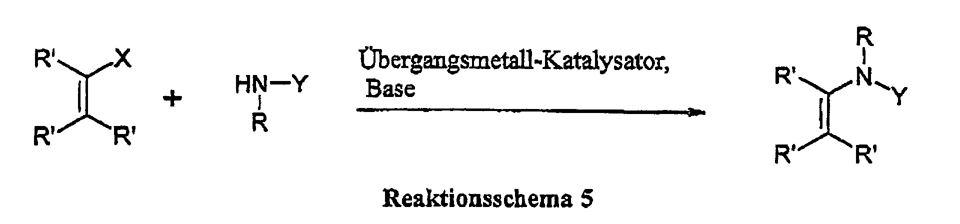 worin sind:
X stellt eine aktivierte Gruppe dar, z.B. ein Halogenid oder ein Sulfonat, die **durch** einen nukleophilen Stickstoff in einer **durch** ein Übergangsmetall-katalysierten Vinylierungsreaktion ausgetauscht werden können;
Y stellt NR₂, OR, N=CR₂, N(R)S(O)₂R oder N(R)C(O)NR₂ dar;
R stellt unabhângig für das jeweilige Auftreten, wie Wertigkeit und Stabilität es erlauben, H dar, ein wahlweise substituiertes Alkyl, Alkenyl oder Aryl; Formyl, Acyl, Sulfonyl oder -(CH₂)ₘ-R₈;
R' stellt unabhängig für das jeweilige Auftreten, wie Wertigkeit und Stabilität es erlauben, H dar, Halogen, niederes Alkyl, niederes Alkenyl, niederes Alkinyl, CarbonylGruppe (z.B. Ester, Carboxylat oder Formiat), Thiocarbonyl (z.B. Thiolester, Thiolcarboxylat oder Thiolformiat), Keton, Aldehyd, Amino, Acylamino, Amido, Amidino, Cyano, Nitro, Azido, Sulfonyl, Sulfoxido, Sulfat, Sulfonat, Sulfamoyl, Sulfonaxnido, Phosphoryl, Phosphonat, Phosphinat, -(CH₂)ₘ-R₈, -(CH₂)ₘ-OH, -(CH₂)ₘ-Oniederes Alkyl, -(CH₂)ₘ-O-niederes Alkenyl, -(CH₂)ₘ-O-(CH₂)ₙ-R₈, -(CH₂)ₘ-SH, -(CH₂)ₘ-S-niederes Alkyl, -(CH₂)ₘ-S-niederes Alkenyl, -(CH₂)ₘ-S-(CH₂)ₙ-R₈ oder Schutzgruppen der vorgenannten oder einen festen oder polymeren Träger;
R₈ stellt unabhängig für das jeweilige Auftreten ein wahlweise substituiertes Aryl, Cycloalkyl, Cycloalkenyl, heterocyclische Verbindung oder polycyclische Verbindung dar; und
n und m sind ganze Zahlen unabhängig für das jeweilige Auftreten, ausgewählt aus dem Bereich von Null bis 8, einschließlich.

45. Verfahren nach Anspruch 44, bei welchem X ausgewählt ist aus der Gruppe, bestehend aus Halogeniden und Sulfonaten.

46. Verfahren nach Anspruch 44, bei welchem X ausgewählt ist aus der Grappe, bestehend aus Chlorid, Bromid, Iodid, Mesylat, Triflat, Tosylat und Nonaflat.

47. Verfahren nach Anspruch 44, bei welchem Y ausgewählt ist aus der Gruppe, bestehend aus NR₂, OR und N=CR₂.

48. Verfahren nach Anspruch 44, bei welchem X ausgewählt ist aus der Gruppe, bestehend aus Chlorid, Bromid, Iodid, Mesylat, Triflat, Tosylat und Nonaflat; und Y ausgewählt ist aus der Gruppe, bestehend aus NR₂, OR and N=CR₂.

49. Verfahren nach Anspruch 2, **gekennzeichnet durch** das allgemeine Reaktionsschema: 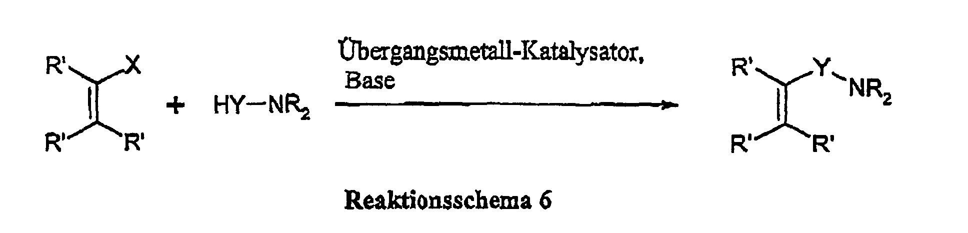 worin sind:
X stellt eine aktivierte Gruppe dar, z.B. ein Halogenid oder ein Sulfonat, die **durch** Y in einer **durch** ein Übergangsmetall-katalysierten Vinylierungsreaktion ausgetauscht werden können;
Y stellt O, S oder Se dar;
R stellt unabhängig für das jeweilige Auftreten, wie Wereigkeit und Stabilität es erlauben, H dar, ein wahlweise substituiertes Alkyl, Alkenyl oder Aryl; Alkyliden, Formyl, Acyl, Sulfonyl oder -(CH₂)ₘ-R₈; wobei die zwei Fälle von R zusammengenommen eine Alkyliden-Gruppe darstellen können;
R' sellt unabhängig für das jeweilige Auftreten, wie Wertigkeit und Stabilität es erlauben, H dar, Halogen, niederes Alkyl, niederes Alkenyl, niederes Alkinyl, eine Carbonyl-Gruppe(z.B. Ester, Carboxylat oder Formiat), Thiocarbonyl (z.B. Thiolester, Thiolcarboxylat oder Thiolformiat), Keton, Aldehyd, Amino, Acylamino, Amido Amidino, Cyano, Nitro, Azido, Sulfonyl, Sulfoxido, Sulfat, Sulfonat, Sulfamoyl, Sulfonamido, Phosphoryl, Phosphonat, Phosphinat, -(CH₂)ₘ-R₈, -(CH₂)ₘ-OH, -(CH₂)ₘ-O-niederes Alkyl, -(CH₂)ₘ-O-niederes Alkenyl, -(CH₂)ₘ-O-(CH₂)ₙ-R₈, -(CH₂)ₘ-SH, -(CH₂)ₘ-S-niederes Alkyl, -(CH₂)ₘ-S-niederes Alkenyl, -(CH₂)ₘ-S-(CH₂)ₙ-R₈ oder Schutzgruppen der vorgenannten oder einen festen oder polymeren Träger,
R₈ stellt unabhängig für das jeweilige Auftreten ein wahlweise substituiertes Aryl, Cycloalkyl, Cycloalkenyl, heterocyclische Verbindung oder polycyclische Verbindung dar; und
n and m sind ganze Zahlen unabhängig für das jeweilige Auftreten, ausgewählt aus dem Bereich von Null bis 8, einschließlich.

50. Verfahren nach Anspruch 49, bei welchem X ausgewählt ist aus der Grruppe, bestehend aus Halogeniden und Sulfonaten,

51. Verfahren nach Anspruch 49, bei welchem X ausgewählt ist aus der Gruppe, bestehend aus Chlorid, Bromid, Iodid, Mesylat, Triflat, Tosylat und Nonaflat.

52. Verfahren nach Anspruch 49, bei welchem Y O ist.

53. Verfahren nach Anspruch 49, bei welchem X ausgewählt ist aus der Gruppe, bestehend aus Chlorid, Bromid, Iodid, Mesylat, Triflat, Tosylat und Nonaflat; und Y O ist.

54. Verfahren nach Anspruch 1 oder 2, bei welchem der Übergangsmetall-Katalysator und der Ligand vor dem Zusatz der übrigen Reagenzien vorgemischt werden.

55. Verfahren für die Übergangsmetall-katalysierte Aminierung von aktivierten aromatischen Verbindungen, wobei der Übergangsmetall-Katalysator und der Ligand vor dem Zusatz der übrigen Reagenzien vorgemischt werden.

56. Verfahren für die synthetische Darstellung von Pyrrolen, Indolen, Furanen, Benzofuranen, bei welchem ein nach Anspruch 1 oder 2 erzeugtes Produkt den Bedingungen einer Broensted'schen oder Lewis-Säure unterworfen wird, um ein Pyrrol, Indol, Furan, Benzofuran zu erzeugen.

57. Verfahren nach Anspruch 56, bei welchem eine vielfältige Sammlung von heterocyclischen Produkten auf dem Wege paralleler, kombinierter Synthesemethoden erzeugt wird.

58. Verfahren nach Anspruch 57, bei welchem die Vertreter der Sammlung auf einem festen Träger aufgebracht sind.

59. Verfahren nach Anspruch 54, bei welchem die eingesetzte Base Cäsiumcarbonat oder Natrium-*tert*-Butoxid ist.

60. Verfahren nach Anspruch 55, bei welchem die eingesetzte Base Cäsiumcarbonat odes Natrium-*tert*-Butoxid ist.

61. Verfahren nach Anspruch 54, bei welchem der eingesetzte Ligand ausgewählt ist aus der Reihe von achiralen und chiralen komplexierenden Liganden.

62. Verfahren nach Anspruch 55, bei welchem der eingesetzte Ligand ausgewählt ist aus der Reihe von achiralen und chiralen komplexierenden Liganden,

## Revendications

1. Procédé pour l'arylation d'hydrazines, d'hydrazones, d'hydroxylamines et d'oximes, et de sels de celles-ci, comprenant la réaction, en présence d'un catalyseur de métal de transition, d'un composé aromatique activé et d'une hydrazine, d'une hydrazone, d'une hydroxylamine ou d'une oxime, ou d'un sel de celles-ci, dans des conditions appropriées pour que le catalyseur de métal de transition effectue la formation d'une nouvelle liaison carbone-hétéroatome entre le carbone activé dudit composé aromatique activé et un hétéroatome de ladite hydrazine, hydrazone, hydroxylamine ou oxime, ou d'un sel de celles-ci.

2. Procédé pour la vinylation d'hydrazines, d'hydrazones, d'hydroxylamines et d'oximes, et de sels de celles-ci, comprenant la réaction, en présence d'un catalyseur de métal de transition, d'un composé de vinyle activé et d'une hydrazine, d'une hydrazone, d'une hydroxylamine ou d'une oxime, ou d'un sel de celles-ci, dans des conditions appropriées pour que le catalyseur de métal de transition effectue la formation d'une nouvelle liaison carbone-hétéroatome entre le carbone activé dudit composé de vinyle activé et un hétéroatome de ladite hydrazine, hydrazone, hydroxylamine ou oxime, ou d'un sel de celles-ci.

3. Procédé suivant la revendication 1 ou 2, dans lequel le métal de transition du catalyseur de métal de transition est choisi dans le groupe constitué de Fe, Ru, Co, Rh, Ir, Ni, Pd, Pt et Cu.

4. Procédé suivant la revendication 1 ou 2, dans lequel le catalyseur de métal de transition est présent dans des quantités en dessous de la stoechiométrie par rapport au réactif limitant parmi ledit composé d'aryle ou de vinyle activé et ladite hydrazine, hydrazone, hydroxylamine ou oxime, ou un sel de celles-ci.

5. Procédé suivant la revendication 3, dans lequel le catalyseur de métal de transition est présent dans des quantités en dessous de la stoechiométrie par rapport au réactif limitant parmi ledit composé d'aryle ou de vinyle activé et ladite hydrazine, hydrazone, hydroxylamine ou oxime, ou un sel de celles-ci.

6. Procédé suivant la revendication 1 ou 2, dans lequel le catalyseur de métal de transition est présent dans une quantité inférieure ou égale à 50% en moles par rapport au réactif limitant parmi ledit composé d'aryle ou de vinyle activé et ladite hydrazine, hydrazone, hydroxylamine ou oxime, ou un sel de celles-ci.

7. Procédé suivant la revendication 3, dans lequel le catalyseur de métal de transition est présent dans une quantité inférieure ou égale à 50% en moles par rapport au réactif limitant parmi ledit composé d'aryle ou de vinyle activé et ladite hydrazine, hydrazone, hydroxylamine ou oxime, ou un sel de celles-ci.

8. Procédé suivant la revendication 1 ou 2, dans lequel le catalyseur de métal de transition est présent dans une quantité inférieure ou égale à 10% en moles par rapport au réactif limitant parmi ledit composé d'aryle ou de vinyle activé et ladite hydrazine, hydrazone, hydroxylamine ou oxime, ou un sel de celles-ci.

9. Procédé suivant la revendication 3, dans lequel le catalyseur de métal de transition est présent dans une quantité inférieure ou égale à 10% en moles par rapport au réactif limitant parmi ledit composé d'aryle ou de vinyle activé et ladite hydrazine, hydrazone, hydroxylamine ou oxime, ou un sel de celles-ci.

10. Procédé suivant la revendication 1 ou 2, dans lequel le catalyseur de métal de transition est présent dans une quantité inférieure ou égale à 5% en moles par rapport au réactif limitant parmi ledit composé d'aryle ou de vinyle activé et ladite hydrazine, hydrazone, hydroxylamine ou oxime, ou un sel de celles-ci.

11. Procédé suivant la revendication 3, dans lequel le catalyseur de métal de transition est présent dans une quantité inférieure ou égale à 5% en moles par rapport au réactif limitant parmi ledit composé d'aryle ou de vinyle activé et ladite hydrazine, hydrazone, hydroxylamine ou oxime, ou un sel de celles-ci.

12. Procédé suivant la reveadication I ou 2, dans lequel le catalyseur de métal de transition est présent dans une quantité inférieure ou égale à 2% en moles par rapport au réactif limitant parmi ledit composé d'aryle ou de vinyle activé et ladite hydrazine, hydrazone, hydroxylamine ou oxime, ou un sel de celles-ci.

13. Procédé suivant la revendication 3, dans lequel le catalyseur de métal de transition est présent dans une quantité inférieure ou égale à 2% en moles par rapport au réactif limitant parmi ledit composé d'aryle ou de vinyle activé et ladite hydrazine, hydrazone, hydroxylamine ou oxime, ou un sel de celles-ci.

14. Procédé suivant la revendieation 1 ou 2, dans lequel le groupe activé sur le composé aromatique ou de vinyle est choisi dans le groupe constitué d'halogénures et de sulfonates.

15. Proeédé suivant la revendication 14, dans lequel le groupe activé sur le composé aromatique ou de vinyle est choisi dans le groupe constitué de chlorure, de bromure, d'iodure, de tosylate, de mésylate, de triflate et de nonaflate.

16. Procédé suivant la revendication 3, dans lequel le groupe activé sur le composé aromatique ou de vinyle est choisi dans le groupe constitué d'halogénures et de sulfonates.

17. Procédé suivant la revendication 16, dans lequel le groupe activé sur le composé aromatique ou de vinyle est choisi dans le groupe constitué de chlorure, de bromure, d'iodure, de tosylate, de mésylate, de triflate et de nonaflate.

18. Procédé suivant la revendication 1 ou 2, dans lequel l'hydrazine, l'hydrazone, l'hydroxylamine ou l'oxime, ou un sel de celles-ci, est choisi dans le groupe constitué de l'hydrazine, d'hydrazines d'alkyle et d'aryle, d'hydrazines de 1,1- et 1,2-dialkyle et diaryle, d'hydrazines de 1,1- et 1,2-alkylaryle, d'hydrazines de 1,1,2-trialkyle et triaryle, d'hydrazines de 1,1,2-dialkylaryle et alkyldiaryle, d'hydrazones d'aldéhyde et de cétone, d'hydrazones de *N*-alkyl- et *N*-aryl- aldéhyde et cétone, de l'hydroxylamine, d'hydraxylamines d'*O*-alkyle et d'*O*-aryle, d'hydroxylamines de *N*-alkyle et de *N*-aryle, d'hydroxylamines de *N*,*O*-dialkyle et de *N*,*O*-diaryle, d'hydroxylamines de *N*,*O*-alkylaryle, d'hydroxylamines de *N*,*N*-dialkyle et de *N*,*N*-diaryle et d'hydroxylamines de *N,N*-alkylaryle, et d'oximes d'aldéhyde et de cétone, et de sels de celles-ci.

19. Procédé suivant la revendication 3, dans lequel l'hydrazine, l'hydrazone, l'hydroxylamine ou l'oxime, ou un sel de celles-ci, est choisi dans le groupe constitué de l'hydrazine, d'hydrazines d'alkyle et d'aryle, d'hydrazines de 1,1- et 1,2-dialkyle et diaryle, d'hydrazines de 1,1- et 1,2-alkylaryle, d'hydrazines de 1,1,2-trialkyle et triaryle, d'hydrazines de 1,1,2-dialkylaryle et alkyldiaryle, d'hydrazones d'aldéhyde et de cétone, d'hydrazones de *N*-alkyl- et *N*-aryl- aldéhyde et cétone, de l'hydroxylamine, d'hydroxylamines d'*O*-alkyle et d'*O*-aryle, d'hydroxylamines de *N*-alkyle et de *N*-aryle, d'hydroxylamines de *N,O*-dialkyle et de *N,O*-diaryle, d'hydroxylamines de *N,O*-alkylaryle, d'hydroxylamines de *N,N*-dialkyle et de *N,N*-diaryle et d'hydroxylamines de *N,N*-alkylaryle, et d'oximes d'aldéhyde et de cétone, ou d'un sel de celles-ci.

20. Procédé suivant la revendication 1, **caractérisé par** le schéma réactionnel général: 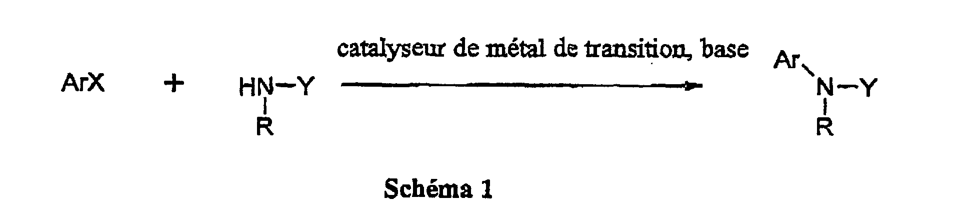 dans lequel:
Ar représente un groupe aromatique ou hétéroaromatique éventuellement substitué;
X représente un groupe activé, par ex., un halogénure ou un sulfonate, qui peut être remplacé par un azote nucléophile dans une réaction d'arylation catalysée par un métal de transition;
Y représente NR₂, OR, N=CR₂, N(R)S(O)₂R ou N(R)C(O)NR₂;
R représente indépendamment pour chaque occurrence, dans la mesure où la valence et la stabilité le permettent, H, un groupe alkyle, alcényle ou aryle éventuellement substitué; un groupe formyle, acyle, sulfonyle ou -(CH₂)ₘ-R₈;
R₈ représente indépendamment pour chaque occurrence un groupe aryle, cycloalkyle, cycloalcényle, un hétérocycle ou un polycycle éventuellement substitué; et
m est un nombre entier dans l'intervalle de 0 à 8 inclus.

21. Procédé suivant la revendication 20, dans lequel Ar est choisi dans le groupe constitué de groupes aromatiques et hétéroaromatiques monocycliques éventuellement substitués.

22. Procédé suivant la revendication 20, dans lequel X est choisi dans le groupe constitué d'halogénures et de sulfonates:

23. Procédé suivant la revendication 20, dans lequel X est choisi dans le groupe constitué de chlorure, de bromure, d'iodure, de mésylate, de triflate, de tosylate et de nonaflate.

24. Procédé suivant la revendication 20, dans lequel Y est choisi dans le groupe constitué de NR₂, OR et N=CR₂.

25. Procédé suivant la revendication 20, dans lequel Ar est choisi dans le groupe constitué de groupes aromatiques et hétéroaromatiques monocycliques éventuellement substitués; X est choisi dans le groupe constitué de chlorure, de bromure, d'iodure, de mésylate, de triflate, de tosylate et de nonaflate; et Y est choisi dans le groupe constitué de NR₂, OR et N=CR₂.

26. Procédé suivant la revendication 1, **caractérisé par** le schéma réactionnel général: 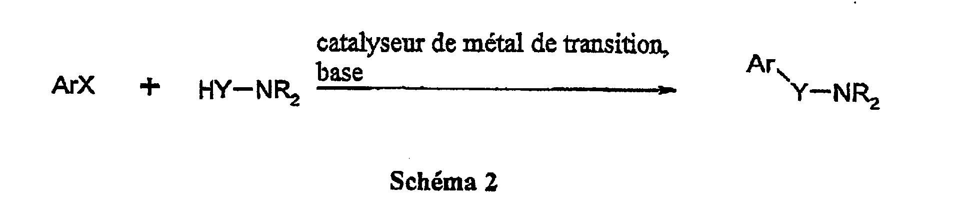 dans lequel;
Ar représente un groupe aromatique ou hétéroaromatique éventuellement substitué;
X représente un groupe activé, par ex., un halogénure ou un sulfonate, qui peut être remplacé par Y dans une réaction d'arylativon catalysée par un métal de transition;
Y représente O, S ou Se;
R représente indépendamment pour chaque occurrence, dans la mesure où la valence et la stabilité le permettent, H, un groupe alkyle, alcényle ou aryle éventuellement substitué; un groupe alkylidène, formyle, acyle, sulfonyle ou -(CH₂)ₘ-R₈; les deux cas de R pris ensemble peuvent représenter un groupe alkylidène;
R₈ représente indépendamment pour chaque occurrence un groupe aryle, cycloallcyle, cycloalcényle, un hétérocycle ou un polycycle éventuellement substitué; et
m est un nombre entier dans l'intervalle de 0 à 8 inclus.

27. Procédé suivant la revendication 26, dans lequel Ar est choisi dans le groupe constitué de groupes aromatiques et hétéroaromatiques monocycliques éventuellement substitués.

28. Procédé suivant la revendication 26, dans lequel X est choisi dans le groupe constitué d'halogénures et de sulfonates.

29. Procédé suivant la revendication 26, dans lequel X est choisi dans le groupe constitué de chlorure, de bromure, d'iodure, de mésylate, de triflate, de tosylate et de nonaflate.

30. Procédé suivant la revendication 26, dans lequel Y est O.

31. Procédé suivant la revendication 26, dans lequel Ar est choisi dans le groupe constitué de groupes aromatiques et hétéroaromatiques monocycliques éventuellement substitués; X est choisi dans le groupe constitué de chlorure, de bromure, d'iodure, de mésylate, de triflate, de tosylate et de nonaflate; et Y est O.

32. Procédé **caractérisé par** le schéma réactionnel général: 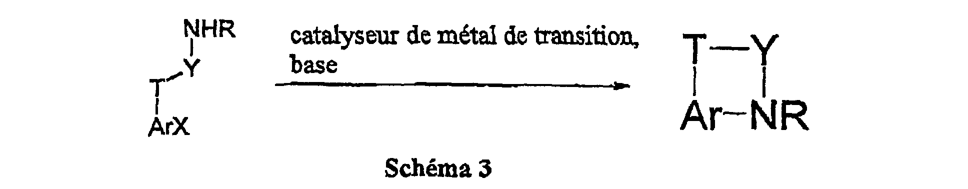 dans lequel:
Ar représente un groupe aromatique ou hétéroaromatique éventuellement substitué;
X représente un groupe activé, par ex., un halogénure ou un sulfonate, qui peut être remplacé par un azote nucléophile dans une réaction d'arylation catalysée par un métal de transition;
Y représente NR, O, N hybridé *sp*^{*2*} dans une liaison π avec T, NS(O)₂R ou NC(O)NR₂;
T représente une attache covalente reliant Y et Ar, ladite attache comprenant entre 0 et 4 atomes de squelette; le squelette de ladite attache peut comprendre une liaison π, à condition que la configuration de ladite liaison π soit telle que la réaction intramoléculaire décrite est faisable géométriquement ou que ladite liaison π puisse adopter une configuration dans les conditions réactionnelles qui rend la réaction intramoléculaire faisable géométriquement; ladite attache peut être éventuellement non substituée, porter un nombre quelconque de substituants de n'importe quel type permis par la stabilité et les règles de valence;
R représente indépendamment pour cheque occurrence, dans la mesure où la valence et la stabilité le permettent, H, un groupe allyle, alcényle ou aryle éventuellement substitué; un groupe formyle, acyle, sulfonyle ou -(CH₂)ₘ-R₈;
R₈ représente indépendamment pour chaque occurrence un groupe aryle, cycloalkyle, cycloalcényle, un hétérocycle ou un polycycle éventuellement substitué; et
m est un nombre entier dans l'intervalle de 0 à 8 inclus.

33. Procédé suivant la revendication 32, dans lequel Ar est choisi dens le groupe constitué de groupes aromatiques et hétéroaromatiques monocycliques éventuellement substitués.

34. Procédé suivant la revendication 32, dans lequel X est choisi dans le groupe constitué d'halogénures et de sulfonates.

35. Procédé suivant la revendication 32, dans lequel X est choisi dans le groupe constitué de chlorure, de bromure, d'iodure, de mésylate, de triflate, de tosylate et de nonaflate.

36. Procédé suivant la revendication 32, dans lequel Y est choisi dans le groupe constitué de NR, O et N hybridé *sp*^{*2*} dans une liaison π avec T.

37. Procédé suivant la revendication 32, dens lequel Ar est choisi dans le groupe constitué de groupes aromatiques et hétéroaromatiques monocycliques éventuellement substitués; X est choisi dans le groupe constitué de chlorure, de bromure, d'iodure, de mésylate, de triflate, de tosylate et de nonaflate; et Y est choisi dans le groupe constitué de NR, O et N hybridé *sp*^{*2*} dans une liaison π avec T.

38. Procédé **caractérisé par** le schéma réactionnel général: 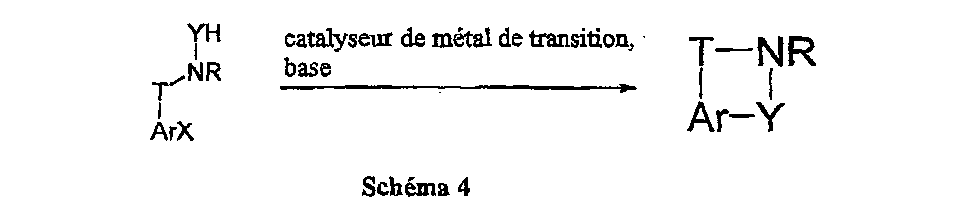 dans lequel:
Ar représente un groupe aromatique ou hétéroaromatique éventuellement substitué;
X représente un groupe activé, par ex., un halogénure ou un sulfonate, qui peut être remplacé par Y dans une réaction d'arylation catalysée par un métal de transition;
Y représente O, S ou Se;
T représente une attache covalence reliant NR et Ar, ladite attache comprenant entre 0 et 4 atomes de squelette; le squelette de ladite attache peut comprendre une liaison π, à condition que la configuration de ladite liaison π soit telle que la réaction intramoléculaire décrite est faisable géométriquement ou que ladite liaison π puisse adopter une configuration dans les conditions réactionnelles qui rend la réaction intramoléculaire faisable géométriquement; ladite attache peut être éventuellement non substituée, porter un nombre quelconque de substituants de n'importe quel type permis par la stabilité et les règles de valence;
R représente indépendamment pour chaque occurrence, dans la mesure où la valence et la stabilité le permettent, H, un groupe alkyle, alcényle ou aryle éventuellement substitué; un groupe formyle, acyle, sulfonyle ou -(CH₂)ₘ-R₈;
R₈ représente indépendamment pour chaque occurrence un groupe aryle, cycloalkyle, cycloalcényle, un hétérocycle ou un polycycle éventuellement substitué; et
m est un nombre entier dans l'intervalle de 0 à 8 inclus.

39. Procédé suivant la revendication 38, dans lequel Ar est choisi dans le groupe constitué de groupes aromatiques et hétéroaromatiques monocycliques éventuellement substitués.

40. Procédé suivant la revendication 38, dans lequel X est choisi dans le groupe constitué d'halogénures et de sulfonates.

41. Procédé suivant la revendication 38, dans lequel X est choisi dans le groupe constitué de chlorure, de bromure, d'iodure, de mésylate, de triflate, de tosylate et de nonaflate.

42. Procédé suivant la revendication 38, dans lequel Y est O.

43. Procédé suivant la revendication 38, dans lequel Ar est choisi dans le groupe constitué de groupes aromatiques et hétéroaromatiques monocycliques éventuellement substitués; X est choisi dans le groupe constitué de chlorure, de bromure, d'iodure, de mésylate, de triflate, de tosylate et de nonaflate; et Y est O.

44. Procédé suivant la revendication 2, **caractérisé par** le schéma réactionnel général: 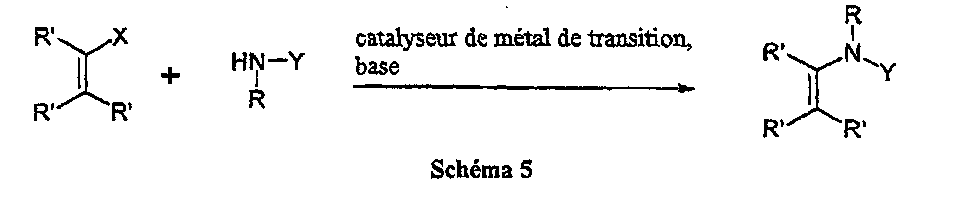 dans lequel:
X représente un groupe activé, par ex., un halogénure ou un sulfonate, qui peut être remplacé par un azote nucléophile dans une réaction de vinylation catalysée par un métal de transition;
Y représente NR₂, OR, N=CR₂, N(R)S(O)₂R ou N(R)C(O)NR₂;
R représente indépendamment pour chaque occurrence, dans la mesure où la valence et la stabilité le permettent, H, un groupe alkyle, alcényle ou aryle éventuellement substitué; un groupe formyle, acyle, sulfonyle ou -(CH₂)ₘ-R₈;
R' représente indépendamment pour chaque occurrence, dans la mesure où la valence et la stabilité le permettent, H, un halogène, un groupe alkyle inférieur, alcényle inférieur, alcynyle inférieur, carbonyle (par ex., un ester, un carboxylate ou un formiate), thiocarbonyle (par ex., un thiolester, un thiolcarboxylate ou un thiolformiate), cétone, aldéhyde, amino, acylamino, amido, amidino, cyano, nitro, azido, sulfonyle, sulfoxydo, sulfate, sulfonate, sulfamoyle, sulfonamuido, phosphoryle, phosphonate, phosphinate, -(CH₂)ₘ-R₈, -(CH₂)ₘ-OH, -(CH₂)ₘ-O-alkyle inférieur, -(CH₂)ₘ-O-alcényle inférieur, -CH₂)ₘ-O-(CH₂)ₙ-R₈, -(CH₂)ₘ-SH, -(CH₂)ₘ-S-alkyle inférieur, -(CH₂)ₘ-S-alcényle inférieur, -(CH₂)ₘ-S-(CH₂)ₙ-R₈, ou des groupes protecteurs de ce qui précède, ou un support solide ou polymère;
R₈ représente indépendamment pour chaque occurrence un groupe aryle, cycloalkyle, cycloalcényle, un hétérocycle ou un polycycle éventuellement substitué; et
n et m sont des nombres entiers choisis indépendamment pour chaque occurrence dans l'intervalle de 0 à 8 inclus.

45. Procédé suivant la revendication 44, dans lequel X est choisi dans le groupe constitué d'halogénures et de sulfonates.

46. Procédé suivant la revendication 44, dans lequel X est choisi dans le groupe constitué de chlorure, de bromure, d'iodure, de mésylate, de triflate, de tosylate et de nonaflate.

47. Procédé suivant la revendication 44, dans lequel Y est choisi dans le groupe constitué de NR₂, OR et N=CR₂.

48. Procédé suivant la revendication 44, dans lequel X est choisi dans le groupe constitué de chlorure, de bromure, d'iodure, de mésylate, de triflate, de tosylate et de nonaflate; et Y est choisi dans le groupe constitué de NR₂, OR et N=CR₂.

49. Procédé suivant la revendication 2, **caractérisé par** le schéma réactionnel général: 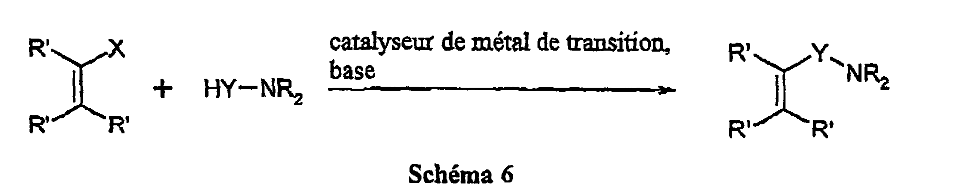 dans lequel:
X représente un groupe activé, par ex., un halogénure ou un sulfonate, qui peut être remplacé par Y dens une réaction de vinylation catalysée par un métal de transition;
Y représente O, S ou Se;
R représente indépendamment pour chaque occurrence, dans la mesure ou la valence et la stabilité le permettent, H, un groupe allyle, alcényle ou aryle éventuellement substitué; un groupe alkylidène, formyle, acyle, sulfonyle ou -(CH₂)ₘ-R₈; les deux cas de R pris ensemble peuvent représenter un groupe alkylidène;
R' représente indépendamment pour chaque occurrence, dans la mesure où la valence et la stabilité le permettent, H, un halogène, un gloupe alkyle inferieur, alcényle inférieur, alcynyle inférieur, carbonyle (par ex., un ester, un carboxylate ou un formiate), thiocarbonyle (par ex., un thiolester, un thiolcarboxylate ou un thiolformiate), cétone, aldéhyde, amino, acylamino, amido, amidino, cyano, nitro, azido, sulfonyle, sulfoxydo, sulfate, sulfonate, sulfamoyle, sulfonamido, phosphoryle, phosphonate, phosphinate, -(CH₂)ₘ-R₈, -(CH₂)ₘ-OH, -(CH₂)ₘ-O-alkyle inférieur, -(CH₂)ₘ-O-alcényle inférieur, -(CH₂)ₘ-O-(CH₂)ₙ-R₈, -(CH₂)ₘ-SH, -(CH₂)ₘ-S-alkyle inférieur, -(CH₂)ₘ-S-alcényle inférieur, -(CH₂)ₘ-S-(CH₂)ₙ-R₈, ou des groupes protecteurs de ce qui précède, ou un support solide ou polymère;
R₈ représente indépendamment pour chaque occurrence un groupe aryle, cycloalkyle, cycloalcényle, un hétérocycle ou un polycycle éventuellement substitué; et
n et m sont des nombres entiers choisis indépendamment pour chaque occurrence dans l'intervalle de 0 à 8 inclus.

50. Procédé suivant la revendication 49, dens lequel X est choisi dans le groupe constitué d'halogénures et de sulfonates.

51. Procédé suivant la revendication 49, dans lequel X est choisi dans le groupe constitué de chlorure, de bromure, d'iodure, de mésylate, de triflate, de tosylate et de nonaflate.

52. Procédé suivant la revendication 49, dans lequel Y est O.

53. Procédé suivant la revendication 49, dans lequel X est choisi dans le groupe constitué de chlorure, de bromure, d'iodure, de mésylate, de triflate, de tosylate et de nonaflate; et Y est O.

54. Procédé suivant la revendication 1 ou 2, dans lequel le catalyseur de métal de transition et le ligand sont mélangés au préalable avant l'ajout des réactifs restants.

55. Procédé pour l'amination catalysée par un métal de transition de composés aromatiques activés, dans lequel le catalyseur de métal de transition et le ligand sont mélangés au préalable avant l'ajout des réactifs restants.

56. Procédé pour la synthèse de pyrroles, d'indoles, de furannes, de benzofurannes, dans lequel un produit obtenu suivant la revendication 1 ou 2 est souamis à des conditions d'acide de Brönsted ou de Lewis pour donner un pyrrole, un indole, un furanne, un benzofuranne.

57. Procédé suivant la revendication 56, dans lequel une bibliothèque variée de produits hétérocycliques est obtenue par des méthodes de synthèse parallèles, combinatoires.

58. Procédé suivant la revendication 57, dans lequel les éléments de la bibliothèque sont fixés à un support solide,

59. Procédé suivant la revendication 54, dans lequel la base utilisée est du carbonate de césium ou du *tert*-butoxyde de sodium.

60. Procédé suivant la revendication 55, dans lequel la base utilisée est du carbonate de césium ou du *tert*-butoxyde de sodium.

61. Procédé suivant la revendication 54, dans lequel le ligand utilisé est choisi dans le groupe de ligands de chélation achiraux et chiraux.

62. Procédé suivant la revendication 55, dans lequel le ligand utilisé est choisi dans le groupe de ligands de chélation achiraux et chiraux.
